# EUROPEAN PATENT SPECIFICATION

(11) **EP 1 819 332 B1**
(45) Date of publication and mention of the grant of the patent: **11.03.2009**
(21) Application number: 05821654.0
(22) Date of filing: 02.12.2005
(51) Int. Cl.: A61K 31/437, A61K 31/4375, A61P 3/10, C07K 5/078, C07K 5/097, A61K 38/05, A61K 38/06

(54) **PYRROLOPYRIDINE-2-CARBOXYLIC ACID AMIDES**
PYRROLOPYRIDIN-2-KARBONSÄUREAMIDE
AMIDES D'ACIDE PYRROLOPYRIDINE-2-CARBOXYLIQUE

(30) Priority: 02.12.2004 US 632461 P
(43) Date of publication of application: 22.08.2007
(73) Proprietor: Prosidion Limited, Oxford, Oxfordshire OX4 6LT (GB)
(72) Inventor: KRULLE, Thomas Martin, Oxford, Oxfordshire OX4 6LT (GB); ROWLEY, Robert John, Oxford, Oxfordshire OX4 6LT (GB); THOMAS, Gerard Hugh, Oxford, Oxfordshire OX4 6LT (GB)
(74) Representative: Blakey, Alison Jane
(86) International application number: PCT/GB2005/050233
(87) International publication number: WO 2006/059164

(56) References cited:
- WO-A-03/027108
- WO-A-03/074513
- WO-A-03/091213
- WO-A-20/04104001
- US-A1- 2004 002 495
- ROSAUER, KEITH G. ET AL: "Novel 3,4-dihydroquinolin-2(1H)-one inhibitors of human glycogen phosphorylase a" BIOORGANIC & MEDICINAL CHEMISTRY LETTERS , 13(24), 4385-4388 CODEN: BMCLE8; ISSN: 0960-894X, 2003, XP002379195

## Description

### BACKGROUND OF THE INVENTION

The present invention is directed to pyrrolopyridine-2-carboxylic acid amides. In particular, the present invention is directed to pyrrolopyridine-2-carboxylic acid amides that are inhibitors of glycogen phosphorylase.

Insulin dependent Type I diabetes and non-insulin dependent Type II diabetes continue to present treatment difficulties even though clinically accepted regimens that include diet, exercise, hypoglycemic agents, and insulin are available. Treatment is patient dependent, therefore there is a continuing need for novel hypoglycemic agents, particularly ones that may be better tolerated with fewer adverse effects.

The liver and certain other organs produce glucose by breaking down glycogen or by synthesizing glucose from small molecule precursors, thereby raising the blood sugar levels. The breakdown of glycogen is catalyzed by glycogen phosphorylase enzyme. Accordingly, inhibiting glycogen phosphorylase ("GP") may lower the elevated blood sugar level in diabetic patients.

Similarly, hypertension and its associated pathologies such as, for example, atherosclerosis, lipidemia, hyperlipidemia and hypercholesterolemia have been associated with elevated insulin levels (hyperinsulinemia), which can lead to abnormal blood sugar levels. Furthermore, myocardial ischemia can result. Such maladies may be treated with hypoglycemic agents, including compounds that inhibit glycogen phosphorylase. The cardioprotective effects of glycogen phosphorylase inhibitors, for example following reperfusion injury, has also been described (see, for example, Ross et al., American Journal of Physiology. Heart and Circulatory Physiology, Mar 2004, 286(3), H1177-84). Accordingly, it is accepted that compounds that inhibit glycogen phosphorylase (see, for example, U.S. Patent No. 6,297,269) are useful in the treatment of diabetes, hyperglycemia, hypercholesterolemia, hyperinsulinemia, hyperlipidemia, atherosclerosis or myocardial ischemia. Nevertheless, it would be desirable to obtain other novel compounds that inhibit glycogen phosphorylase.

R. Kurukulasuriya,1.T. Link, et al., Current Medicinal Chem., 10:99-121(2003) describes "Prospects for Pharmacologic Inhibition of Hepatic Glucose Production." R. Kurukulasuriya, J.T. Link, et al., Current Medicinal Chem., 10:123-153(2003) describes "Potential Drug Targets and Progress Towards Pharmacologic Inhibition of Hepatic Glucose Production."

U.S. Patent No. 6,297,269 and European Patent No. EP 0832066 describe substituted N-(indole-2-carbonyl)amides and derivatives as glycogen phosphorylase inhibitors. U.S. Patent No. 6,107,329 and 6,277,877 describe substituted *N*-(indole-2-carbonyl)glycinamides and derivatives as glycogen phosphorylase inhibitors. U.S. Patent No. 6,399,601 describes bicyclic pyrrolyl amides as glycogen phosphorylase inhibitors. European Patent Application Nos. EP 0978276 and EP 1136071 describe inhibitors of human glycogen phosphorylase and their use. International Patent Publication No. WO 01/68055 describes glycogen phosphorylase inhibitors. U.S. Patent Application No. US2004/0002495 describes glycogen phosphorylase inhibitors. U.S. Patent No. 5,952,322 describes a method of reducing non-cardiac ischemial tissue damage using glycogen phosphorylase inhibitors.

International Patent Publication No. WO 01/55146 describes arylamidines. International Patent Publication No. WO 01/62775 describes antiarrhythmic peptides. International Patent Publication No. WO 01/96346 describes tricyclic compounds. International Patent Publication No. WO 02/16314 describes substituted polyamine compounds. International Patent Publication No. WO 02/20475 describes serine protease activity inhibitors. International Patent Publication No. WO 02/40469 describes bombesin receptor antagonists. International Patent Publication No. WO 02/46159 describes guanidine and amidine derivatives. International Patent Publication No. WO 00/69815 describes ureido-substituted cyclic amine derivatives. International Patent Publication No. WO 03/074513 discloses various indole derivatives as glycogen phosphorylase inhibitors.

International Patent Publication No. WO 00/43384 describes aromatic heterocyclic compounds. International Patent Publication Nos. WO 02/26697 and WO 00/76970 describe aromatic derivatives. International Patent Publication No. WO 01/32622 describes indoles. European Patent Application No. EP 1101759 describes phenylazole compounds. European Patent Application No. EP 1179341 describes cyclic amino compounds. U.S. Patent No. 6,037,325 describes substituted heterocyclic compounds. U.S. Patent No. 5,672,582 describes 4-substituted cyclohexylamine derivatives. European Patent Application No. EP 1201239 describes cyclic amine CCR3 antagonists. International Patent Publication No. WO 98/25617 describes substituted aryl piperazines. U.S. Patent No. 5,756,810 describes preparing 3-nitrobenzoate compounds.

U.S. Patent No. 5,710,153 describes tetrazole compounds. U.S. Patent Nos. 6,174,887 and 6,420,561 describe amide compounds. S.P. Hiremath et al., Acta Ciencia Indica, XVIII:397(1992) describes the synthesis and biological activities of indolylthiosemicarbazides and semicarbazides. International Patent Publication No. WO 96/36595 describes 3,4-disubstituted phenylsulfonamides. U.S. Patent No. 5,618,825 describes combinatorial sulfonamide libraries. European Patent Application No. EP 0810221 describes oxygen-containing heterocyclic derivatives. European Patent Application No. EP 0345990 describes polypeptide compounds. European Patent Application No. EP 0254545 describes diamine compounds.

International Patent Publication No. WO 97/31016 describes inhibitors of SH2-mediated processes. U.S. Patent No. 6,034,067 describes serine protease inhibitors. International Patent Publication No. WO 97/17985 and U.S. Patent No. 6,107,309 describe hemoregulatory compounds. U.S. Patent No. 6,432,921 describes thrombin inhibitors. U.K. Patent Application No. GB 2292149 describes peptide inhibitors of pro-interleukin-1β converting enzyme. U.S. Patent No. 5,821,241 describes fibrinogen receptor antagonists.

International Patent Publication No. WO 01/02424 describes peptide boronic acid compounds. U.S. Patent Nos. 6,001,811, 5,869,455 and 5,618,792 describe oxadiazole, thiadiazole and triazole peptoids. U.S. Patent Nos. 5,885,967, 6,090,787 and 6,124,277 describe thrombin inhibiting peptide derivatives. U.S. Patent No. 6,455,529 describes adhesion receptor antagonists. U.S. Patent No. 6,410,684 describes serine protease inhibitors.

International Patent Publication No. WO 01/94310 describes bis-heterocyclic alkaloids. U.S. Patent Publication No. 20030004162A1, European Patent Application No. EP 0846464, and International Publication No. WO 96/39384 describe glycogen phosphorylase inhibitors. International Patent Publication No. WO 97/28798 describes pyrrolidine derivatives. U.S. Patent No. 5,346,907 describes amino acid analogs.

International Patent Application No. PCT/US2004/016243 (published after the priority date of the present invention) discloses pyrrolopyridine-2-carboxylic acid amide inhibitors of glycogen phosphorylase.

### SUMMARY OF THE INVENTION

Compounds represented by Formula (I): 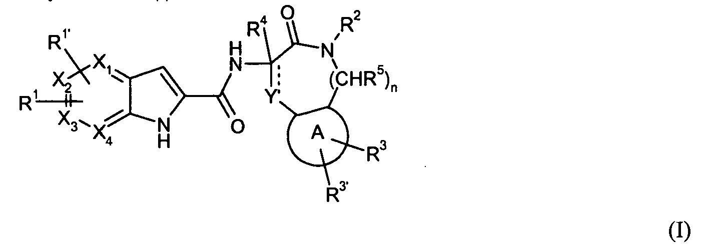 or stereoisomers or pharmaceutically acceptable salts thereof, are inhibitors of glycogen phosphorylase and are useful in the prophylactic or therapeutic treatment of diabetes, hyperglycemia, hypercholesterolemia, hyperinsulinemia, hyperlipidemia, hypertension, atherosclerosis or tissue ischemia e.g. myocardial ischemia, and as cardioprotectants.

### DETAILED DESCRIPTION OF THE INVENTION

The present invention provides a compound of Formula (I): 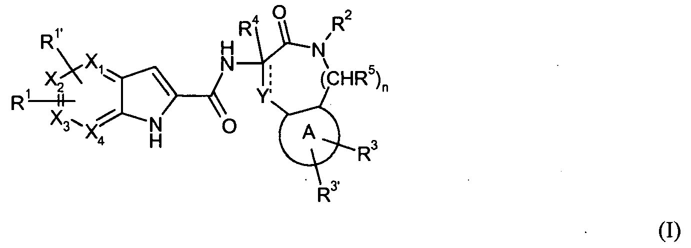 or a stereoisomer, or a pharmaceutically acceptable salt thereof, wherein:
one of X₁, X₂, X₃ and X₄ is N and the others are C;
---- is a single or double bond;
when ---- is a single bond Y is CHR⁶, NH, O, S, SO₂ CHR⁶O, CHR⁶S, CHR⁶SO₂, CHR⁶CO or CH₂CHR⁶; and when ---- is a double bond Y is CR⁶ or N;
A is aryl or heteroaryl;
R¹and R^{1'}are independently selected from hydrogen, halogen, hydroxy, cyano, C₁₋₆alkyl, C₁₋₆alkoxy, fluoromethyl, difluoromethyl, trifluoromethyl, C₂₋₆alkenyl, C₂₋₆alkynyl, aryl, -C₁₋₆alkylaryl, - C₁₋₆alkylheteroaryl and aryloxy;
R² is hydrogen, C₁₋₆alkyl optionally substituted by cyano, O-C₁₋₄alkyl-OR⁷, OR⁷, COOR⁷, CONR⁸R⁹, CONR⁸OR⁹, C(NH₂)=NOH, NR¹⁶R¹⁷, NHC(O)OR¹⁶, NHS(O)₂,R¹⁸, NHC(O)R¹⁸, SR¹⁶, S(O)R¹⁸ or S(O)₂R¹⁸; or R² is C₁₋₄alkyl-CONR¹⁰R¹¹, C₂₋₆alkenyl, aryl, -C₁₋₆alkylaryl, -C₁₋₆alkylheterocyclyl or -C₁₋₆alkylheteroaryl;
R³ and R^{3'} are independently selected from hydrogen, halogen, hydroxy, cyano, C₁₋₄alkyl, C₁₋₄alkoxy, fluoromethyl, difluoromethyl, trifluoromethyl, ethenyl and ethynyl;
when ---- is a single bond R⁴ is hydrogen, C₁₋₆alkyl, aryl, or C₂₋₆alkenyl or when ---- is a double bond R⁴ is absent;
R⁵ and R⁶ are independently selected from hydrogen, C₁₋₆alkyl, aryl, C₂₋₆alkenyl, cyano, tetrazole, COOR¹², CONR¹²R¹³ and CONR¹²OR¹³;
R⁷, R⁸ and R⁹ are independently selected from hydrogen and C₁₋₄alkyl;
R¹⁰ and R¹¹ are independently selected from hydrogen, C₁₋₄alkyl optionally substituted by OR⁷, COOR⁷ or NR¹⁴R¹⁵, aryl, heteroaryl, C₃₋₇cycloalkyl, heterocyclyl, -C₁₋₄alkylaryl, -C₁₋₄alkylheteroaryl, -C₁₋₄alkylC₃₋₇CyCloalkyl or -C₁₋₄alkylheterocyclyl wherein any of the rings is optionally substituted by 1 or 2 substituents independently selected from halogen, hydroxy, cyano, C₁₋₄alkyl, C₁₋₄alkoxy, fluoromethyl, difluoromethyl and trifluoromethyl;
or R¹⁰ and R¹¹ together with the nitrogen to which they are attached form a 4- to 7-membered heterocycle optionally containing a further heteroatom selected from N and **O,** which heterocycle is optionally substituted by C₁₋₄alkyl, halo, OR⁷ or COR⁷, or two bonds on a ring carbon of the heterocycle optionally can form an oxo ( =O) substituent;
R¹² and R¹³ are independently selected from hydrogen, C₁₋₄alkcyl, aryl, -C₁₋₄alkylaryl and -C₁₋₄alkylheteroaryl;
or R¹² and R¹³ may be cyclised to form an optionally substituted 4- to 7-membered heterocycle;
R¹⁴ and R¹⁵ are independently selected from hydrogen and C₁₋₄alkyl;
or R¹⁴ and R¹⁵ together with the nitrogen to which they are attached form a 4- to 7-membered heterocycle optionally containing a further heteroatom selected from N and **O,** which heterocycle is optionally substituted by C₁₋₄alkyl, halo, OR⁷ or COR⁷, or two bonds on a ring carbon of the heterocycle optionally can form an oxo ( =O) substituent;
R¹⁶ and R¹⁷ are independently selected from hydrogen and C₁₋₄alkyl;
R¹⁸ is C₁₋₄alkyl; and
n is 0 or 1.

The molecular weight of the compounds of Formula (I) is preferably less than 800, more preferably less than 600.

A is preferably a fused benzene, pyridine or thiophene ring, more preferably a fused benzene ring.

When ---- is a single bond R⁴ is preferably hydrogen.

In R² the C₁₋₆alkyl and C₁₋₄alkyl groups are preferably C₁₋₂alkyl.

When R² is C₁₋₆alkyl-CO-NR⁸R⁹ it is preferably CH₂-CO-NR⁸R⁹ and when R² is C₁₋₄alkyl-CO-NR¹⁰R¹¹ it is preferably CH₂-CO-NR¹⁰R¹¹.

When R¹⁰ and R¹¹, R¹² and R¹³ or R¹⁴ and R¹⁵ together with the nitrogen to which they are attached form a 4- to 7-membered heterocycle optionally containing a further heteroatom selected from N and **O,** said heterocycle is preferably optionally substituted by 1 or 2 substituents selected from C₁₋₄alkyl, halo, OR⁷ and COR⁷, or two bonds on a ring carbon of the heterocycle optionally can form an oxo ( =O) substituent

Suitably R⁵ represents hydrogen.

A specific group of compounds of Formula (I) which may be mentioned are those wherein R² is hydrogen, C₁₋₆alkyl optionally substituted by OR⁷, COOR⁷ or CONR⁸R⁹; or C₁₋₄alkyl-CONR¹⁰R¹¹, C₂₋₆alkyl, aryl, -C₁₋₆alkylaryl or -C₁₋₆alkylheteroaryl.

A preferred group of compounds of the present invention are the compounds of Formula (Ia): 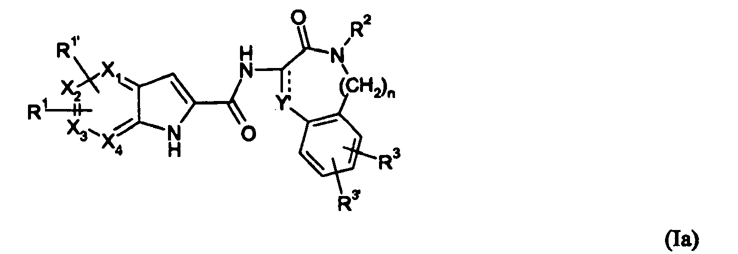 or a stereoisomer, or a pharmaceutically acceptable salt thereof, wherein:
one of X₁, X₂, X₃ and X₄ is N and the others are C;
---- is a single or double bond;
when ---- is a single bond Y is CH₂, NH or O; and when ---- is a double bond Y is CH or N;
R¹ and R^{1'} are independently selected from hydrogen, halogen, hydroxy, cyano, C₁₋₄alkyl, C₁₋₄alkoxy, fluoromethyl, difluoromethyl, trifluoromethyl, ethenyl and ethynyl;
R² is hydrogen, C₁₋₄alkyl optionally substituted by cyano, O-C₁₋₄alkyl-OR⁴, OR⁴, COOR⁴, CONR⁵R⁶, CONR⁵OR⁶, C(NH₂)=N(OH), NR¹⁶R¹⁷, NHC(O)OR¹⁶, NHS(O)₂R¹⁸, NHC(O)R¹⁸, SR¹⁶, S(O)R¹⁸ or S(O)₂R¹⁸; or R² is C₁₋₄alkyl-CONR⁷R⁸ or -C₁₋₄alkylheterocyclyl;
R³ and R^{3'} are independently selected from hydrogen, halogen, hydroxy, cyano, C₁₋₄alkyl, C₁₋₄alkoxy, fluoromethyl, difluoromethyl, trifluoromethyl, ethenyl and ethynyl;
R⁴, R⁵ and R⁶ are independently selected from hydrogen and C₁₋₄alkyl;
R⁷ and R⁸ are independently selected from hydrogen, C₁₋₄alkyl optionally substituted by OR⁴, COOR⁴ or NR⁹R¹⁰, aryl, heteroaryl, C₃₋₇cycloalkyl, heterocyclyl, -C₁₋₄alkylaryl, -C₁₋₄alkylheteroaryl,-C₁₋₄alkylC₃₋₇cycloalkyl or -C₁₋₄alkylheterocyclyl wherein any of the rings is optionally substituted by 1 or 2 substituents independently selected from halogen, hydroxy, cyano, C₁₋₄alkyl, C₁₋₄alkoxy, fluoromethyl, difluoromethyl and trifluoromethyl;
or R⁷ and R⁸ together with the nitrogen to which they are attached form a 4- to 7-membered heterocycle optionally containing a further heteroatom selected from N and O, which heterocycle is optionally substituted by C₁₋₄alkyl, halo, OR⁴ or COR⁴, or two bonds on a ring carbon of the heterocycle optionally can form an oxo ( =O) substituent;
R⁹ and R¹⁰ are independently selected from hydrogen and C₁₋₄alkyl;
or R⁹ and R¹⁰ together with the nitrogen to which they are attached form a 4- to 7-membered heterocycle optionally containing a further heteroatom selected from N and O, which heterocycle is optionally substituted by C₁₋₄alkyl, halo, OR⁴ or COR⁴, or two bonds on a ring carbon of the heterocycle optionally can form an oxo ( =O) substituent;
R¹⁶ and R¹⁷ are independently selected from hydrogen and C₁₋₄alkyl;
R¹⁸ is C₁₋₄alkyl; and
n is 0 or 1.

When R² is C₁₋₄alkyl-CO-NR⁵R⁶ it is preferably CH₂-CO-NR⁵R⁶.

When R⁹ and R¹⁰ together with the nitrogen to which they are attached form a 4- to 7-membered heterocycle optionally containing a further heteroatom selected from N and O, said heterocycle is preferably optionally substituted by 1 or 2 substituents selected from C₁₋₄alkyl, halo, OR⁷ and COR⁷, or two bonds on a ring carbon of the heterocycle optionally can form an oxo ( =O) substituent.

A specific group of compounds of Formula (Ia) which may be mentioned are those wherein R² represents hydrogen, C₁₋₄alkyl optionally substituted by OR⁴, COOR⁴ or CONR⁵R⁶; or C₁₋₄alkyl-CONR⁷R⁸_{.}

In the compounds of Formulae (I) and (Ia)**:**
Preferably one of X₂, X₃ and X₄ is N, more preferably X₃ is N.
---- is preferably a single bond.
Y is preferably CH or CH₂, more preferably CH₂.

Preferably R¹ and R^{1'} are independently selected from hydrogen, halogen and cyano.

A preferred group of compounds are those where X₃ is N, one of R¹ and R^{1'} is hydrogen and the other is a 5-halo or 5-cyano group, especially a 5-chloro group.

Preferably R³ and R³' are independently selected from hydrogen, halogen, C₁₋₄alkyl e.g. methyl, C₁₋₄alkoxy e.g. methoxy, and trifluoromethyl. Preferably at least one of R³ and R^{3'} is hydrogen.

n is preferably 0.

Specific compounds of the invention which may be mentioned are those included in the examples, as the free base or a pharmaceutically acceptable salt thereof.

While the preferred groups for each variable have generally been listed above separately for each variable, preferred compounds of this invention include those in which several or each variable in Formula (I) is selected from the preferred, more preferred, especially or particularly listed groups for each variable. Therefore, this invention is intended to include all combinations of preferred, more preferred, most preferred, especially and particularly listed groups.

As used herein, unless stated otherwise, "alkyl" as well as other groups having the prefix "alk" such as, for example, alkoxy, alkanyl, alkenyl, alkynyl, and the like, means carbon chains which may be linear or branched or combinations thereof. Examples of alkyl groups include methyl, ethyl, propyl, isopropyl, butyl, sec- and tert-butyl, pentyl, hexyl, heptyl and the like. "Alkenyl", "alkynyl" and other like terms include carbon chains having at least one unsaturated carbon-carbon bond.

As used herein, for example, "C₁₋₄alkyl" is used to mean an alkyl having 1-4 carbons - that is, 1, 2, 3, or 4 carbons in a straight or branched configuration. The term "C₁₋₆alkyl" may be interpreted in an analogous fashion.

The term "cycloalkyl" means carbocycles containing no heteroatoms, and include mono-, bi-, and tricyclic saturated carbocycles, as well as fused and bridged systems. Such fused ring systems can include one ring that is partially or fully unsaturated, such as a benzene ring, to form fused ring systems, such as benzofused carbocycles. Cycloalkyl includes such fused ring systems as spirofused ring systems. Examples of cycloalkyl and carbocyclic rings include C₃₋₇Cycloalkyl such as cyclopropyl, cyclobutyl, cyclopentyl, cyclohexyl, and decahydronaphthalene, adamantane, indanyl, 1,2,3,4-tetrahydronaphthalene and the like.

The term "halogen" includes fluorine, chlorine, bromine, and iodine atoms.

The term "aryl" is well known to chemists. The preferred aryl groups are phenyl and naphthyl, more preferably phenyl.

The term "heteroaryl" is well known to chemists. The term includes 5- or 6-membered heteroaryl rings containing 1-4 heteroatoms chosen from oxygen, sulfur, and nitrogen in which oxygen and sulfur are not next to each other. Examples of such heteroaryl rings are furyl, thienyl, pyrrolyl, pyrazolyl, imidazolyl, oxazolyl, isoxazolyl, thiazolyl, isothiazolyl, triazolyl, oxadiazolyl, thiadiazolyl, tetrazolyl, pyridyl, pyridazinyl, pyrimidinyl, pyrazinyl, and triazinyl. The term "heteroaryl" includes heteroaryl rings with fused carbocyclic ring systems that are partially or fully unsaturated, such as a benzene ring, to form a benzofused hetaryl. For example, benzimidazole, benzoxazole, benzothiazole, benzofuran, quinoline, isoquinoline, quinoxaline, and the like.

Unless otherwise stated, the terms "heterocyclic ring" and "heterocycle" are equivalent, and include 4-8-membered saturated or partially saturated rings containing one or two heteroatoms chosen from oxygen, sulfur, and nitrogen. The sulfur and oxygen heteroatoms are not directly attached to one another. Any nitrogen heteroatoms in the ring may optionally be substituted with C₁₋₄alkyl. Examples of heterocyclic rings include azetidine, oxetane, tetrahydrofuran, tetrahydropyran, oxepane, oxocane, thietane, thiazolidine, oxazolidine, oxazetidine, pyrazolidine, isoxazolidine, isothiazolidine, tetrahydrothiophene, tetrahydrothiopyran, thiepane, thiocane, azetidine, pyrrolidine, piperidine, *N-*methylpiperidine, azepane, azocane, [1,3]dioxane, oxazolidine, piperazine, homopiperazine, morpholine, thiomorpholine, 1,2,3,6-tetrahydropyridine and the like. Other examples of heterocyclic rings include the oxidized forms of the sulfur-containing rings. Thus, tetrahydrothiophene-1-oxide, tetrahydrothiophene-1,1-dioxide, thiomorpholine-1-oxide, thiomorpholine-1,1-dioxide, tetrahydrothiopyran-1-oxide, tetrahydrothiopyran-1,1-dioxide, thiazolidine-1-oxide, and thiazolidine-1,1-dioxide are also considered to be heterocyclic rings. The term "heterocyclic" also includes fused ring systems and can include a carbocyclic ring that is partially or fully unsaturated, such as a benzene ring, to form benzofused heterocycles. For example, 3,4-dihydro-1,4-benzodioxine, tetrahydroquinoline, tetrahydroisoquinoline and the like.

Compounds described herein may contain one or more asymmetric centers and may thus give rise to diastereomers and optical isomers. The present invention includes all such possible diastereomers as well as their racemic mixtures, their substantially pure resolved enantiomers, all possible geometric isomers, and pharmaceutically acceptable salts thereof. The above Formula (I) is shown without a definitive stereochemistry at certain positions. The present invention includes all stereoisomers of Formula (I) and pharmaceutically acceptable salts thereof. Further, mixtures of stereoisomers as well as isolated specific stereoisomers are also included. During the course of the synthetic procedures used to prepare such compounds, or in using racemization or epimerization procedures known to those skilled in the art, the products of such procedures can be a mixture of stereoisomers.

When a tautomer of the compound of Formula (I) exists, the present invention includes any possible tautomers and pharmaceutically acceptable salts thereof, and mixtures thereof, except where specifically drawn or stated otherwise.

When the compound of Formula (I) and pharmaceutically acceptable salts thereof exist in the form of solvates or polymorphic forms, the present invention includes any possible solvates and polymorphic forms. A type of a solvent that forms the solvate is not particularly limited so long as the solvent is pharmacologically acceptable. For example, water, ethanol, propanol, acetone or the like can be used.

The invention also encompasses a pharmaceutical composition that is comprised of a compound of Formula (I) in combination with a pharmaceutically acceptable carrier.

Preferably the composition is comprised of a pharmaceutically acceptable carrier and a non-toxic therapeutically effective amount of a compound of Formula (I) as described above (or a pharmaceutically acceptable salt thereof).

Moreover, within this preferred embodiment, the invention encompasses a pharmaceutical composition for use in the treatment of disease by inhibiting glycogen phosphorylase, resulting in the prophylactic or therapeutic treatment of diabetes, hyperglycemia, hypercholesterolemia, hyperinsulinemia, hyperlipidemia, hypertension, atherosclerosis or tissue ischemia e.g. myocardial ischemia comprising a pharmaceutically acceptable carrier and a non-toxic therapeutically effective amount of compound of Formula (I) as described above (or a pharmaceutically acceptable salt thereof).

The term "pharmaceutically acceptable salts" refers to salts prepared from pharmaceutically acceptable non-toxic bases or acids. When the compound of the present invention is acidic, its corresponding salt can be conveniently prepared from pharmaceutically acceptable non-toxic bases, including inorganic bases and organic bases. Salts derived from such inorganic bases include aluminum, ammonium, calcium, copper (ic and ous), ferric, ferrous, lithium, magnesium, potassium, sodium, zinc and the like salts. Particularly preferred are the ammonium, calcium, magnesium, potassium and sodium salts. Salts derived from pharmaceutically acceptable organic non-toxic bases include salts of primary, secondary, and tertiary amines, as well as cyclic amines and substituted amines such as naturally occurring and synthesized substituted amines. Other pharmaceutically acceptable organic non-toxic bases from which salts can be formed include arginine, betaine, caffeine, choline, *N'N'*-dibenzylethylenediamine, diethylamine, 2-diethylaminoethanol,2-dimethylaminoethanol, ethanolamine, ethylenediamine, *N*-ethylmotpholine, *N*-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like.

When the compound of the present invention is basic, its corresponding salt can be conveniently prepared from pharmaceutically acceptable non-toxic acids, including inorganic and organic acids. Such acids include, for example, acetic, benzenesulfonic, benzoic, camphorsulfonic, citric, ethanesulfonic, fumaric, gluconic, glutamic, hydrobromic, hydrochloric, isethionic, lactic, maleic, malic, mandelic, methanesulfonic, mucic, nitric, pamoic, pantothenic, phosphoric, succinic, sulfuric, tartaric, p-toluenesulfonic acid and the like. Particularly preferred are citric, hydrobromic, hydrochloric, maleic, phosphoric, sulfuric and tartaric acids.

Since the compounds of Formula (I) are intended for pharmaceutical use they are preferably provided in substantially pure form, for example at least 60% pure, more suitably at least 75% pure especially at least 98% pure (% are on a weight for weight basis).

The pharmaceutical compositions of the present invention comprise a compound represented by Formula (I) (or a pharmaceutically acceptable salt thereof) as an active ingredient, a pharmaceutically acceptable carrier and optionally other therapeutic ingredients or adjuvants. The compositions include those suitable for oral, rectal, topical, and parenteral (including subcutaneous, intramuscular, and intravenous) administration, although the most suitable route in any given case will depend on the particular host, and nature and severity of the conditions for which the active ingredient is being administered. The pharmaceutical compositions may be conveniently presented in unit dosage form and prepared by any of the methods well known in the art of pharmacy.

In practice, the compounds represented by Formula (I), or pharmaceutically acceptable salts thereof, of this invention can be combined as the active ingredient in intimate admixture with a pharmaceutical carrier according to conventional pharmaceutical compounding techniques. The carrier may take a wide variety of forms depending on the form of preparation desired for administration, e.g. oral or parenteral (including intravenous). Thus, the pharmaceutical compositions of the present invention can be presented as discrete units suitable for oral administration such as capsules, sachets or tablets each containing a predetermined amount of the active ingredient. Further, the compositions can be presented as a powder, as granules, as a solution, as a suspension in an aqueous liquid, as a non-aqueous liquid, as an oil-in-water emulsion, or as a water-in-oil liquid emulsion. In addition to the common dosage forms set out above, the compound represented by Formula (I), or a pharmaceutically acceptable salt thereof, may also be administered by controlled release means and/or delivery devices. The compositions may be prepared by any of the methods of pharmacy. In general, such methods include a step of bringing into association the active ingredient with the carrier that constitutes one or more necessary ingredients. In general, the compositions are prepared by uniformly and intimately admixing the active ingredient with liquid carriers or finely divided solid carriers or both. The product can then be conveniently shaped into the desired presentation.

Thus, the pharmaceutical compositions of this invention may include a pharmaceutically acceptable carrier and a compound or a pharmaceutically acceptable salt of Formula (I). The compounds of Formula (I), or pharmaceutically acceptable salts thereof, can also be included in pharmaceutical compositions in combination with one or more other therapeutically active compounds.

The pharmaceutical carrier employed can be, for example, a solid, liquid, or gas. Examples of solid carriers include lactose, terra alba, sucrose, talc, gelatin, agar, pectin, acacia, magnesium stearate, and stearic acid. Examples of liquid carriers are sugar syrup, peanut oil, olive oil, and water. Examples of gaseous carriers include carbon dioxide and nitrogen.

In preparing the compositions for oral dosage form, any convenient pharmaceutical media may be employed. For example, water, glycols, oils, alcohols, flavoring agents, preservatives, coloring agents, and the like may be used to form oral liquid preparations such as suspensions, elixirs and solutions; while carriers such as starches, sugars, microcrystalline cellulose, diluents, granulating agents, lubricants, binders, disintegrating agents, and the like may be used to form oral solid preparations such as powders, capsules and tablets. Because of their ease of administration, tablets and capsules are the preferred oral dosage units whereby solid pharmaceutical carriers are employed. Optionally, tablets may be coated by standard aqueous or nonaqueous techniques.

A tablet containing the composition of this invention may be prepared by compression or molding, optionally with one or more accessory ingredients or adjuvants. Compressed tablets may be prepared by compressing, in a suitable machine, the active ingredient in a free-flowing form such as powder or granules, optionally mixed with a binder, lubricant, inert diluent, surface active or dispersing agent. Molded tablets may be made by molding in a suitable machine, a mixture of the powdered compound moistened with an inert liquid diluent. Each tablet preferably contains from about 0.05mg to about 5g of the active ingredient and each sachet or capsule preferably contains from about 0.05mg to about 5g of the active ingredient.

For example, a formulation intended for oral administration to humans may contain from about 0.5mg to about 5g of active agent, compounded with an appropriate and convenient amount of carrier material, which may vary from about 5 to about 95 percent of the total composition. Unit dosage forms will generally contain from about 1mg to about 2g of the active ingredient, typically 25mg, 50mg, 100mg, 200mg, 300mg, 400mg, 500mg, 600mg, 800mg, or 1000mg.

Pharmaceutical compositions of the present invention suitable for parenteral administration may be prepared as solutions or suspensions of the active compounds in water. A suitable surfactant can be included such as, for example, hydroxypropylcellulose. Dispersions can also be prepared in glycerol, liquid polyethylene glycols, and mixtures thereof in oils. Further, a preservative can be included to prevent the detrimental growth of microorganisms.

Pharmaceutical compositions of the present invention suitable for injectable use include sterile aqueous solutions or dispersions. Furthermore, the compositions can be in the form of sterile powders for the extemporaneous preparation of such sterile injectable solutions or dispersions. In all cases, the final injectable form must be sterile and must be effectively fluid for easy syringability. The pharmaceutical compositions must be stable under the conditions of manufacture and storage; thus, preferably should be preserved against the contaminating action of microorganisms such as bacteria and fungi. The carrier can be a solvent or dispersion medium containing, for example, water, ethanol, polyol (e.g. glycerol, propylene glycol and liquid polyethylene glycol), vegetable oils, and suitable mixtures thereof.

Pharmaceutical compositions of the present invention can be in a form suitable for topical use such as, for example, an aerosol, cream, ointment, lotion, dusting powder, or the like. Further, the compositions can be in a form suitable for use in transdermal devices. These formulations may be prepared, utilizing a compound represented by Formula (I), or a pharmaceutically acceptable salt thereof, via conventional processing methods. As an example, a cream or ointment is prepared by admixing hydrophilic material and water, together with about 5wt% to about 10wt% of the compound, to produce a cream or ointment having a desired consistency.

Pharmaceutical compositions of this invention can be in a form suitable for rectal administration wherein the carrier is a solid. It is preferable that the mixture forms unit dose suppositories. Suitable carriers include cocoa butter and other materials commonly used in the art. The suppositories may be conveniently formed by first admixing the composition with the softened or melted carrier(s) followed by chilling and shaping in molds.

In addition to the aforementioned carrier ingredients, the pharmaceutical formulations described above may include, as appropriate, one or more additional carrier ingredients such as diluents, buffers, flavoring agents, binders, surface-active agents, thickeners, lubricants, preservatives (including anti-oxidants) and the like. Furthermore, other adjuvants can be included to render the formulation isotonic with the blood of the intended recipient. Compositions containing a compound of Formula (I), or pharmaceutically acceptable salts thereof, may also be prepared in powder or liquid concentrate form.

Generally, dosage levels on the order of 0.01mg/kg to about 150mg/kg of body weight per day are useful in the treatment of the above-indicated conditions, or alternatively about 0.5mg to about 7g per patient per day. For example, diabetes and hyperglycemia may be effectively treated by the administration of from about 0.01 to 50mg of the compound per kilogram of body weight per day, or alternatively about 0.5mg to about 3.5g per patient per day. Similarly, hypercholesterolemia, hyperinsulinemia, hyperlipidemia, hypertension, atherosclerosis or tissue ischemia e.g. myocardial ischemia may be effectively treated by the administration of from about 0.01 to 50mg of the compound per kilogram of body weight per day, or alternatively about 0.5mg to about 3.5g per patient per day.

It is understood, however, that the specific dose level for any particular patient will depend upon a variety of factors including the age, body weight, general health, sex, diet, time of administration, route of administration, rate of excretion, drug combination and the severity of the particular disease undergoing therapy.

The compounds of Formula (I) may be used in the treatment of diseases or conditions in which glycogen phosphorylase plays a role.

Thus the invention also provides a compound of Formula (I), or a pharmaceutically acceptable salt thereof, for use in the treatment of a disease or condition in which glycogen phosphorylase plays a role.

Diseases or conditions in which glycogen phosphorylase plays a role include diabetes (including Type I and Type II, impared glucose tolerance, insulin resistance and diabetic complications such as neuropathy, nephropathy, retinopathy and cataracts), hyperglycemia, hypercholesterolemia, hyperinsulinemia, hyperlipidemia, hypertension, atherosclerosis, tissue ischemia e.g. myocardial ischemia

The invention also provides a compound of Formula (I), or a pharmaceutically acceptable salt thereof, for use in the treatment of hyperglycemia or diabetes.

The invention also provides a compound of Formula (I), or a pharmaceutically acceptable salt thereof, for use in the prevention of diabetes in a human demonstrating pre-diabetic hyperglycemia or impaired glucose tolerance.

The invention also provides a compound of Formula (I), or a pharmaceutically acceptable salt thereof, for use in the treatment of hypercholesterolemia, hyperinsulinemia, hyperlipidemia, hypertension, atherosclerosis or tissue ischemia.

The invention also provides a compound of Formula (I), or a pharmaceutically acceptable salt thereof, for use as a cardioprotectant e.g. following reperfusion injury.

The invention also provides the use of a compound of Formula (I), or a pharmaceutically acceptable salt thereof, in the manufacture of a medicament for the treatment of a condition as defined above.

In the invention the term "treatment" includes both therapeutic and prophylactic treatment.

The compounds of Formula (I), or pharmaceutically acceptable salts thereof, may be administered alone or in combination with one or more other therapeutically active compounds. The other therapeutically active compounds may be for the treatment of the same disease or condition as the compounds of Formula (I) or a different disease or condition. The therapeutically active compounds may be administered simultaneously, sequentially or separately.

The compounds of Formula (I) may be administered with other active compounds for the treatment of diabetes, for example insulin and insulin analogs, sulfonyl ureas and analogs, biguanides, α2 agonists, fatty acid oxidation inhibitors, α-glucosidase inhibitors, β-agonists, phosphodiesterase inhibitors, lipid lowering agents, antiobesity agents, amylin antagonists, lipoxygenase inhibitors, somostatin analogs, glucokinase activators, glucagon antagonists, insulin signalling agonists, PTP1B inhibitors, gluconeogenesis inhibitors, antilypolitic agents, GSK inhibitors, galanin receptor agonists, anorectic agents, CCK receptor agonists, leptin, CRF antagonists or CRF binding proteins.

The compounds of Formula (I) may also be administered in combination with thyromimetic compounds, aldose reductase inhibitors, glucocorticoid receptor antagonists, NHE-1 inhibitors or sorbitol dehydrogenase inhibitors.

The compounds of Formula (I) may exhibit advantageous properties compared to known glycogen phosphorylase inhinbitors, for example, the compounds may exhibit improved solubility thus improving absorption properties and bioavailability.

All publications, including, but not limited to, patents and patent applications cited in this specification, are herein incorporated by reference as if each individual publication were specifically and individually indicated to be incorporated by reference herein as fully set forth.

In accordance with this invention, the compounds of Formula (I) can be prepared as outlined in Scheme 1 below wherein R¹, R^{1'}, R², R³**,** R^{3'}, R⁴, R⁵**,** X₁, X₂, X₃, X₄, Y, A, ---- and n are as defined above for Formula (I):

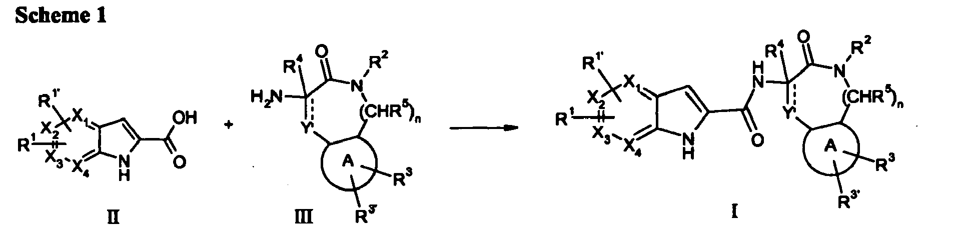

According to Scheme 1, the compounds of Formula (I) may be prepared by coupling the appropriate pyrrolopyridine-2-carboxylic acid of Formula (II), or a protected or activated derivative thereof, with the appropriate amine of Formula (III). Typically, the compound of Formula (II), or a protected or activated derivative thereof, is combined with compounds of Formula (III) in the presence of a suitable coupling agent. Examples of suitable coupling reagents are 1-(3-dimethylaminopropyl)-3-ethylcarbodiimide hydrochloride/hydroxybenzotriazole (EDCI /HOBt), 1,1-carbonyldiimidazole (CDI), dicyclohexylcarbodiimide/ hydroxybenzotriazole (DCC /HOBt), *O-(1H-*benzotriazol-1-yl)-*N,N,N,N'*-tetramethyluronium tetrafluoroborate (R. Knorr et al., Tetrahedron Lett., 1989, 30, 1927-1930) and polymer supported carbodiimide-1-hydroxybenzotriazole (for representative procedures, see for example, Argonaut Technical Note 501 available from Argonaut Technologies, Inc., Foster City, California). The couplings are performed in an inert solvent, preferably an aprotic solvent at a temperature of about 0°C to about 45°C for about 1 to 72h in the presence of a tertiary amine base such as diisopropylethylamine (DIPEA) or triethylamine. Exemplary solvents include acetonitrile, chloroform, dichloromethane, *N,N-*dimethylformamide (DMF) or mixtures thereof. Use of these coupling agents and appropriate selection of solvents and temperatures are known to those skilled in the art or can be readily determined from the literature. These and other exemplary conditions useful for coupling carboxylic acids are described in Houben-Weyl, Vol XV, part II, E. Wunsch, Ed., G. Thieme Verlag, 1974, Stuttgart, and M. Bodansky, Principles of Peptide Synthesis, Springer-Verlag, Berlin, 1984 and The Peptides, Analysis, Synthesis and Biology (Ed., E. Gross and J. Meienhofer), Vols 1-5, Academic Press NY 1979-1983.

Compounds of Formula (II) can be obtained by the synthesis outlined in Scheme 2 below.

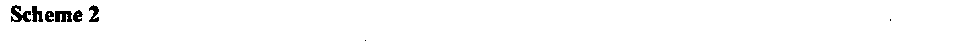

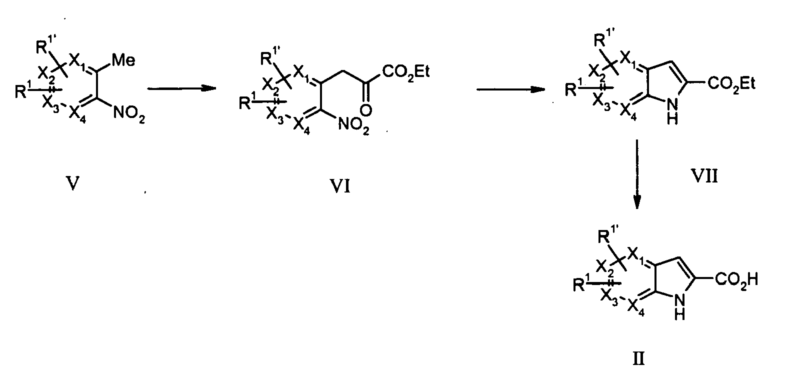

Compounds of Formula (VI) may be prepared by condensation of *ortho* methyl nitro compounds of Formula (V) with an oxalate ester in a solvent such as diethyl ether in the presence of a base such as potassium ethoxide or DBU. Compounds of Formula (VII) are prepared from compounds of Formula (VI) under reducing conditions, such as iron powder and ammonium chloride, or by hydrogenation in ethanol using palladium catalysis. Compounds of Formula (VII) undergo ester hydrolysis using aqueous alkali to give pyrrolopyridine-2-carboxylic acids of Formula (II). Further information on the conversion of compounds of Formula (V) to compounds of Formula (II) are described in the literature (Kermack, et al., J. Chem, Soc., (1921), 119, 1602; Cannon et al., J. Med. Chem., (1981), 24, 238; Julian et al., in Heterocyclic Compounds, Vol 3 (Wiley, New York, NY, 1962, R.C. Elderfield, Ed.) p 18).

Alternatively, the compound of Formula (VII) wherein X₂ is nitrogen can be prepared as outlined in Scheme 3.

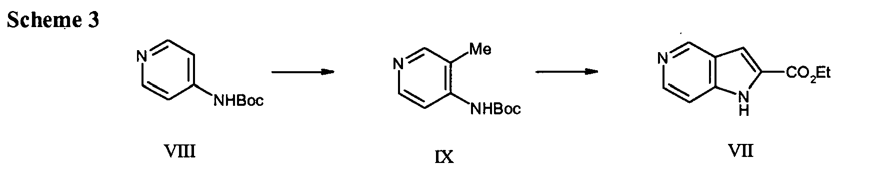

Deprotonation of compounds of Formula (VIII) with an organolithium such as n-butyllithium in a suitable solvent such as THF, followed by quenching with methyl iodide gives compounds of Formula (IX). Such compounds can undergo further deprotonation with *tert*-butyllithium, in a suitable solvent such as THF, followed by quenching with diethyl oxalate and subsequent heating of the intermediate under reflux in hydrochloric acid, to give compounds of Formula (VII).

Compounds of Formula (II) may also be prepared according to Scheme 4 by Heck coupling of an *ortho*-iodo aminopyridine (XI) followed by cyclisation at a temperature of between 100 to 150°C in the presence of catalyst such as palladium acetate and a base such as DABCO in a solvent such as DMF (see Chen et al, J. Org. Chem. 1997, 62, 2676). The *ortho*-iodo aminopyridines (XI) can be made by direct iodination of the appropriate aminopyridine (X) using iodine in the presence of silver sulfate in a solvent such as ethanol at ambient temperature (see Sy, W., Synth. Commun., 1992, 22, 3215).

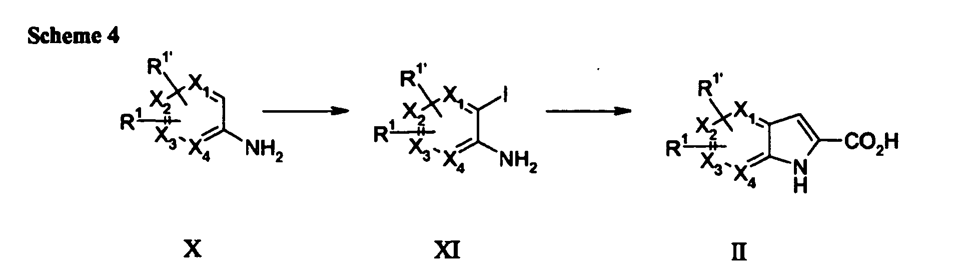

Alternatively compounds of Formula (XI) may be prepared according to Scheme 5 by deprotection of N-pivaloyl compounds (XII) by heating under reflux using hydrochloric acid. The N-pivaloyl compounds (XII) are in turn made by deprotonation of compounds of Formula (XIII) with an organolithium such as *tert*-butyllithium in a suitable solvent such as THF, followed by quenching with iodine at a low temperature. Compounds of formula (XIII) may be made by protection of commercially available aminopyridines (X) with trimethylacetyl chloride and a base such as triethylamine in a solvent such as dichloromethane.

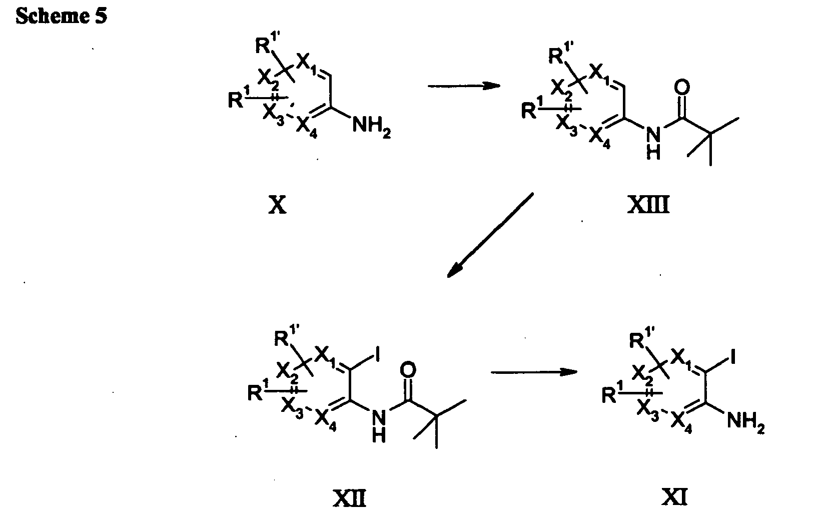

Alternatively compounds of Formula (XI) may be prepared according to Scheme 6 by deprotection of N-BOC protected compounds (XIV) using an acid such as trifluoroacetic acid in a solvent such as dichloromethane at ambient temperature. The N-BOC compounds (XIV) are in turn made by deprotonation of compounds of Formula (XV) with an organolithium such as *n*-butyllithium in the presence of N,N,N',N'-tetramethylethylenediamine (TMEDA) in a suitable solvent such as ether at temperatures around -70°C followed by the addition of iodine at temperatures around -10°C. The N-BOC aminopyridines (XV) are routinely made from the commercially available aminopyridines (X) using di-*tert*-butyldicarbonate by heating in a solvent such as 1,4-dioxane.

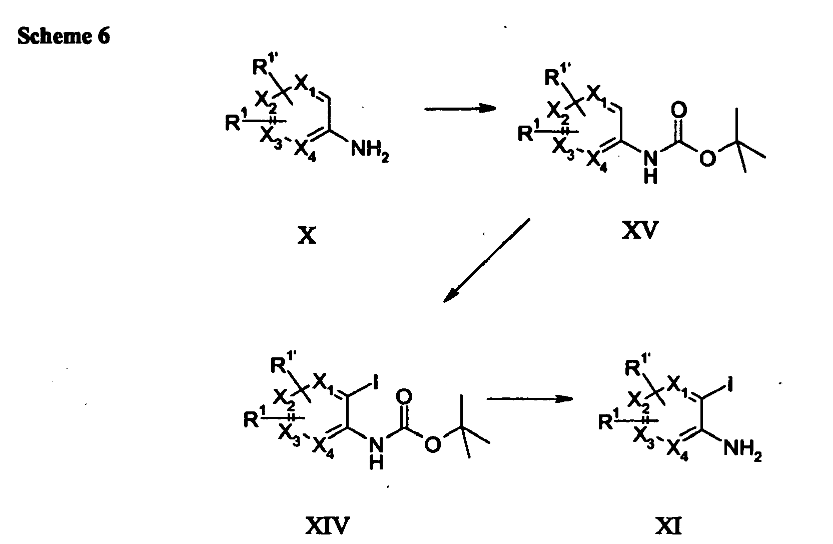

Protected or activated derivatives of the compounds of Formula (II) may be prepared by methods known to those skilled in the art.

Compounds of Formula (III) may be prepared by reacting an amine of Formula (XVI): 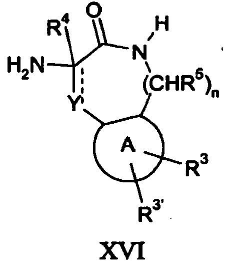 with R²-L, where L is a leaving group (for example chloro, bromo or iodo) in the presence of a base such as sodium hydride in a suitable solvent such as DMF.

Compounds of Formula (XVI) where A is phenylene, n is 0, R⁴ is hydrogen, Y is CH₂ and ---- is a single bond may be prepared from 3-amino-3,4-dihydroquinolin-2-(1H)-one (J. Med. Chem., (1985), 28, 1511-1516). Compounds of Formula (XVI) where A is phenylene and ---- is a double bond may be prepared by reductive cyclisation of a compound of Formula (XVII) using e.g. tin (II) chloride in HC1, followed by removal of the Boc protecting group, using e.g. trifluoroacetic acid. Compounds of Formula (XVII) may be prepared by reaction of a compound of Formula (XVIII) with a compound of Formula (XIX) in the presence of a base, e.g. tetramethylguanidine.

Compounds of Formula (XVIII) are commercially available or described in the literature.

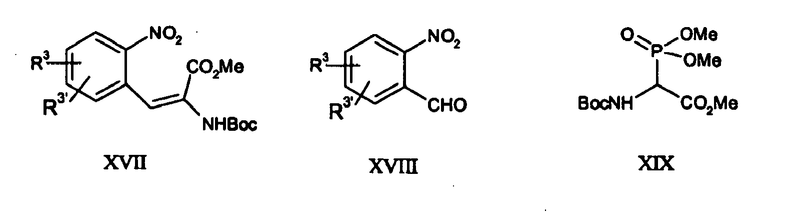

Compounds of Formula (III) wherein A is heteroarylene can be prepared from cyclisation of suitably functionalised heteroaryls when ---- is a single bond suitable methods for the synthesis of such compounds are as described in U.S. Patent Application No. US2004/0002495. For example when ring A is: 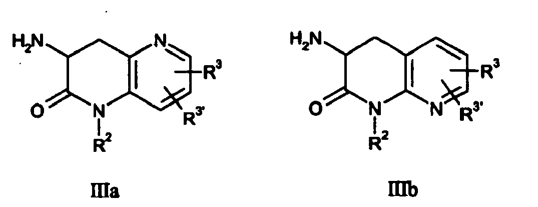 compounds of Formula (IIIa) and (IIIb) may be prepared from an appropriately substituted 3-nitro-2-methylpyridine or 2-aminopyridine according to Schemes 7 and 8.

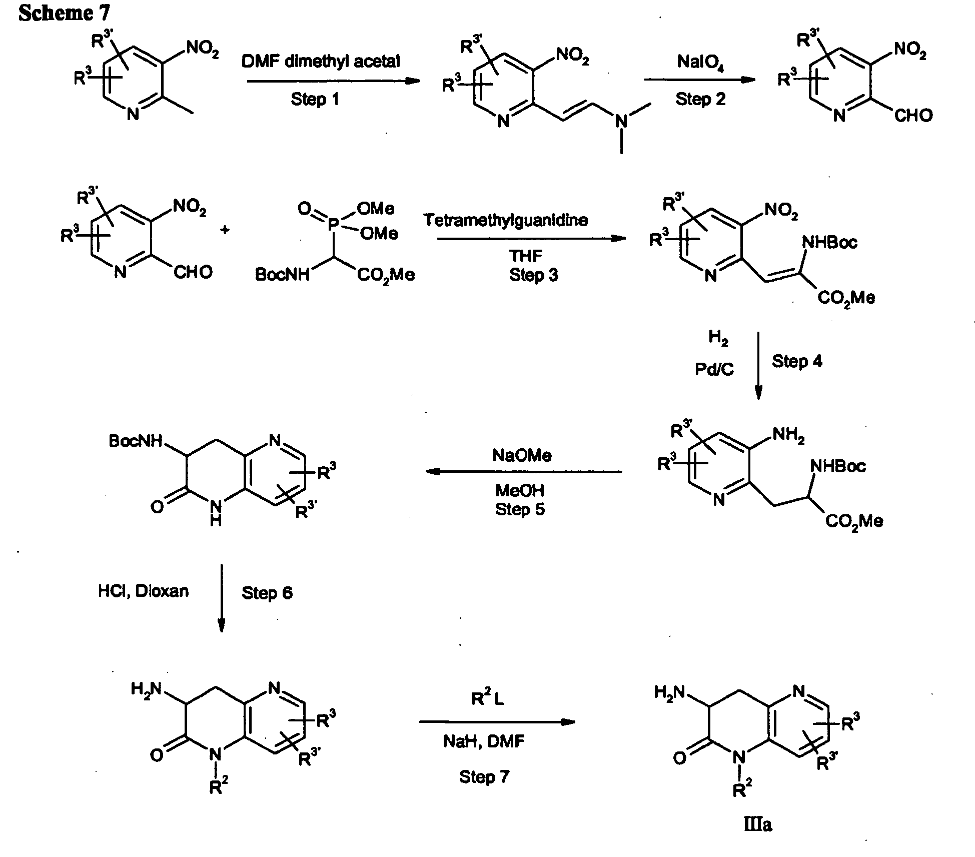

Steps 1 and 2 may be carried out according to the process described in Tetrahedron (1998), 54(23), 6311-6318. Step 3 may be carried out according to the method described in Synthesis (1992), 5, 487. Asymmetric hydrogenation reactions of olefins as shown in Step 4 are well known (see e.g. JACS, (1993), 115, 10125) and lead to homochiral final products. Step 5 may alternatively be carried out by hydrolysisng the ester, activating the resulting acid with a carbodiimide such as EDCI or DCC, or by preparing an acid chloride, or activated ester such as an N-hydroxysuccinimide ester. Suitable bases are organic bases such as triethylamine or diisopropylamine (DIPEA) or 1,8-diazabicylo[5.4.0]undec-7-ene (DBU). In Step 6 alternative solvents such as dichloromethane or other acids such as trifluoroacetic acid may be used. In Step 7, L is a leaving group, for example Cl, Br, I, or OMs.

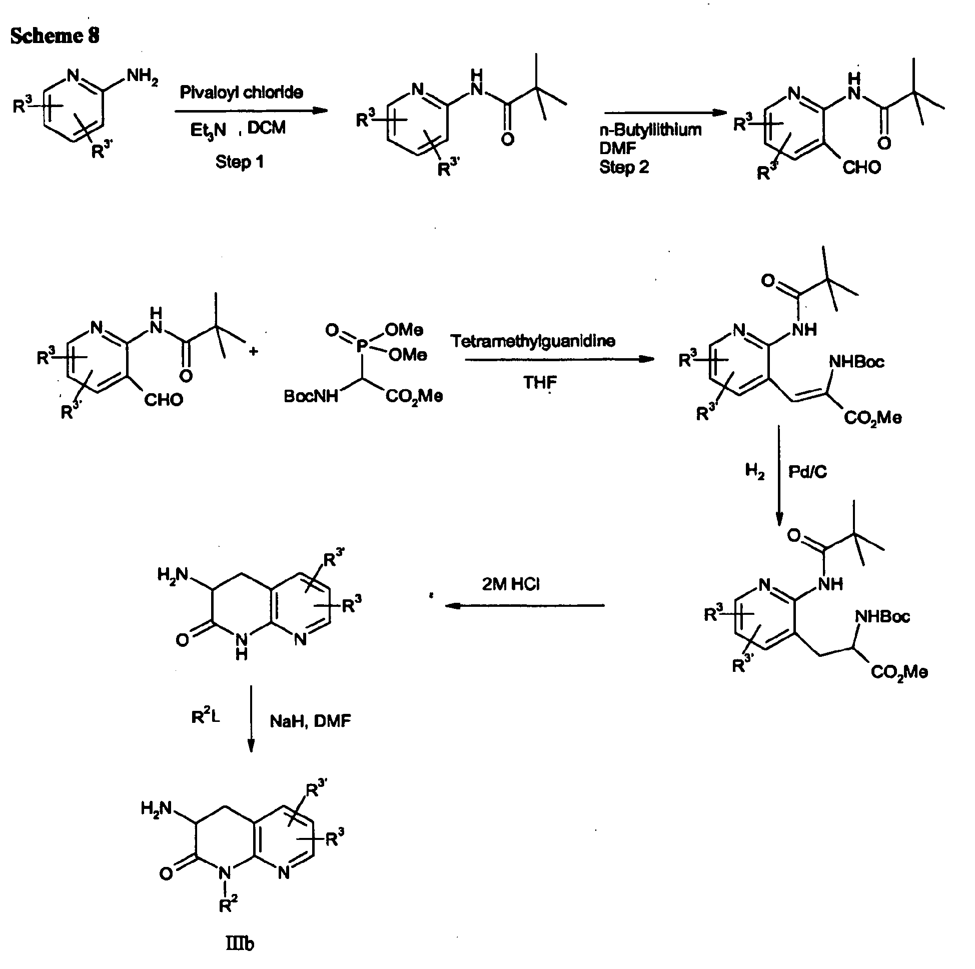

Steps 1 and 2 are described in JOC , (1983), 48, 3401-3408.

The processes described above and shown in Schemes 7 and 8 may also be used for the preparation of other isomeric pyridines or six membered heteroaryls containing more than one nitrogen.

Compounds of Formula (XVI) wherein A is heteroarylene and there is a bridgehead nitrogen, for example a compound of Formula (XVIa), may be prepared by cyclisation of a compound of Formula (XX): 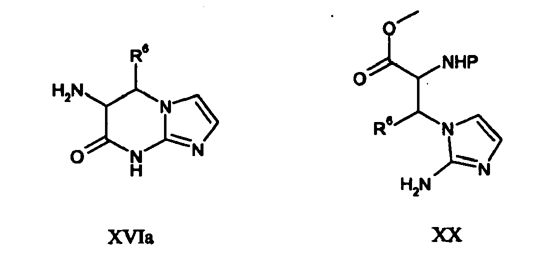 wherein P is an amino protecting group such as triphenylmethyl. The transformation may be induced by heating compounds of Formula (XX) under reflux in a solvent, for example, ethanol.

Compounds of Formula (XX) may be prepared from compounds of Formula (XXI) by hydrogenation using a catalyst such as Pd/C at ambient temperature.

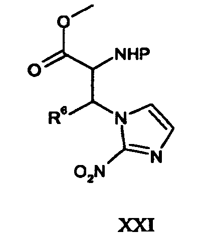

Compounds of Formula (XXI) may be prepared from compounds of Formulae (XXII) and (XXIII) using conditions known for the Mitsonobu reaction (Bull. Chem. Soc. Jpn., (1967), 40, 2380).

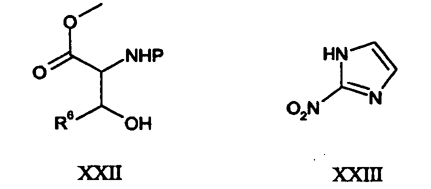

Compounds of Formulae (XXII) and (XXIII) are commercially available.

Compounds of Formula (XVI) wherein A is heteroarylene and there is a bridgehead heteroatom, for example, compounds of Formula (XVIb), may be made by analogous processes to that for making compounds of Formula (XVIa).

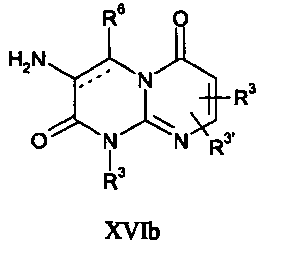

Various ring substituents in the compounds of the present invention, for example R³ and R^{3'}, may be introduced by standard aromatic substitution reactions or generated by conventional functional group modifications either prior to or following the processes described above. Such reactions and modifications include, for example, introduction of a substituent by means of an aromatic substitution reaction, reduction of substituents, alkylation of substituents and oxidation of substituents. Particular examples of aromatic substitution reactions include the introduction of a nitro group using concentrated nitric acid, the introduction of an acyl group using, for example, an acyl halide and Lewis acid (such as aluminium trichloride) under Friedal Crafts conditions; the introduction of an alkyl group using, for example, an alkyl halide and Lewis acid (such as aluminium trichloride) under Friedal Crafts conditions; and the introduction of a halogen group. Particular examples of modifications include the reduction of a nitro group to an amino group by for example, catalytic hydrogenation with a Nickel catalyst or treatment with iron in the presence of hydrochloric acid with heating; and oxidation of alkylthio to alkylsulfinyl or alkylsulfonyl.

The compounds of Formula (I) may be prepared singly or as compound libraries comprising at least 2, for example 5 to 1,000 compounds and more preferably 10 to 100 compounds of Formula (I). Compound libraries may be prepared by a combinatorial "split and mix" approach or by multiple parallel synthesis using either solution or solid phase chemistry, using procedures known to those skilled in the art.

During the synthesis of the compounds of Formula (I), labile functional groups in the intermediate compounds, e.g. hydroxy, carboxy and amino groups, may be protected. The compounds of Formula (II) may be protected in the 1-position e.g. with an arylmethyl, acyl, alkoxycarbonyl, sulfonyl or silyl group. The protecting groups may be removed at any stage in the synthesis of the compounds of Formula (I) or may be present on the final compound of Formula (I). A comprehensive discussion of the ways in which various labile functional groups may be protected and methods for cleaving the resulting protected derivatives is given in for example, Protective Groups in Organic Chemistry, T.W. Greene and P.G.M. Wuts, (1991) Wiley-Interscience, New York, 2nd edition.

Any novel intermediates as defmed above, e.g. intermediates of Formula (III), are also included within the scope of the invention.

### EXPERIMENTAL

### Materials & methods

Column chromatography was carried out on SiO₂ (40-63 mesh). LCMS data were obtained using a Waters Symmetry 3.5µ C₁₈ column (2.1 x 30.0mm, flow rate = 0.8mL/min) eluting with a (5% MeCN in H₂O)-MeCN solution containing 0.1% HCH₂H over 6min and UV detection at 220nm. Gradient information: 0.0-1.2main: 100% (5% MeCN in H₂O); 1.2-3.8min: ramp up to 10% (5% MeCN in H₂O)-90% MeCN; 3.8-4.4min: hold at 10% (5% MeCN in H₂O)-90% MeCN; 4.4-5.5min: ramp up to 100% MeCN; 5.5-6.0min: return to 100% (5% MeCN in H₂O). The mass spectra were obtained employing an electrospray ionisation source in the positive (ES⁺) ion mode. NMR spectra were acquired at 27°C on a Varian Mercury 400 spectrometer operating at 400 MHz or on a Bruker AMX2 500 spectrometer operating at 500MHz. Mass directed purification was performed on a Micromass Platform LC with cone voltage 30v, employing an electrospray ionisation source in the positive (ES⁺) ion mode, Waters 996 Photodiode Array Detector (210-390nm), Xterra Prep MS, C₁₈, 5µ 19x50mm columns, and a mobile Phase of MeCN + 0.1% Formic Acid / H₂0+5%MeCN+0.1% Formic Acid

Abbreviations and acronyms: DABCO: 1,4-Diazabicyclo [2.2.2] octane; DCM: Dichloromethane; DIPEA: *N,N*-Diisopropylethylamine; DMA: *N,N-*Dimethylacetamide; DMF: *N,N-*Dimethylformamide; DMSO: Dimethylsulfoxide; DMTMM: 4-(4,6-Dimethoxy[1.3.5]triazin-2-yl)-4-methylmorpholinium chloride hydrate; EDCI: 1-(3-Dimethylaminopropyl)-3-ethylcarbodiimide hydrochloride; EtOAc: Ethylacetate; GP: Glycogen Phosphorylase; HOBt: 1-Hydroxybenzotriazole; MgSO₄: Magnesium sulfate; NMP: N-Methytpyrrotidine; rt: Room temperature; RT: Retention time; TBAF: Tert-Butylammonium fluoride; THF: Tetrahydrofuran.

### Preparation 1 : 3-(2-Chloro-5-nitropyridin-4-yl)-2-oxopropionic acid ethyl ester

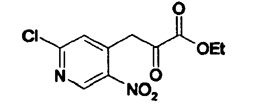

**Route A:** To a solution of potassium ethoxide (1.46g, 17.4mmol) in diethyl ether (80mL) and ethanol (10mL) under an argon atmosphere was added diethyl oxalate (2.4mL, 17.4mmol) and the mixture stirred at rt for 0.5h. A solution of 2-chloro-4-methyl-5-nitropyridine (3.0g, 17.4mmol) in diethyl ether (20mL) was added resulting in the formation of a dark green precipitate. The reaction was stirred at rt for 15h, cooled to 0°C, filtered and washed with cold diethyl ether to give a dark green solid. The solid was dissolved in water (200mL) and acidified to pH 4 with acetic acid to give an orange precipitate. The solid was collected by filtration and dried to give the title compound. m/z (ES⁺) = 273 [*M*+ H]⁺.

**Route B** : To a solution of 2-chloro-4-methyl-5-nitropyridine (1.0g, 5.8mmol) in diethyl oxalate (4.23g, 29mmol) under an argon atmosphere was added 1,8-diazabicyclo[5.4.0]undec-7-ene (0.95mL, 6.4mol). The mixture was stirred at rt for 1.5h then diluted with t-butyl methyl ether (40mL), water (30mL) and acetic acid (1mL). The organic layer was separated, washed with water, dried (MgS0₄) and evaporated to dryness. The resultant damp red solid residue was finally dried under high vacuum at 40-50°C to give the title compound.

### Preparation 2 : 5-Chloro-1H-pyrrolo[2,3-c]pyridine-2-carboxylic add ethyl ester

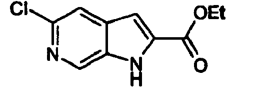

3-(2-Chloro-5-nitropyridin-4-yl)-2-oxopropionic acid ethyl ester (**Preparation 1**, 3.0g, 11.0mmol) was dissolved in ethanol (100mL) and THF (50mL). Iron powder (3.7g, 66.0mmol) and saturated ammonium chloride solution (50mL) were added and the mixture heated under reflux for 2h. The mixture was cooled, filtered through celite and washed several times with ethyl acetate. The organic layers were combined, washed with brine (100mL), dried (MgSO₄) and concentrated *in vacuo* to give the title compound as a brown solid. δ_{H} (CD₃OD): 1.42 (3H, t), 4.44 (2H, q), 7.15 (1H, s), 7.70 (1H, s), 8.59 (1H, s); m/z (ES⁺) = 225 [*M* + H]⁺.

### Preparation 3 : 5-Chloro-1H-pyrrolo[2,3-c] pyridine-2-carboxylic acid

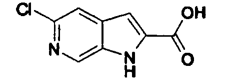

**Route A** : To a solution of 5-chloro-1*H*-pyrrolo[2,3-c]pyridine-2-carboxylic acid ethyl ester (**Preparation 2**,1.78g, 7.9mmol) in ethanol (70mL) was added sodium hydroxide solution (5.2mL, 2M, 10.3mmol) and the mixture heated under reflux for 2h. The solvent was removed *in vacuo* and the solid dissolved in water (150mL) and acidified to pH 4 with acetic acid to give the title compound as a brown solid that was isolated by filtration. δ_{H} (CD₃OD): 7.13 (1H, s), 7.68 (1H, s), 8.58 (1H, s); m/z (ES⁺) = 197 [M+ H]⁺.

**Route B** : A mixture of 6-chloro-4-iodopyridin-3-ylamine (**Preparation 8**, 0.33g, 1.30mmol), pyruvic acid (0.27mL, 3.89mmol), DABCO (0.44g, 3.89mmol) and palladium acetate (0.015g, 0.07mmol) in dry DMF was stirred vigorously and degassed with argon for 15min. The reaction mixture was heated to 107°C for 5h. The reaction mixture was allowed to cool to rt and stirred for 16h. The volatiles were removed under reduced pressure and the residue partitioned between EtOAc (100mL) and water (50mL). The layers were separated and the aqueous extracted with EtOAc (2x50mL). The combined organics were extracted with aqueous NaOH (2M, 3x70mL). The combined aqueous extracts were acidified to pH 4 by careful addition of glacial acetic acid, then extracted with EtOAc (3x60mL). The combined organics were washed with brine (50mL), dried (MgSO₄), filtered and concentrated *in vacuo* to give the title compound as a brown solid. RT=2.72min, m/z (ES⁺) =197 [*M*+ H]⁺.

### Preparation 4 : 5-Chloro-3-iodopyridin-2-ylamine

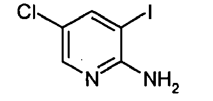

Silver sulfate (3.40g, 10.9mmol) and 2-amino-5-chloropyridine (1g, 7.8mmol) was added to a solution of iodine (2.76g, 10.9mmol) in ethanol (50mL) and the reaction mixture stirred at rt for 72h. The mixture was filtered, washed with methanol and the filtrate concentrated *in vacuo.* The residue was partitioned between saturated Na₂S₂O₃ solution (50mL) and DCM (2 x 50mL). The combined organics were dried (MgSO₄), concentrated *in vacuo* and purified by chromatography on silica gel eluting with DCM to give the title compound as a beige solid. δ_{H} (CDCl₃): 4.95 (2H, br s), 7.84 (1H, d), 7.98 (1H, d).

### Preparation 5 : 5-Chloro-1H-pyrrolo[2,3-b]pyridine-2-carboxylic acid

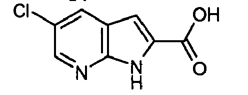

Pyruvic acid (0.43ml, 6.24mmol) was added to a solution of 5-chloro-3-iodopyridin-2-ylamine (**Preparation 4**, 500mg, 2.08mmol), palladium acetate (23mg, 0.10mmol) and DABCO (700mg, 6.24mmol) in anhydrous DMF (20mL). The reaction mixture was degassed with argon for 20min, then heated to 110°C for 16h. The solvent was removed *in vacuo* and the residue suspended in water (10mL) and acetic acid (5mL) and then filtered. The solid was dissolved in EtOAc (50mL), extracted into 2N NaOH solution (50mL) and the organic layer discarded. The aqueous solution was acidified with concentrated HCl and extracted into EtOAc (2x40mL). The combined organics were dried (MgSO₄) and concentrated *in vacuo* to give the title compound as a beige solid. δ_{H} (CD₃OD): 7.14 (1H, s), 8.14 (1H, d), 8.35 (1H, d).

### Preparation 6 : (6-Chloropyridin-3-yl)carbamic acid tert-butyl ester

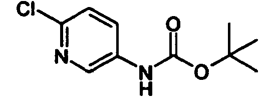

The title compound was prepared according to the method described in US 2002/0022624 A1. δ_{H} (CDCl₃): 1.52 (9H, s), 6.52 (1H, s), 7.26 (1H, d), 7.97 (1H, d), 8.23 (1H, d).

### Preparation 7 : (6-Chloro-4-iodopyridin-3-yl)carbamic acid tert-butyl ester

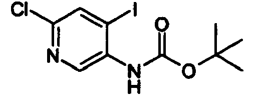

the title compound was prepared according to the method described in US 2002/0022624 A1 from the compound of **Preparation 6**. δ_{H}(CDCl₃): 1.54 (9H, s), 6.62 (1H, s), 7.72 (1H, s), 8.93 (1H, s).

**Preparation 8 : 6-Chloro-4-iodopyridin-3-ylamine**

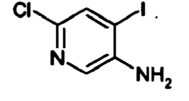

The title compound was prepared according to the method described in US 2002/0022624 A1 from the compound of **Preparation 7**. δ_{H} (CDCl₃): 4.12 (2H, br s), 7.60 (1H, s), 7.79 (1H, s).

### Preparation 9 : N-(6-Chloropyridin-2-yl)-2,2-dimethyl propionamide

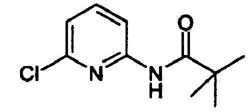

To a solution of 2-amino-6-chloropyridine (3.0g, 23.3mmol) in DCM (45mL) under argon was added triethylamine (4.10mL, 29.2mmol) and the reaction cooled to 0°C (ice bath). A solution of trimethylacetyl chloride (3.16mL, 25.7mmol) in DCM (10mL) was added dropwise over 20min before stirring for 30min at 0°C. The reaction was brought up to rt and stirred for a further 5h, then water (30mL) was added. The organics were separated and washed with Na₂CO₃ solution (2x50mL), dried (MgSO₄) and the solvent removed *in vacuo.* Purification by column chromatography (SiO₂, DCM) gave the title compound. m/z (ES⁺) = 213.04 [M + H]⁺.

### Preparation 10 : N-(6-Chloro-3-iodopyridin-2-yl)-2,2-dimethyl propionamide

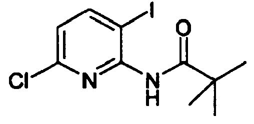

To a dry solution of N-(6-chloropyridin-2-yl)-2,2-dimethyl propionamide (**Preparation 9,** 8.0g, 37.6mmol) in THF (120mL), cooled to -78°C, was added dropwise, a solution of *tert-*butyllithium in pentane (1.7M, 48.7mL, 82.8mmol) over 40min. The reaction was stirred at -78°C for 3h before adding a solution of iodine (11.46g, 45.1mmol) in THF (40mL) dropwise. The mixture was brought up to rt and stirred for 16h. 2M HCl (30mL) was added to the reaction, and after 20min the solvent was removed *in vacuo.* Crude material was partitioned between EtOAc (200mL) and water (150mL). Organics were separated and washed with 10% sodium thiosulfate solution (4x100mL) then NaHCO₃ solution (2x100mL), dried (MgSO₄) and the solvent removed *in vacuo.* The residue was purified by column chromatography (SiO₂, CH₂Cl₂) to give the title compound. m/z (ES⁺) = 338.93 [*M*+H]⁺.

### Preparation 11 : 6-Chloro-3-iodopyridin-2-ylamine

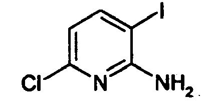

A suspension of N-(6-chloro-3-iodopyridin-2-yl)-2,2-dimethyl propionamide (**Preparation 10**, 5.0g, 14.8mmol) in 1M HCl was heated to reflux for 4.5h. The reaction was cooled to rt and then extracted with diethyl ether (2x50mL). The organics were washed with Na₂CO₃ solution (2x50mL) before being dried (MgSO₄) and the solvent removed *in vacuo.* Purification by column chromatography (SiO₂, DCM) afforded the title compound. δ_{H} (CDCl₃): 7.76 (1H, d), 6.46 (1H, d), 5.43-5.20 (2H, br s).

### Preparation 12 : 6-Chloro-1H-pyrrolo[2,3]pyridine-2-carboxylic acid

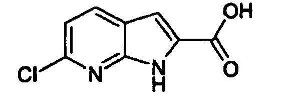

To a dry solution of 6-chloro-3-iodo-pyridin-2-ylamine (**Preparation 11**, 2.80g, 11.0mmol) in DMF (80mL) under argon was added pyruvic acid (2.29mL, 33.0mmol), DABCO (3.70g, 33.0mmol) then palladium(II)acetate (124mg, 0.55mmol) and the mixture purged with argon for 20min. The reaction was heated to 105°C (bath temp.) for 3h before being allowed to cool to rt. Solvent was removed *in vacuo* then crude material partitioned between EtOAc (100mL) and water (75mL). The organic layer was separated and washed with water (2x75mL) before being extracted into 2M NaOH (2x75mL). The aqueous layer was acidified to pH 3 with 2M HCl and extracted into EtOAc (2x100mL). Organic layers were combined, dried (MgSO₄) and concentrated *in vacuo.* The residue was suspended in water and the filtrate removed to give the title compound m/z (ES⁺) = 196.91 [M+ H]⁺; RT = 3.07min.

### Preparation 13 : 2-Chloro-5-iodopyridin-4-ylamine

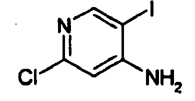

Silver sulfate (7.1g, 22.8mmol) and 4-amino-2-chloropyridine (4.06g, 31.6mmol) were added to a solution of iodine (5.65g, 22.3mmol) in ethanol (100mL) and the reaction mixture stirred at rt for 72h. The bright yellow suspension was filtered, washed with methanol and the filtrate concentrated *in vacuo.* The residue was partitioned between saturated Na₂CO₃ solution (200mL) and EtOAc (200ml). After separation the organic layer was washed with Na₂S₂O₃ solution (50mL, 25%) and brine (50mL), dried (MgSO₄), concentrated *in vacuo* and purified by chromatography on silica gel eluting with isohexane/EtOAc (3:1 to 2.5:1) to give the title compound. δ_{H} (CDCl₃): 4.81 (2H, br s), 6.63 (1H, s), 8.38 (1H, s); m/z (ES⁺) = 254.86 [*M* + H]⁺; RT = 2.51 min.

### Preparation 14: 6-Chloro-1H-pyrrolo[3,2-c]pyridine-2-carboxylic acid

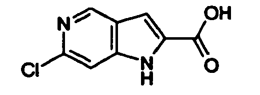

Pyruvic acid (0.86ml, 12.4mmol) was added to a solution of 2-chloro-5-iodopyridin-4-ylamine (**Preparation 13,** 1.05mg, 4.13mmol), palladium acetate (56mg, 0.25mmol) and DABCO (1.39g, 12.4mmol) in anhydrous DMF (30mL). The reaction mixture was degassed with argon for 20min, then heated to 145°C for 2h. The solvent was removed *in vacuo* and the residue taken up in water (200mL). The suspension was made alkaline (pH 9-10) with dilute NaOH solution (1M) and filtered through Celite. After washing of the filtrate with EtOAc (50mL) and ether (50mL) the pH was adjusted to 3 with dilute HCl solution (1M). Extraction with EtOAc (5x50mL), drying of the combined extracts (MgSO₄) and concentration gave the title compound. δ_{H} (d₆ DMSO): 7.24 (1H, s), 7.42 (1H, s), 8.80 (1H, s); m/z (ES) = 195.02 [*M*- H]⁻; RT = 2.36min.

### Preparation 15 : 3-Amino-3,4-dihydro-1H-quinolin-2-one

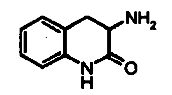

Diethyl acetamidomalonate (63.3g, 0.29mol) was added to a solution of sodium ethoxide (20.8g, 0.31mol) in ethanol (300mL) and the reaction mixture heated to 50°C for 15min. 2-Nitrobenzyl chloride (50g, 0.29mol) and potassium iodide (2.4g, 0.02mol) were added and the reaction mixture heated at 60°C for 3.5h. Water (300mL) was added to the reaction mixture and this was then concentrated by half *in vacuo.* The solid was filtered, washed with water and dried under vacuum affording 2-acetylamino-2-(2-nitrobenzyl)-malonic acid diethyl ester as a yellow solid. δ_{H} (d₆ DMSO): 1.13 (6H, t), 1.84 (3H, s), 3.81 (2H, s), 4.04-4.13 (4H, m), 7.22 (1H, dd), 7.50 (1H, td), 7.62 (1H, td), 7.86 (1H, dd), 8.11 (1H, s).

Saturated ammonium chloride solution (50mL) and iron powder (19g, 341mmol) were added to a solution of 2-acetylamino-2-(2-nitrobenzyl)malonic acid diethyl ester (30g, 85.1mmol) in ethanol (200mL) and THF (100mL). The reaction mixture was heated to reflux for 3.5h, then filtered through celite and washed several times with methanol. The solvent was removed *in vacuo* and the residue partitioned between water (300mL) and EtOAc (3x150mL). The combined organic fractions were dried (MgSO₄) and concentrated *in vacuo* affording 3-acetylamino-2-oxo-1,2,3,4-tetrahydroquinoline-3-carboxylic acid ethyl ester as a beige solid. m/z (ES⁺) 277 [M+H]⁺; RT = 2.70min.

3-Acetylamino-2-oxo-1,2,3,4-tetrahydroquinoline-3-carboxylic acid ethyl ester (18.5g, 67.0mmol) was dissolved in concentrated hydrochloric acid (100mL) and heated to reflux for 3h. The reaction mixture was diluted with water (300mL) and extracted into EtOAc (2x150mL). The combined organic fractions were discarded and the aqueous phase was basified with sodium hydroxide solution (12M) and extracted into EtOAc (3x150mL). The combined organic fractions were washed with brine (100mL), dried (MgSO₄) and concentrated *in vacuo* affording the title compound as a brown solid. m/z (ES⁺) 163 [M+H]⁺; RT = 2.44min.

### Preparation 16 : 3-Amino-1-[2-(tert-butyldimethylsilanyloxy)ethyl]-3,4-dihydro-1R-quinolin-2-one

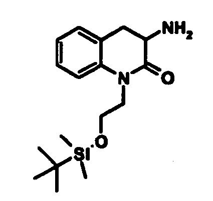

Sodium hydride (151mg, 3.78mmol) was added to a suspension of 3-amino-3,4-dihydro-1H-quinolin-2-one hydrochloride (**Preparation 15**, 300mg, 1.51mmol) in DMF (10mL) at 0°C over a period of 5min. After 1h, (2-bromoethoxy)-tert-butyldimethylsilane (0.39mL, 1.81mmol) was added and the reaction mixture stirred at rt for 16h, then 60°C for 3h. The reaction mixture was quenched with hydrochloric acid solution (1M, 3mL) and the solvent removed *in vacuo.* The residue was partitioned between saturated NaHCO₃ solution (20mL) and DCM (3x30mL). The combined organic fractions were dried (MgSO₄), concentrated *in vacuo* and purified by chromatography on silica gel eluting with methanol:DCM (1:19) affording the title compound as a yellow oil. δ_{H} (CDCl₃): 0.00 (3H, s), 0.02 (3H, s), 0.86 (9H, s), 1.85 (2H, br s), 2.84 (1H, t), 3.05 (1H, dd), 3.56 (1H, dd), 3.83-3.98 (3H, m), 4.15-4.21 (1H, m), 7.02 (1H, td), 7.18 (1H, d), 7.22-7.30 (2H, m).

### Preparation 17 : 3-Amino-1-(tetrahydrofuran-2-ylmethyl)-3,4-dihydro-1H-quinolin-2-one

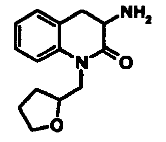

Sodium hydride (169mg, 4.23mmol) was added to a suspension of 3-amino-3,4-dihydro-1H-quinolin-2-one hydrochloride (**Preparation 15,** 400mg, 2.01mmol) in DMF (10mL) at 0°C over a period of 5min. After 1h, tetrahydrofurfuryl bromide (0.28mL, 2.21mmol) was added, and the reaction mixture stirred at rt for 16h. The solvent was removed *in vacuo* and the residue partitioned between saturated K₂CO₃ solution (30mL) and EtOAc (3x40mL). The combined organic fractions were dried (MgSO₄), concentrated *in vacuo* and purified by chromatography on silica gel eluting with methanol:DCM (1:24) affording the title compound as a yellow oil. m/z (ES⁺) 247 [M+H]⁺.

### Preparation 18 : 3-Amino-1-[2-(2-methoxyethoxy)ethyl]-3,4-dihydro-1H-quinolin-2-one

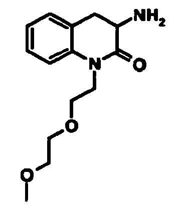

Sodium hydride (169mg, 4.23mmol) was added to a suspension of 3-amino-3,4-dihydro-1H-quinolin-2-one hydrochloride (**Preparation 15**, 400mg, 2.01mmol) in DMF (10mL) at 0°C over a period of 5min. After 1h 1-bromo-2-(2-methoxyethoxy)ethane (0.30mL, 2.21 mmol) was added, and the reaction mixture stirred at rt for 5 days. The solvent was removed *in vacuo* and the residue partitioned between saturated K₂CO₃ solution (40mL) and EtOAc (3 x 40mL). The combined organic fractions were dried (MgSO₄), concentrated *in vacuo* and purified by chromatography on silica gel eluting with methanol:DCM (1:19) affording the title compound as a yellow oil. δ_{H} (CDCl₃): 1.99 (2H, br s), 2.85 (1H, t), 3.06 (1H, dd), 3.37 (3H, s), 3.51-3.68 (5H, m), 3.74 (2H, t), 4.07 (1H, dt), 4.25 (1H, dt), 7.03 (1H, td), 7.18-7.30 (3H, m).

### Preparation 19 : 3-Amino-7-chloro-3,4-dihydro-1H-quinolin-2-one

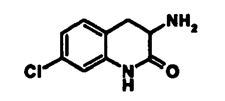

**Route A:** By a similar procedure to **Preparation 15**, the title compound was prepared using 4-chloro-2-nitrobenzyl chloride affording a peach solid. δ_{H} (CD₃OD): 2.85 (1H, t), 3.11 (1H, dd), 3.61 (1H, dd), 6.92 (1H, d), 6.99 (1H, dd), 7.20 (1H, d).

**Route B**: To a solution of 2-tert-butoxycarbonylamino-3-[4-chloro-2-(2,2-dimethyl-propionylamino)phenyl]propionic acid methyl ester (**Preparation 49**, 1.23g, 2.98mmol) in THF (50mL) was added dilute hydrochloric acid (2N, 50mL) and the mixture stirred under reflux for 72h. After cooling to rt and concentration *in vacuo* the residue was distributed between saturated sodium carbonate solution (200mL) and EtOAc (200mL). The layers were separated and the aqueous layer extracted with EtOAc (2x150mL). Washing of the combined EtOAc fractions with saturated sodium carbonate solution (100mL) and brine (100mL) gave a solution which was concentrated after drying (MgSO₄). Purification of the residue by flash chromatography on silica gel (eluent: DCM / methanol : 9/1,0.5% triethylamine) gave the title compound as a colourless solid. δ_{H} (d₄ MeOH): 2.85 (1H, t), 3.11 (1H, dd), 3.61 (1H, dd), 6.92 (1H, d), 6.99 (1H, dd), 7.20 (1H, d).

### Preparation 20 : 3-Amino-7-trifluoromethyl-3,4-dihydro-1H-quinolin-2-one hydrochloride

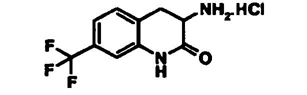

By a similar procedure to **Preparation 15**, the title compound was prepared using 2-nitro-4-(trifluoromethyl)benzyl chloride affording a white solid. δ_{H} (d₆ DMSO): 3.19 (1H, t), 3.34 (1H, dd), 4.26-4.31 (1H, m), 7.25 (1H, s), 7.34 (1H, dd), 7.52 (1H, d), 8.72 (3H, s), 11.00 (1H, s).

### Preparation 21 : 3-Amino-6,7-dimethoxy-3,4-dihydro-1H-quinolin-2-one

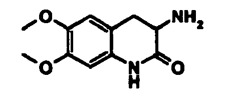

By a similar procedure to **Preparation 15**, the title compound was prepared using 4,5-dimethoxy-2-nitrobenzyl bromide affording a yellow solid. δ_{H} (CD₃OD): 2.75 (1H, t), 2.96 (1H, dd), 3.51 (1H, dd), 3.75 (6H, s), 6.49 (1H, s), 6.78 (1H, s).

### Preparation 22 : 3-Amino-5-fluoro-3,4-dihydro-1H-quinolin-2-one

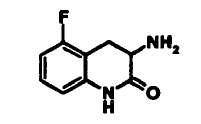

By a similar procedure to **Preparation 15**, the title compound was prepared using 2-fluoro-6-nitrobenzyl bromide affording a pink solid. m/z (ES⁺) 181 [M+H]⁺.

### Preparation 23 : 3-Amino-6-methyl-3,4-dihydro-1H-quinolin-2-one

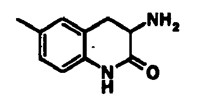

By a similar procedure to **Preparation 15**, the title compound was prepared using 5-methyl-2-nitrobenzyl chloride affording a tan solid. m/z (ES⁺) 177 [M+H]⁺.

### Preparation 24 : 3-(R)-Amino-3,4-dihydro-1H-quinolin-2-one hydrochloride

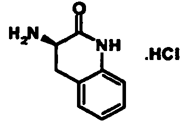

The title compound was prepared according to the method of Davis et. al. (J. Med. Chem, 1972, 15, 325). δ_{H} (d₆ DMSO): 7.30-7.19 (2H, m), 7.04-6.93 (2H, m), 4.26-4.14 (1H, m), 3.29-3.07 (2H, m).

### Preparation 25 : 3-(S)-Amino-3,4-dihydro-1H-quinolin-2-one hydrochloride

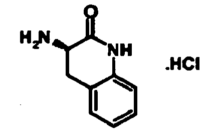

The title compound was prepared according to **Preparation 24.** δ_{H} (d₆ DMSO): 7.30-7.20 (2H, m), 7.03-6.91 (2H, m), 4.26-4.14 (1H, m), 3.28-3.09 (2H, m).

### Preparation 26 : (3-(R)-Amino-2-oxo-3,4-dihydro-2H-quinolin-1-yl)-acetic acid methyl ester

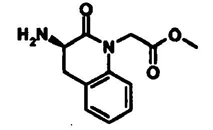

To a solution of 3-(*R*)-amino-3,4-dihydro-1H-quinolin-2-one hydrochloride (**Preparation 24**, 50mg, 0.25mmol) in DMF (5mL) under argon, cooled to 0°C (ice bath) was added sodium hydride (60% in mineral oil, 25mg, 0.63mmol), portion-wise, and the reaction stirred for 30min. Methyl bromoacetate (26µL, 0.28mmol) was added and the mixture stirred at 0°C for 1h before warming to rt for 1.5h. Conc.HCl (1mL) was added to the reaction, which was stirred for 10min before concentrating the solvent *in vacuo.* The residue was dissolved in DCM (30mL) and washed with NaHCO₃ solution (2x10mL) before being dried (MgSO₄) and the solvent removed *in vacuo*. Purification by column chromatography (SiO₂, 95:5 CH₂Cl₂/MeOH) afforded the tide compound. m/z (ES⁺) = 235.04 [*M*+ H]⁺; RT = 2.05min.

### Preparation 27 : (3-(S)-Amino-2-oxo-3,4-dihydro-2H-quinolin-1-yl)-acetic acid methyl ester

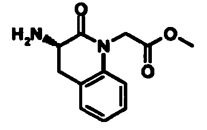

The title compound was prepared from 3-(*S*)-amino-3,4-dihydro-1H-quinolin-2-one hydrochloride (**Preparation 25**) according to **Preparation 26**. m/z (ES⁺) = 235.03 [*M*+ H]⁺; RT = 1.97min.

### Preparation 28 : 3-(R)-Amino-1-[2-(tert-butyl-dimethyl-silanyloxy)-ethyl]-3,4-dihydro-1H-quinolin-2-one

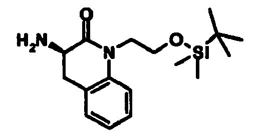

To a suspension of 3-(*R*)-amino-3,4-dihydro-1H-quinolin-2-one hydrochloride (**Preparation 24**, 199mg, 1.0mmol) in DMF (10mL), cooled to 0°C (ice bath), was added sodium hydride (60% suspension in mineral oil, 100mg, 2.5mmol), portion wise over 5min. The reaction was stirred at 0°C for 1h before adding (2-bromoethoxy) *tert*-butyldimethylsilane (260µL, 1.2mmol). The mixture was brought up to rt then heated to 60°C (bath temp) for 3h. Solvent was concentrated *in vacuo* then crude residue taken into EtOAc (50mL). Organics were washed with NaHCO₃ solution (2x30mL) then brine (30mL) before being dried (MgSO₄) and solvent removed *in vacuo.* Purification by column chromatography (SiO₂, 9:1 DCM/MeOH) afforded the title compound. δ_{H} (CDCl₃): 7.31-7.15 (3H, m), 7.07-7.0 (1H, m), 4.23-4.14 (1H, m), 4.0-3.83 (3H, m), 3.6-3.50 (1H, m), 3.06 (1H, dd), 2.90-2.80 (1H, m), 1.86 (9H, s), 0.04-0.00 (6H, m); m/z (ES⁺) = 321.13 [M+H]⁺; RT= 3.04min.

### Preparation 29 : 3-(R)-Amino-1-[2-(tert-butyl-dimethyl-silanyloxy)-ethyl]-3,4-dihydro-1H-quinolin-2-one

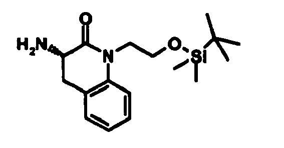

The title compound was prepared from 3-(S)-amino-3,4-dihydro-1H-quinolin-2-one hydrochloride (**Preparation 25**) according to **Preparation 28**. δ_{H} (CDCl₃): 7.31-7.15 (3H, m), 7.07-7.0 (1H, m), 4.23-4.14 (1H, m), 4.0-3.83 (3H, m), 3.6-3.50 (1H, m), 3.06 (1H, dd), 2.90-2.80 (1H, m), 1.86 (9H, s), 0.04-0.00 (6H, m); m/z (ES⁺) = 321.15 [*M*+ H]⁺; RT = 3.09min.

### Preparation 30 : (R)-5-Chloro-1H-pyrrolo[2,3-c]pyridine-2-carboxylic acid{1-[2-(tert-butyldimethylsilanyloxy)ethyl]-2-exo-1,2,3,4-tetrahydroquinolin-3-yl}amide

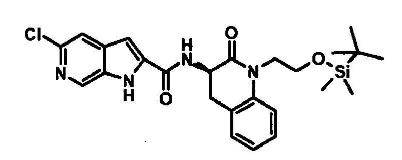

To a solution of 3-(*R*)-amino-1-[2-(tert-butyl-dimethyl-silanyloxy)-ethyl]-3,4-dihydro-1H-quinolin-2-one (**Preparation 28**, 130mg, 0.41mmol) in DMF (5mL) was added 5-chloro-1*H*-pyrrolo[2,3-c]pyridine-2-carboxylic acid (**Preparation 3**, 80mg, 0.41mmol) followed by DIPEA (177µL, 1.01 mmol) and HOBt (68mg, 0.45mmol). After 5min EDCI (93mg, 0.49mmol) was added and the reaction stirred for 16h at rt. Solvent was concentrated *in vacuo* and the residue partitioned between EtOAc (50mL) and water (30mL). Organics were washed with NaHCO₃ solution (2x30mL) and brine (30mL) then dried (MgSO₄), and solvent removed *in vacuo.* Purification by column chromatography (SiO₂, 95:5 DCM/MeOH) afforded the title compound. δ_{H} (CDCl₃): 10.2 (1H, s), 8.69 (1H, s), 7.80 (1H, m), 7.60 (1H, s), 7.4-7.24 (2H, m), 7.16-7.09 (1H, m), 6.97 (1H, s), 4.76-4.66 (1H, m), 4.30-4.23 (1H, m), 4.09-3.86 (3H, m), 3.67-3.59 (1H, m), 2.99-2.90 (1H, m), 1.87 (9H, s), 0.04 (3H, s), 0.00 (3H, s); m/z (ES⁺) = 499.09 [*M* +H]⁺; RT = 4.23min.

### Preparation 31 : (S)-5-Chloro-1H-pyrrolo[2,3-c]pyridine-2-carboxylic acid{1-[2-(tert-butyldimethylsilyloxy)ethyl]-2-oxo-1,2,3,4-tetrahydroquinolin-3-yl}amide

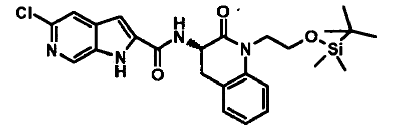

The title compound was prepared from 3-(*R*)-amino-1-[2-(tert-butyl-dimethyl-silanyloxy)-ethyl]-3,4-dihydro-1H-quinolin-2-one (**Preparation 29**) according to **Preparation 30**. δ_{H} (CDCl₃): 9.76 (1H, s), 8.70 (1H, s), 7.69 (1H, m), 7.63 (1H, s), 7.40-7.23 (2H, m), 7.14-7.09 (1H, m), 6.69 (1H, s), 4.71-4.63 (1H, m), 4.30-4.23 (1H, m), 4.07-3.86 (3H, m), 3.68-3.60 (1H, m), 2.96-2.84 (1H, m), 1.86 (9H, s), 0.04 (3H, s), 0.00 (3H, s).

### Preparation 32 : 4-Fluoro-2-nitrobenzaldehyde

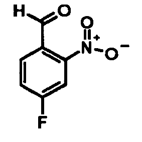

To a solution of 2-nitro-4-fluorotoluene (5.0g, 32.2mmol) in DMF (5mL) was added DMF.DMA (12.84mL, 97mmol) and the reaction heated to 135°C (bath temp) for 20h. The mixture was cooled to rt before being added to a stirring solution of sodium periodate (6.9g, 97mmol) in water/DMF (23mL: 12mL) via canula. The reaction was stirred at rt for 3h then filtered and the precipitate washed with toluene (200mL). The filtrate was separated and the organics washed with water (3x100mL) and dried (MgSO₄) before removing the solvent *in vacuo.* Purification by column chromatography (SiO₂, 9:1 hexane/EtOAc) afforded the title compound. δ_{H} (CDCl₃): 10.36 (1H, s), 8.03 (1H, dd), 7.81 (1H, dd), 7.49 (1H, m).

### Preparation 33 : 2-tert-Butoxycarbonylamino-3-(4-fluoro-2-nitrophenyl) acrylic acid ethyl ester

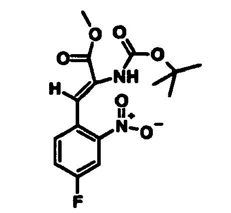

To a solution of (+/-)-BOC-α-phosphonoglycine trimethyl ester (1.66g, 5.59mmol) in THF (20mL), cooled to 78°C was added a solution of tetramethylguanidine (670µL, 5.34mmol) in THF (6mL), dropwise. The reaction was stirred for 20min before addition of a solution of 4-fluoro-2-nitro benzaldehyde (**Preparation 32**, 860mg, 5.09mmol) in THF (12mL) via canula. The reaction was stirred at rt for 16h before concentrating the solvent *in vacuo.* The residue was taken into EtOAc (100mL) and washed with water (2x30mL) then brine (30mL) before being dried (MgSO₄) and the solvent removed *in vacuo*. Purification by column chromatography afforded the title compound. δ_{H} (CDCl₃): 7.83 (1H, dd), 7.55 (1H, dd), 7.49 (1H, s), 7.33-7.25 (1H, m), 3.88 (3H, s), 1.31 (9H, s); m/z (ES⁺) = 241.06 [*M*+ H]⁺; RT = 3.58min.

### Preparation 34 : (7-Fluoro-2-oxo-1,2,3,4-tetrahydroquinolin-3-yl) carbamic acid tert-butyl ester

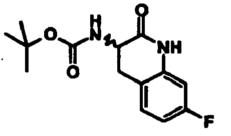

To a solution of 2-*tert*-butoxycarbonylamino-3-(4-fluoro-2-nitrophenyl)acrylic acid methyl ester (**Preparation 33**, 1.65g, 4.85mmol) in ethanol (80mL) was added Palladium (10%) on carbon (516mg, 0.48mmol) and the reaction stirred under an atmosphere of hydrogen for 16h. The mixture was filtered through celite, then 25% sodium methoxide in methanol (1.1mL, 4.85mmol) was added and the reaction stirred for a further 16h. Water (50mL) was added and the organics extracted into EtOAc (2x200mL), washed with brine (2x50mL) and dried (MgSO₄). Solvent was removed *in vacuo*, then trituration from diethyl ether/hexane afforded the title compound. δ_{H} (CD₃OD): 7.23-7.14 (1H, m), 6.74-6.66 (1H, m), 6.62 (1H, dd), 4.32-4.24 (1H, m), 3.14-3.06 (1H, m), 2.98-2.86 (1H, m), 1.46 (9H, s).

### Preparation 35:3-Amino-7-fluoro-3,4-dihydro-1H-quinolin-2-one hydrochloride

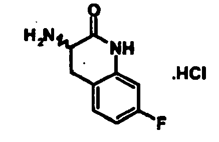

To a solution of (7-fluoro-2-oxo-1,2,3,4-tetrahydroquinolin-3-yl) carbamic acid *tert*-butyl ester (**Preparation 34**, 840mg, 3.0mmol) in methanol (20mL) was added a solution of 4M hydrogen chloride in dioxane (1.5mL, 6.0mmol) and the reaction stirred for 3h. Solvent was concentrated *in vacuo,* then the residue was dissolved in water (20mL) and washed with EtOAc (20mL). The aqueous solvent was removed *in vacuo* to afford the title compound. δ_{H} (CD₃OD): 7.23 (1H, dd), 6.80-6.70 (1H, m), 6.65 (1H, dd), 4.17 (1H, dd), 3.32-3.19 (1H, m), 3.13-2.98 (1H, m).

### Preparation 36: (3-Amino-7-fluoro-2-oxo-3,4-dihydro-2H-quinolin-1-yl) acetic acid methyl ester

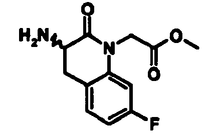

3-Amino-7-fluoro-3,4-dihydro-1H-quinolin-2-ane hydrochloride (**Preparation 35**) was alkylated according to **Preparation 26.** Purification by column chromatography (SiO₂, 92:8 CH₂Cl₂, MeOH) afforded the title compound. δ_{H}(d₆ DMSO): 7.31-7.22 (1H, m), 6.93 (1H, dd), 6.88-6.78 (1H, m), 4.82-4.48 (2H, m), 3.66 (3H, s), 3.46 (1H, dd), 2.98 (1H, dd), 2.77-2.65 (1H, m).

### Preparation 37 : 3-Amino-1-[2-(tert-butyldimethylsilyloxy)ethyl]-7-fluoro-3,4-dihydro-1H-quinolin-2-one

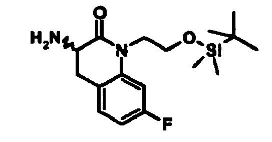

3-Amino-7-fluoro-3,4-dihydro-1H-quinolin-2-one hydrochloride (**Preparation 35**) was alkylated according to **Preparation 28** to give the title compound. δ_{H} (CDCl₃): 7.12-7.04 (2H, m), 6.72-6.64 (1H, m), 4.18-4.09 (1H, m), 3.92-3.79 (3H, m), 3.52 (1H, dd), 3.0 (1H, dd), 2.81-2.70 (1H, m), 0.83 (9H, s), 0.05-0.00 (6H, m):

### Preparation 38 : 5-Fluoro-2-nitrobenzaldehyde

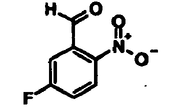

5-Fluoro-2-nitrotoluene was reacted in the same way as 4-fluoro-2-nitrotoluene according to **Preparation 32** to afford the title compound. δ_{H}(CDCl₃): 10.43 (1H, d), 8.20 (1H, dd), 7.61 (1H, dd), 7.45-7.37 (1H, m).

### Preparation 39 : 2-tert-Butoxycarbonylamino-3-(5-fluoro-2-nitrophenyl) acrylic add methyl ester

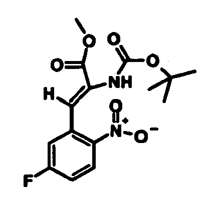

5-Fluoro-2-nitrobenzaldehyde (**Preparation 38**) was reacted according to **Preparation 33** to afford the title compound.δ_{H} (CDCl₃): 8.16 (1H, dd), 7.50 (1H, s), 7.22 (1H, dd), 7.11-7.05 (1H, m), 6.52 (1H, brs), 3.87 (3H, s), 1.27 (9H, s).

### Preparation 40 : (6-Fluoro-2-oxo-1,2,3,4-tetrahydroquinolin-3-yl) carbamic acid tert-butyl ester

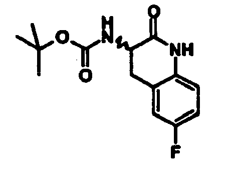

2-*tert*-Butoxycarbonylamino-3-(5-fluoro-2-nitrophenyl) acrylic acid methyl ester (**Preparation 39**) was reacted according to **Preparation 15** to afford the title compound. δ_{H} (CDCl₃): 8.43 (1H, br s), 6.94-6.87 (2H, m), 6.80-6.74 (1H, m), 5.6 (1H, br s), 4.38-4.25 (1H, m), 3.53-3.39 (1H, m), 2.90-2.75 (1H, m), 1.47 (9H, s).

### Preparation 41 : 3-Amino-6-fluoro-3,4-dihydro-1H-quinolin-2-one hydrochloride

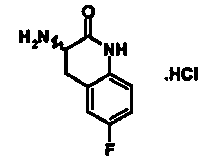

(6-Fluoro-2-oxo-1,2,3,4-tetrahydroquinolin-3-yl) carbamic acid *tert*-butyl ester (Preparation 40) was deprotected according to Preparation 35 to afford the title compound. δ_{H} (CD₃OD): 7.08-6.89 (3H, m), 4.22-4.13 (1H, m), 3.35-3.23 (1H, m), 3.19-3.08 (1H, m).

### Preparation 42 : 5-Chloro-1H-pyrrolo[2,3-c]pyridine-2-carboxylic acid{1-[2-(tert-butyldimethylsilyloxy)ethyl]-7-fluoro-2-oxo-1,2,3,4-tetrahydroquinolin-3-yl}amide

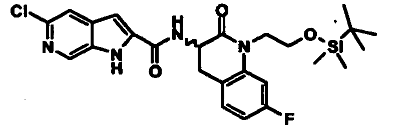

The title compound was prepared as described in **Example 12** from 5-chloro-1*H*-pyrrolo[2,3-c]pyridine-2-carboxylic acid (**Preparation 18**) and the appropriate amine. m/z (ES⁺) = 519.09[*M*+ H]+; RT 4.28min.

### Preparation 43 and 44 :

The procedure described in **Example 48** was used to prepare the compounds of **Preparation 43** and **44** from 5-chloro-1H-pyrrolo[2,3-c]pyridine-2-carboxylic acid (**Preparation 3**) and the appropriate amine.

| **Prep** | **Structure** | **Amine** | **RT (min)** | **m/z (ES⁺)** |
|---|---|---|---|---|
| 43 | 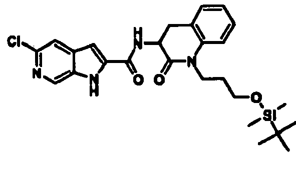 | WO03/074532 | 4.39 | 513 [*M*+H]⁺ |
| 44 | 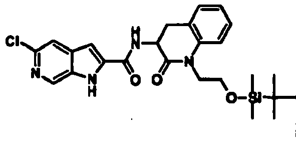 | Preparation 16 | 4.42 | 499 [*M+*H]⁺ |

### Preparation 45: 4-Chloro-2-(2,2-dimethylpropionylamino)benzoic acid methyl ester

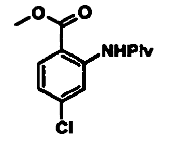

To a solution of commercially available 2-amino-4-chloro-benzoic acid methyl ester (LANCASTER, 3.0g, 16.2mmol) in dry DCM (50mL) was added DIPEA (5.1mL, 29.3mmol), pivaloyl chloride (3.6mL, 29.2mmol) and 4-dimethylaminopyridine (50mg, 0.41mmol). The mixture was stirred for 2.5h, then diluted with EtOAc (300mL) and washed with diluted hydrochloric acid (1N, 100mL) and brine (100mL) before being dried (MgSO₄) and concentrated to give a solid residue. Recrystallisation from methanol gave the title compound as colourless needles. δ_{H} (CDCl₃): 1.35 (9H, s), 3.93 (3H, s), 7.04 (1H, d), 7.96 (1H, d), 8.91 (1H, s), 11.35 (1H, br s); m/z (ES) = 268.08 [M-H]; RT = 4.06 min.

### Preparation 46 : N-(5-Chloro-2-hydroxymethylphenyl)-2,2-dimethylpropionamide

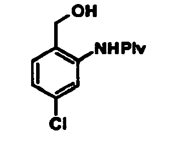

To a solution of 4-chloro-2-(2,2-dimethylpropionylamino)benzoic acid methyl ester (**Preparation 45**, 1.83g, 6.78mmol) in ethanol (100mL) and water (10mL) was added sodium borohydride (530mg, 14.0mmol) and the mixture stirred for 3h at rt. The solution was acidified with dilute hydrochloric acid (pH 2-3) and diluted with water (200mL) before being concentrated to half the original volume. The aqueous layer extracted with EtOAc (3x50mL) and the combined extracts were dried (MgSO₄) and concentrated to an oily residue. Purification by flash chromatography on silica gel (eluent: hexane / EtOAc : 2 / 1) gave the title compound as colourless oil. δ_{H} (CDCl₃): 1.29 (9H, s), 2.80 (1H, br s) 4.65 (2H, s), 7.00 (1H, dd), 7.04 (1H, d), 8.19 (1H, d), 9.09 (1H, br s); m/z (ES) = 240.06 [M-H]⁻; RT = 3.19 min.

### Preparation 47 : N-(5-Chloro-2-formylphenyl)-2,2-dimethylpropionamide

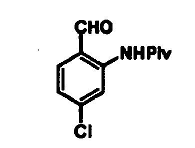

To a solution of N-(5-chloro-2-hydroxymethylphenyl)-2,2-dimethylpropionamide (**Preparation 46**, 970mg, 4.01mmol) in dry DCM (40mL) was added Dess-Martin periodinane (1.80g, 4.24mmol). After stirring for 2h at rt alkaline sodium thiosulfate solution was added (27g Na₂SO₃ dissolved in 100mL saturated NaHCO₃ solution) and the emulsion was vigorously stirred for additional 20min. The layers were separated and the aqueous layer extracted with ethyl acetate (2x50mL). Washing of the combined extracts with saturated sodium hydrogen carbonate solution (50mL) and brine (50mL) gave a solution, which was concentrated after drying (MgSO₄). Purification of the residue by flash chromatography on silica gel (eluent: hexane / EtOAc : 5 / 1) gave the title compound as colourless oil. δ_{H} (CDCl₃): 1.36 (9H, s), 7.19 (1H, dd), 7.60 (1H, d), 8.91 (1H, d), 9.90 (1H, s), 11.45 (1H, br s); m/z (ES⁺) = 240.06 [M+H]⁺; RT = 3.79 min.

### Preparation 48 : (E,Z)-2-tert-Butoxycarbonylamino-3-[4-chloro-2-(2,2-dimethylpropionylamino)phenyl]acrylic acid methyl ester

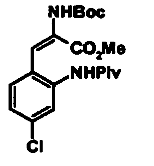

To a solution of (+/-)-Boc-α-phosphonoglycine trimethyl ester (1.10g, 3.70 mmol) in dry THF (10mL) at-78°C was added 1,1,3,3-tetramethylguanidine (0.45mL, 3.59mmol) and the mixture was stirred in the cold for 20min. A solution of N-(5-chloro-2-formylphenyl)-2,2-dimethylpropionamide (**Preparation 47**, 740mg, 3.09mmol) in dry THF (10mL) was added and the resulting mixture was allowed to warm up to rt and stirred for additional 12h. Distribution between EtOAc (200mL) and water (100mL) followed by separation of the organic layer gave after drying (MgSO₄) and concentration an oily residue. Purification of the residue by flash chromatography on silica gel (eluent: hexane / EtOAc : 2 /1) gave the title compound(s) as a colourless oil(s). δ_{H} (CDCl₃) - major product: 1.33 (9H, s), 1.34 (9H, s), 3.91 (3H, s), 6.43 (1H, br s), 7.08 (1H, s), 7.11 (1H, dd), 7.22 (1H, d), 7.61 (1H, br s), 8.11 (1H, d) - minor product: 1.31 (9H, s), 1.54 (9H, s), 3.63 (3H, s), 6.99 (1H, brs), 7.06 (2H, m), 7.54 (1H, m), 7.61 (1H, br s), 8.34 (1H, m); m/z (ES⁻) = 409.14 [M-H]⁻; RT = 3.72 min.

### Preparation 49 : 2-tert-Butoxycarbonylamino-3-[4-chloro-2-(2,2-dimethylpropionylamino)phenyl]propionic acid methyl ester

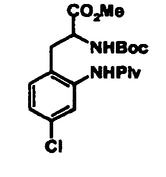

To a solution of (E,Z)-2-tert-butoxycarbonylamino-3-[4-chloro-2-(2,2-dimethyl propionylamino)phenyl]acrylic acid methyl ester (**Preparation 48**, 502mg, 1.22mmol) in methanol (10mL) were added magnesium turnings (60mg, 2.47 mmol) and the mixture stirred at rt for 12h. Toluene (200mL) was added and the resulting organic layer was washed with diluted hydrochloric acid (0.1N, 50mL) and brine (50mL) before being dried (MgSO₄) and concentrated. The resulting oil was used in the next step without further purification. δ_{H} (CDCl₃): 1.38 (9H, s), 1.43 (9H, s), 2.96-3.10 (2H, m), 3.74 (3H, s), 4.56 (1H, m), 5.19 (1H, br d), 7.06 (1H, dd), 7.11 (1H, dd), 7.84 (1H, m), 8.06 (1H, br s); m/z (ES⁻) = 411.18 [M-H]⁻; RT = 3.92 min.

### Preparation 50 : (3-Amino-7-chloro-2-oxo-3,4-dihydro-2H-quinolin-1-yl)acetic acid methyl ester

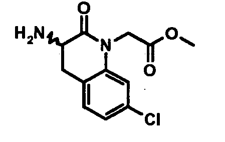

To a vigorously stirred solution of 3-amino-7-chloro-3,4-dihydro-1H-quinolin-2-one (**Preparation 19**, 630mg, 3.24 mmol) in DMF (20mL) at 0°C was added sodium hydride dispersion (146mg, 3.65mmol, 60%). After 30min methyl bromoacetate (0.31mL, 3.27mmol) was added, the cooling bath was removed and the resulting mixture stirred for further 12h before added into saturated sodium hydrogencarbonate solution (150mL). Extraction with EtOAc (3x50mL) and washing of the combined extracts with brine (50mL) gave after drying (MgSO₄) and concentration *in vacuo* an oily residue. Purification of the residue by flash chromatography on silica gel (eluent: DCM then DCM / methanol: 19 / 1 then DCM / methanol: 9/1) gave the title compound as colourless oil. δ_{H} (CDCl₃): 1.80 (2H, br s), 2.87 (1H, dd), 3.08 (1H, dd), 3.63 (1H, m), 3.79 (3H, s), 4.43 (1H, d), 4.86 (1H, d), 6.74 (1H, d), 7.02 (1H, dd), 7.15 (1H, d); m/z (ES⁺) = 269.04 [M+H]⁺; RT = 2.04 min.

### Preparation 51 : 3-Amino-1-[2-(tert-butyldimethylsilanyloxy)ethyl]-7-chloro-3,4-dihydro-1H-quinolin-2-one

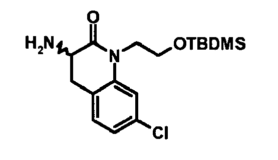

To a vigorously stirred solution of 3-amino-7-chloro-3,4-dihydro-1H-quinolin-2-one (**Preparation 19**, 149mg, 0.76mmol) in DMF (5mL) at 0°C was added sodium hydride dispersion (38mg, 0.95mmol, 60%). After 1h (2-bromoethoxy)-*tert*-butyldimethylsilane (165µL, 0.77mmol) was added, the cooling bath was removed and the resulting mixture was stirred for further 12h before being added into saturated sodium hydrogen carbonate solution (100mL). Extraction with EtOAc (3x50mL) and washing of the combined extracts with brine (50mL) gave after drying (MgSO₄) and concentration *in vacuo* an oily residue. Purification of the residue by flash chromatography on silica gel (eluent: DCM / methanol: 19 / 1) gave the title compound as a colourless oil. δ_{H} (CDCl₃): 0.00, 0.03 (6H, 2xs), 0.85 (9H, s), 1.73 (2H, br s), 2.79 (1H, dd), 3.03 (1H, dd), 3.54 (1H, dd), 3.86-3.93 (3H, m), 4.19 (1H, m), 6.98 (1H, dd), 7.09 (1H, d), 7.39 (1H, d); m/z (ES⁺) = 355.15 [M+H]⁺; RT = 3.27 min.

### Preparation 52 : 2-tert-Butoxycarbonylamino-3-[2-(2,2-dimethylpropionylamino)-pyridin-3-yl]acrylic acid methyl ester

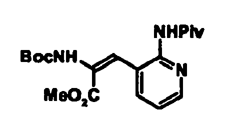

To a solution of (+/-)-Boc-α-phosphonoglycine trimethyl ester (1.52g, 5.11 mmol) in dry THF (20mL) at-78°C was added 1,1,3,3-tetramethylguanidine (0.60mL, 4.78mmol) and the mixture was stirred in the cold for 20min. A solution of commercially available N-(3-formylpyridin-2-yl)-2,2-dimethylpropionamide (SPECS and BioSPECS, 960mg, 4.65mmol) in dry THF (20mL) was added dropwise and the resulting mixture allowed to warm up to rt and stirred for additional 12h before being poured into water (200mL). Extraction with EtOAc (3x75mL) and washing of the combined extracts with brine (50mL) gave after drying (MgSO₄) and concentration *in vacuo* an oily residue. Purification of the residue by flash chromatography on silica gel (eluent: hexane / EtOAc : 1/3) gave the title compound as a colourless oil. δ_{H} (CDCl₃): 1.33 (9H, s), 1.37 (9H, s), 3.84 (3H, s), 6.37 (1H, br s), 7.06 (1H, s), 7.16 (1H, dd), 7.80-7.83 (2H, m), 8.38 (1H, d); m/z (ES⁺ = 377.97 [M+H]⁺; RT = 3.36 min.

### Preparation 53 : 2-tert-Butoxycarbonylamino-3-[2-(2,2-dimethylpropionylamino)pyridin-3-yl]propionic acid methyl ester

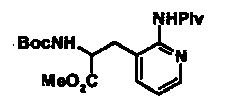

Palladium-on-carbon (266mg, 10wt%) was added to a solution of 2-tert-butoxycarbonylamino-3-[2-(2,2-dimethylpropionylamino)pyridin-3-yl]acrylic acid methyl ester (Preparation 52, 1.40g, 3.71mmol) in ethanol (50mL) and the mixture stirred under an atmosphere of hydrogen for 12h. After filtration through celite and repeated washing of the catalyst with methanol, the filtrate and washings were combined and concentrated *in vacuo* to give the title compound as colourless oil. δ_{H} (CDCl₃): 1.34 (9H, s), 1.37 (9H, s), 2.83 (1H, dd), 3.22 (1H, dd), 3.71 (3H, s), 4.62 (1H, m), 5.32 (1H, br d), 7.14 (1H, dd), 7.58 (1H, d), 7.98 (1H, br s), 8.34 (1H, d); m/z (ES⁺) = 380.11 [M+H]⁺; RT = 3.24 min.

### Preparation 54 : 3-Amino-3,4-dihydro-1H-[1,8]naphthyridin-2-one dihydrochloride

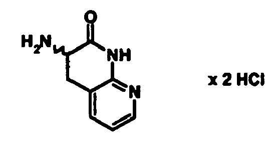

2-tert-Butoxycarbonylamino-3-[2-(2,2-dimethylpropionylamino)pyridin-3-yl]propionic acid methyl ester (**Preparation 53**, 1.30g, 3.43 mmol) was dissolved in dilute hydrochloric acid (2N, 50mL) and the solution heated under reflux for 72h. After cooling to rt and concentration *in vacuo* the residue was taken up in water (200mL). The aqueous layer was washed with ethyl acetate (2x50mL) and concentrated. Addition of methanol (~10mL) led to the precipitation of the title compound, which was obtained as an off-white solid after filtration. δ_{H} (D₂O): 3.36 (1H, dd), 3.59 (1H, dd), 4.55 (1H, dd), 7.43 (1H, dd), 8.15 (1H, d), 8.27 (1H, d); m/z (ES⁺) = 164.05 [M-2HCl+H]⁺; RT = 0.31 min.

### Preparation 55 : 4-[N-(tert-Butyloxycarbonyl)amino]-3-pyridine-carboxaldehyde

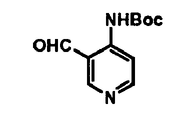

The title compound was synthesized via a known method from 4-[N-(*tert-*butyloxycarbonyl)amino]pyridine (M.C. Venuti et al., J. Med Chem., 1988, 31, 2136-2145). δ_{H} (CDCl₃): 1.55 (9H, s), 8.34 (1H, d), 8.59 (1H, d), 8.76 (1H, s), 9.98 (1H, s), 10.43 (1H, br s); m/z (ES⁺) = 223.01 [M+H]⁺; RT = 3.01 min.

### Preparation 56 : 2-tert-Butoxycarbonylamino-3-(4-tert-butoxycarbonylaminopyridin-3-yl) acrylic acid methyl ester

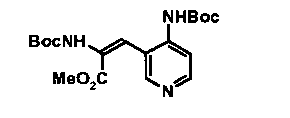

To a solution of (+/-)-Boc-oc-phosphonoglycine trimethyl ester (1.50g, 5.05mmol) in dry THF (20mL) at -78°C was added 1,1,3,3-tetramethylguanidine (0.60mL, 4.78mmol) and the mixture was stirred in the cold for 20min. A solution of 4-[N-(*tert*-butyloxycarbonyl)amino]-3-pyridinecarboxaldehyde (**Preparation 55**, 1.0g, 4.50mmol) in dry THF (10mL) was added slowly and the resulting mixture allowed to warm up to rt and stirred for additional 12h before being poured into water (200mL). Extraction with EtOAc (3x75mL) and washing of the combined extracts with brine (50mL) gave after drying (MgSO₄) and concentration *in vacuo* an oily residue. Purification of the residue by flash chromatography on silica gel (eluent: toluene / acetone : 2 /1) gave the title compound as colourless oil. δ_{H} (CDCl₃): 1.31 (9H, s), 1.53 (9H, s), 3.91 (3H, s), 6.61, 6.76 (2H, 2 xbr s), 7.05 (1H, s), 8.06 (1H, d), 8.37-8.39 (2H, m); m/z (ES⁺) = 394.13 [M+H]⁺; RT = 2.81 min.

### Preparation 57 : 2-tert-Butoxycarbonylamino-3-(4-tert-butoxycarbonylaminopyridin-3-yl) propionic acid methyl ester

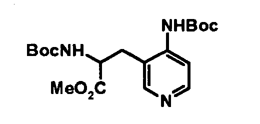

Palladium-on-carbon (225mg, 10wt%) was added to a solution of 2-tert-butoxycarbonylamino-3-(4-tert-butoxycarbonylaminopyridin-3-yl)acrylic acid methyl ester (**Preparation 56**, 1.02g, 2.59mmol) in ethanol (40mL) and the mixture stirred under an atmosphere of hydrogen for 12h. After filtration through celite and repeated washing of the catalyst with methanol, the filtrate and washings were combined and concentrated *in vacuo* to give the title compound as colourless oil. δ_{H} (CDCl₃): 1.50 (9H, s), 1.56 (9H, s), 2.94 (1H, dd), 3.16 (1H, m), 3.74 (3H, s), 4.30 (1H, m), 5.61 (1H, br d), 8.13-8.16 (2H, m), 8.37 (1H, d), 8.54 (1H, br s); m/z (ES⁺) = 396.15 [M+H]⁺; RT = 2.95 min.

### Preparation 58 : 3-Amino-3,4-dihydro-1H-[1,6]naphthyridin-2-one dihydrochloride

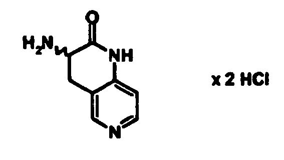

2-tert-Butoxycarbonylamino-3-(4-tert-butoxycarbonylaminopyridin-3-yl)propionic acid methyl ester (**Preparation 57**, 830mg, 2.10mmol) was dissolved in dilute hydrochloric acid (2N, 50mL) and the solution was heated under reflux for 2h. After cooling to rt and concentration *in vacuo* the residue was taken up in water (200mL). The aqueous layer was washed with ethyl acetate (50mL) and diethyl ether (50mL) and then concentrated again to give the title compound as off-white solid. δ_{H} (D₂O): 3.40 (1H, dd), 3.69 (1H, m), 4.60 (1H, m), 7.44 (1H, m), 8.53 (1H, m), 8.61 (1H, m); m/z (ES+) = 164.03 [M-2HCl+H]⁺; RT = 0.22 min.

### Preparation 59 : (3-Amino-2-oxo-3,4-hydro-2H-[1,6]naphthyridin-1-yl)acetic acid methyl ester dihydrochloride

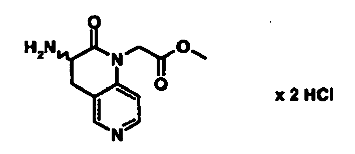

To a vigorously stirred solution of 3-amino-3,4-dihydro-1H-[1,6]naphthyridin-2-one dihydrochloride (**Preparation 58**, 203mg, 0.86mmol) in DMF (10mL) at 0°C was added sodium hydride dispersion (119mg, 2.98mmol, 60%). After 1.5h methyl bromoacetate (80µL, 0.85mmol) was added, the cooling bath was removed and the resulting mixture was stirred for further 12h before added into water (100mL). The solution was made acidic (pH 2-3) with dilute hydrochloric acid (1N) and washed with ethyl acetate (30mL) After concentration *in vacuo* the title compound was obtained as an oil, which was used in the next step without further purification. δ_{H} (D₂O): 3.53 (1H, dd), 3.75 (1H, dd), 4.74 (1H, m), 4.92 (1H, d), 5.14 (1H, d), 7.66 (1H, d), 8.70 (1H, d), 8.75 (1H, s); m/z (ES⁺) = 236.03 [M-2HCl+H]⁺; RT = 0.42 min.

### Preparation 60 : (3-Amino-2-oxo-3,4-dihydro-2H-[1,5]naphthyridin-1-yl)acetic add methyl ester

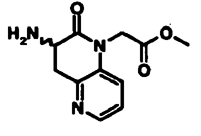

To a vigorously stirred solution of 3-amino-3,4-dihydro-1H-[1,5]naphthyridin-2-one dihydrochloride (WO 03/074532, 202mg, 0.86mmol) in DMF (10mL) at 0°C was added sodium hydride dispersion (120mg, 3.00mmol, 60%). After 1.5h methyl bromoacetate (80µL, 0.85mmol) was added, the cooling bath was removed and the resulting mixture was stirred for further 12h before added into saturated sodium hydrogencarbonate solution (150mL).
Extraction with EtOAc (3x50mL) and washing of the combined extracts with brine (50mL) gave after drying (MgSO₄) and concentration *in vacuo* an oily residue. Purification of the residue by flash chromatography on silica gel (eluent: DCM / methanol: 85 / 15) gave the tide compound as colourless oil. δ_{H} (d₄ MeOH): 3.17 (1H, dd), 3.32 (1H, dd), 3.76 (3H, s), 3.79 (1H, dd), 4.60 (1H, d), 4.89 (1H, d), 7.34 (1H, dd) 7.41 (1H, d), 8.18 (1H, d); m/z (ES⁺) = 236.05 [M+H]⁺; RT = 0.53 min.

### Preparation 61 : 3-Amino-1-[2-(tert-butyldimethylsilanyloxy)ethyl)-3,4-dihydro-1H-[1,5] naphthyridin-2-one

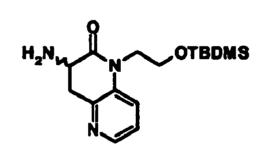

To a vigorously stirred solution of 3-amino-3,4-dihydro-1H-[1,5]naphthyridin-2-one dihydrochloride (WO 03/074532, 200mg, 0.85mmol) in DMF (10mL) at 0°C was added sodium hydride dispersion (114mg, 2.85mmol, 60%). After 45min (2-bromoethoxy)-*tert*-butyldimethylsilane (185µL, 0.86mmol) was added, the cooling bath was removed and the resulting mixture was stirred for further 12h before added into saturated sodium hydrogencarbonate solution (150mL). Extraction with ethyl acetate (3x50mL) and washing of the combined extracts with brine (50mL) gave after drying (MgSO₄) and concentration *in vacuo* an oily residue. Purification of the residue by flash chromatography on silica gel (eluent: DCM / methanol: 9/1) gave the title compound as colourless oil. δ_{H} (d₄ MeOH): 0.00, 0.02 (6H, 2xs), 0.84 (9H, s), 3.09 (1H, dd), 3.30 (1H, dd), 3.74 (1H, dd), 3.89-4.23 (4H, 3xm), 7.36 (1H, dd), 7.82 (1H, d), 8.17 (1H, d); m/z (ES⁺) = 322.13 [M+H]⁺; RT = 2.74 min.

### Preparation 62 : 3-Amino-3,4-dihydro-1H-[1,7] naphthyridin-2-one dihydrochloride

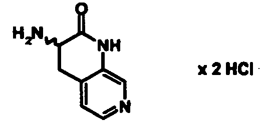

To a solution of *tert*-butyl(2-oxo-1,2,3,4-tetrahydro-[1,7]naphthyridin-3-yl)carbamate (WO 03/074532, 400mg, 1.52mmol) in DCM (20mL) was added hydrochloric acid (3mL, 4N in dioxane). After stirring for 4h at rt the solution was concentrated *in vacuo* before the residue was taken up in water (150mL). The aqueous layer was washed with EtOAc (2x50mL) and concentrated. Addition of methanol (~5mL) led to the precipitation of the title compound, which was obtained as a white solid after filtration. δ_{H} (D₂O): 3.56 (1H, dd), 3.75 (1H, dd), 4.55 (1H, dd), 7.95 (1H, d), 8.41 (1H, s), 8.46 (1H, d); m/z (ES⁺) = 164.05 [M-2HCl+H]⁺; RT = 0.21 min.

### Preparation 63 : 5-Chloro-1H-pyrrolo[2,3-c]pyridine-2-carboxylic acid {1-[2-(tert-butyldimethyl-silanyloxy)ethyl]-2-oxo-1,2,3,4-tetrahydro-[1,5]naphthyridin-3-yl}amide

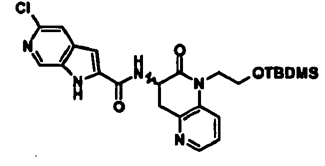

The title compound was prepared according to the method of **Example 69** from chloro-1H-pyrrolo[2,3-c]pyridine-2-carboxylic acid (**Preparation 3**) and the appropriate amine: m/z (ES⁺)= 500.11 [M+H]⁺; RT = 3.98 min.

### Preparation 64 : 5-Chloro-1H-pyrrolo[2,3-c]pyridine-2-carbozylic acid {1-[2-(tert-butyldimethyl silanyloxy)ethyl]-7-chloro-2-oxo-1,2,3,4-tetrahydroquinolin-3-yl}amide

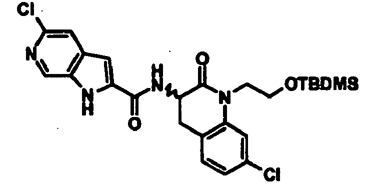

To a solution of 5-chlom-1H-pyrrolo[2,3-c]pyridine-2-carboxylic acid (**Preparation 16,** 115mg, 0.59mmol), EDCI (132mg, 0.69mmol) and HOBt monohydrate (89mg, 0.58mmol) in DMF (10mL) was added 3-amino-1-[2-(tert-butyldimethylsilanyloxy)-ethyl]-7-chloro-3,4-dihydro-1H-quinolin-2-one (**Preparation 7**, 200mg, 0.56mmol) and DIPEA (220µL, 1.26mmol). The resulting solution was stirred for 12h at rt before the reaction mixture was partitioned between EtOAc (50mL) and water/brine (100mL, 1:1). The layers were separated and the aqueous phase was extracted with EtOAc (3x50mL), then the combined organics were washed with dilute HCl solution (1M, 50mL), dilute NaOH solution (1M, 50mL) and brine (50mL). The organic phase was dried (MgSO₄), filtered and concentrated *in vacuo.* Purification of the residue by flash chromatography on silica gel (eluent: hexane / EtOAc : 1 / 2) gave the title compound as a colourless solid. TLC (hexane / EtOAc : 1 / 3): R_{f} 0.60; δ_{H} (d₆ DMSO): 0.00, 0.01 (6H, 2xs), 0.83 (9H, s), 3.14-3.21 (2H, m), 3.84-3.98 (3H, 2xm), 4.20 (1H, m), 4.78 (1H, ddd), 7.15 (1H, dd), 7.30 (1H, s), 7.34 (1H, d), 7.49 (1H, d), 7.83 (1H, s), 8.63 (1H, s), 9.15 (1H, d), 12.41 (1H, br s); m/z (ES⁺) = 533.19 [M+H]⁺; RT = 4.77 min.

### Example 1: 6-Chloro-1H-pyrrolo[2,3-b]pyridine-2-carboxylic acid (2-oxo-1,2,3,4-tetrahydroquinolin-3-yl)amide

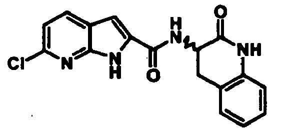

To a solution of 3-amino-3,4-dihydro-1H-quinolin-2-one (**Preparation 15**, 27mg, 0.17mmol) in DMF (4mL) was added 6-chloro-1H-pyrrolo[2,3b]pyridine-2-carboxylic acid (**Preparation 12**, 30mg, 0.15mmol), HOBt (26mg, 0.17mmol) and DIPEA (66µL, 0.38mmol) and the reaction stirred for 5min. EDCI (35mg, 0.18mmol) was added and the reaction stirred at rt for 16h. Solvent was removed *in vacuo* and the residue partitioned between EtOAc (30mL) and water (30mL). Organics were washed with water (30mL), NaHCO₃ solution (2x25mL) then brine (2x25mL) before being dried (MgSO₄) and concentrated *in vacuo.* Purification by Prep HPLC afforded the title compound. δ_{H} (d₆ DMSO): 8.20 (1H, d), 7.29-7.17 (4H, m), 7.00-6.89 (2H, m), 4.80-4.70 (1H, m), 3.21-3.06 (2H, m); m/z (ES⁺) = 341.09 [*M*+ H]⁺; RT = 3.33min.

### Example 2: 6-Chloro-1H-pyrrolo[2,3-b]pyridine-2-carboxyllic acid (7-chloro-2-oxo-1,2,3,4-tetrahydroquinolin-3-yl)amide

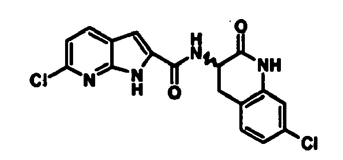

The title compound was prepared according to **Example 1** using 3-amino-7-chloro-3,4-dihydro-1H-quinolin-2-one (**Preparation 19**) instead of 3-amino-3,4-dihydro-1H-quinolin-2-one. δ_{H} (d₆ DMSO): 8.19 (1H, d), 7.30-7.24 (3H, m), 7.20 (1H, d), 6.94 (1H, s), 4.81-4.71 (1H, m), 3.16-3.09 (2H, m); m/z (ES⁺) = 375.05 [*M*+H]⁺; RT = 3.46 min.

### Example 3: (R)-5-Chloro-1H-pyrrolo[2,3-c]pyridine-2-carborylic acid (2-oxo-1,2,3,4-tetrahydroquinolin-3-yl)amide

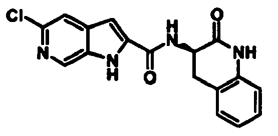

To a suspension of 3-*(R)*-amino-3,4-dihydro-1H-quinolin-2-one hydrochloride (**Preparation 24**, 67mg, 0.34mmol) in DMF (5mL) under argon was added DIPEA (186µL, 1.07mmol), 5-chloro-1*H*-pyrrolo[2,3-c]pyridine-2-carboxylic acid (**Preparation 3,** 60mg, 0.31mmol) and HOBt (51mg, 0.34mmol) and the reaction stirred for 5min. EDCI (76mg, 0.5mmol) was added and the reaction stirred for 16h at rt. Solvent was removed *in vacuo* and the residue partitioned between EtOAc (50mL) and water (40mL). Organics were washed with NaHCO₃ solution (2x15mL) then brine (15mL) before being dried (MgSO₄) and solvent removed *in vacuo.* Purification by crystallisation from methanol afforded the title compound. δ_{H} (d₆ DMSO): 8.61 (1H, s), 7.80 (1H, s), 7.29-7.17 (3H, m), 7.00-6.90 (2H, m), 4.82-4.73 (1H, m), 3.22-3.06 (2H, m); m/z (ES⁺) = 341.03 [*M*+ H]⁺; RT = 3.24min.

### Example 4: (S)-5-Chloro-1A-pyrrolo[2,3-c]pyridine-2-carboxylic add (2-oxo-1,2,3,4-tetrahydroquinolin-3-yl)amide

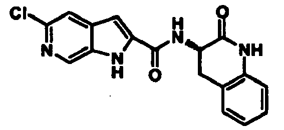

The title compound was prepared from 3-*(S)*-amino-3,4-dihydro-1H-quinolin-2-one hydrochloride (**Preparation 25**) according to **Example 3**. &_{H}(d₆ DMSO): 8.61 (1H, s), 7.80 (1H, s), 7.29-7.17 (3H, m), 7.00-6.90 (2H, m), 4.82-4.73 (1H, m), 3.22-3.06 (2H, m); m/z (ES⁺) = 341.02 [M+ H]⁺; RT = 3.20min.

### Example 5: {3-(R)-[(5-Chloro-1H-pyrrolo[2,3-c]pyridine-2-carbonyl)amino]-2-oxo-3,4-dithydro-2H-quinolin-1-yl}acetic add methyl ester

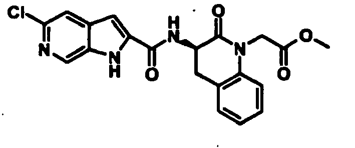

To a solution of 5-chloro-1*H-* pyrrolo[2,3-c]pyridine-2-carboxylic acid (**Preparation 3**, 30mg, 0.15mmol) in DMF (5mL) was added (3-(*R*)-amino-2-oxo-3,4-dihydro-2H-quinolin-1-yl)-acetic acid methyl ester (**Preparation 26**, 36mg, 0.15mmol), HOBt (26mg, 0.17mmol) and DIPEA (67µL, 0.38mmol) and the reaction stirred for 5min. EDCI (35mg, 0.18mmol) was added and the reaction stirred for 16h at rt. Solvent was concentrated *in vacuo* and the residue partitioned between EtOAc (30mL) and water (30mL). Organics were washed with 1M NaOH (2x20mL) and brine (20mL) then dried (MgSO₄) and the solvent removed *in vacuo.* Trituration with methanol afforded the title compound. δ_{H} (d₆ DMSO): 8.61 (1H, s), 7.80 (1H, s), 7.39-7.26 (3H, m), 7.14-7.06 (2H, m), 4.91-4.77 (2H, m), 7.10-7.04 (1H, m), 3.70 (3H, s), 3.34-3.06 (2H, m); m/z (ES⁺) = 413.05 [*M*+ H]⁺; RT = 3.31 min.

### Example 6: {3-(S)-[(5-Chloro-1H-pyrrolo[2,3-c]pyridine-2-carbonyl)amino]-2-oxo-3,4-dihydro-2H-quinolin-1-yl}acetic acid methyl ester

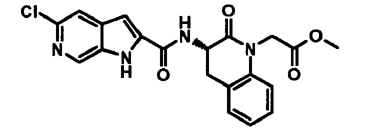

The title compound was prepared from (3-(S)-amino-2-oxo-3,4-dihydro-2H-quinolin-1-yl)-acetic acid methyl ester (**Preparation 27**) according to **Example 5**. δ_{H} (d₆ DMSO): 8.61 (1H, s), 7.80 (1H, s), 7.39-7.26 (3H, m), 7.14-7.06 (2H, m), 4.91-4.77 (2H, m), 7.10-7.04 (1H, m), 3.70 (3H, s), 3.34-3.06 (2H, m); m/z (ES⁺) = 413.04 [*M* + H]⁺; RT = 3.34min.

### Example 7: {3-(R)-[(5-Chloro-1H-pyrrolo[2,3-c]pyridine-2-carbonyl)amino]-2-oxo-3,4-dihydro-2H-quinolin-1-yl}acetic acid

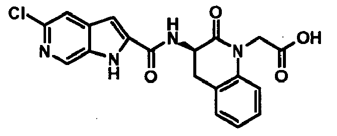

To a suspension of {3-(*R*)-[(5-chloro-1H-pyrrolo[2,3-c]pyridine-2-carbonyl)amino]-2-oxo-3,4-dihydro-2H-quinolin-1-yl}acetic acid methyl ester (**Example 5**, 15.2mg, 0.04mmol) in THF (2mL) was added a 2M solution of LiOH (40µL, 0.08mmol) and the reaction stirred for 3h. Solvent was concentrated *in vacuo* then the residue dissolved in water (15mL). The aqueous phase was washed with EtOAc before being acidified to pH 2 with 2M HCl. Organics were extracted into EtOAc (20mL) and the solvent removed *in vacuo* to afford the title compound. δ_{H} (CD₃OD): 8.63 (1H, s), 7.73 (1H, s), 7.39-7.30 (2H, m), 7.23 (1H, s), 7.14 (1H, m), 7.04 (1H, d), 5.03-4.89 (2H, m), 4.63 (1H,d), 3.37-3.20 (2H, m); m/z (ES) = 399.01 [*M*+ H]⁺; RT = 3.20min.

### Example 8: {3-(S)-[(5-Chloro-1H-pyrrolo[2,3-c]pyridine-2-carbonyl)amino]-2-oxo-3,4-dihydro-2H-quinolin-1-yl}acetic acid

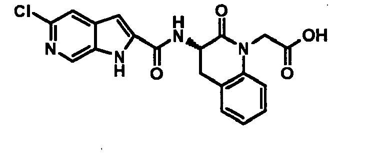

The title compound was prepared from {3-*(S)*-[(5-chloro-1H-pyrrolo[2,3-c]pyridine-2-carbonyl)amino]-2-oxo-3,4-dihydro-2H-quinolin-1-yl}acetic acid methyl ester (**Example 6**) according to **Example 7**. δ_{H} (CD₃OD): 8.50 (1H, s), 7.60 (1H, s), 7.23-7.20 (2H, m), 7.10 (1H, s), 7.01 (1H, m), 6.91 (1H, d), 4.89-4.77 (2H, m), 4.50 (1H, d), 3.23-3.08 (2H, m); m/z (ES⁺) = 399.00 [*M*+ H]⁺; RT = 3.18min.

### Example 9: (R)-5-Chloro-1H-pyrrolo[2,3-c]pyridine-2-carboxylic acid[1-(2-hydroxyethyl)-2-oxo-1,2,4-tetrahydroquinolin-3-yl}amide

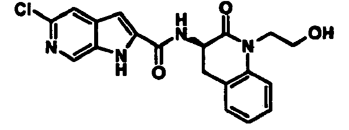

To a solution of (*R*)-5-chloro-1H-pyrrolo[2,3-c]pyridine-2-carboxylic acid{1-[2-(*tert-*butyldimethylsilyloxy)ethyl]-2-oxo-1,2,3,4-tetrahydroquinolin-3-yl}amide (**Preparation 30**, 120mg, 0.24mmol) in THF (5mL) was added a solution of TBAF in THF (1.0M, 360µl, 0.36mmol) and the reaction stirred for 16h at rt before concentrating the solvent *in vacuo.* Purification by column chromatography (SiO₂, 91:9 DCM/MeOH) afforded the title compound. δ_{H} (d₆ DMSO): 8.66 (1H, s), 7.80 (1H, s), 7.39-7.24 (4H, m), 7.09 (1H, m), 4.81-4.71 (1H, m), 4.11-3.89 (2H, br m), 3.70-3.57 (2H, m), 3.26-3.01 (2H, m); m/z (ES⁺) = 385.09 [*M*+ H]⁺; RT = 3.04min.

### Example 10: (S)-5-Chloro-1H-pyrrolo[2,3-c]pyridine-2-carboxyllic acid[1-(2-hydroxyethyl)-2-oxo-1,2,3,4-tetrahydroquinolin-3-yl}amide

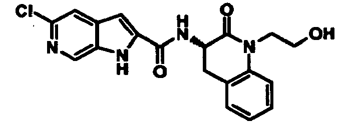

The title compound was prepared from (*S*)-5-chloro-1H-pyrrolo[2,3-c]pyridine-2-carboxylic acid{1-[2-(*tert*-butyldimethylsilyloxy)ethyl]-2-oxo-1,2,3,4-tetrahydroquinolin-3-yl} amide (**Preparation 31**) according to **Example 9**. δ_{H}(d₆ DMSO): 8.66 (1H, s), 7.80 (1H, s), 7.39-7.24 (4H, m), 7.09 (1H, m), 4.81-4.71 (1H, m), 4.11-3.89 (2H, br m), 3.70-3.57 (2H, m), 3.26-3.01 (2H, m); m/z (ES⁺) = 385.06 [M+ H]⁺; RT = 3.02min.

### Example 11: 5-Chloro-1H-pyrrolo[2,3-c]pyridine-2-carboxylic acid (7-fluoro-2-oxo-1,2,3,4-tetrahydroquinolin-3-yl)amide

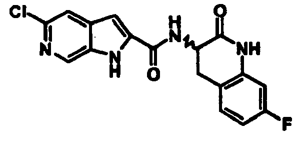

To a suspension of 3-amino-7-fluoro-3,4-dihydro-1H-quinolin-2-one hydrochloride (**Preparation 35**, 100mg, 0.4mmol) in anhydrous THF (5mL) was added DMTMM (349mg, 1.19mmol) and 4-methyl morpholine (130µL, 1.19mmol), followed by 5-chloro-1*H*-pyrrolo[2,3-c]pyridine-2-carboxylic acid (**Preparation 3**, 71mg, 0.36mmol), and the reaction stirred at rt for 16h. Solvent was concentrated *in vacuo* and the residue taken into EtOAc (50mL). Organics were washed with water (30mL), 1M HCl (30mL) and brine (30mL) before being dried (MgSO₄) and removing the solvent *in vacuo.* Trituration from methanol afforded the title compound. δ_{H}(d₆ DMSO): 12.35 (1H, s), 10.50 (1H, s), 9.07 (1H; d), 8.59 (1H, s), 7.78 (1H, s), 7.33-7.20 (2H, m), 6.83-6.64 (2H, m), 4.83-4.71 (1H, m), 3.11 (2H, d); m/z (ES⁺) = 359.06 [M + H]⁺; RT = 3.44min.

### Example 12: {3-[(5-Chloro-1H-pyrrolo[2,3-c]pyridine-2-carbonyl)amino]-7-fluoro-2-oxo-3,4-dihydro-2H-quinolin-1-yl}acetic add methyl ester

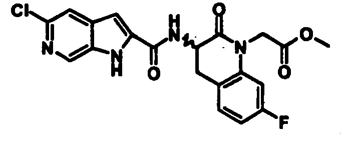

5-Chloro-1H-pyrrolo[2,3-c]pyridine-2-carboxylic acid (**Preparation 3**) was reacted with (3-amino-7-fluoro-2-oxo-3,4-dihydro-2H-quinolin-1-yl) acetic acid methyl ester (**Preparation 36**) according to **Example 11**. Purification by column chromatography (SiO₂, DCM/MeOH) afforded the title compound. δ_{H}(d₆ DMSO): 12.35 (1H, s), 9.19 (1H, d), 8.59 (1H, s), 7.79 (1H, s), 7.38-7.32 (1H, m), 7.26 (1H, s), 7.09-7.03 (1H, m), 6.95-6.88 (1H, m), 4.86-4.65 (3H, m), 3.68 (3H, s), 3.33-3.08 (2H, m); m/z (ES⁺) = 431.03 [*M*+ H]⁺; RT = 3.38min.

### Example 13: 5-Chloro-1H-pyrrolo[2,3-c]pyridine-2-carboxylic acid(6-fluoro-2-oxo-1,2,3,4-tetrahydroquinolin-3-yl)amide

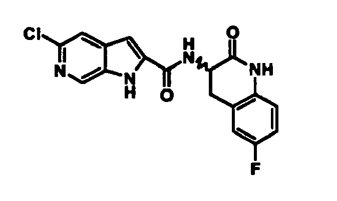

The title compound was prepared from 5-chloro-1*H*-pyrrolo[2,3-c]pyridine-2-carboxylic acid (**Preparation 3**) and the appropriate amine as described in **Example 12**. m/z (ES⁺) = 359.01 [*M*+ H]⁺; RT = 3.31 min.

### Example 14: {3-[(5-Chloro-1H-pyrrolo[2,3-c]pyridine-2-carbonyl)amino]-7-fluoro-2-oxo-3,4-dihydro-2H-quinolin-1-yl}acetic acid

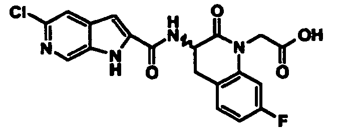

{3-[(5-Chloro-1H-pyrrolo[2,3-c]pyridine-2-carbonyl)amino]-7-fluoro-2-oxo-3,4-dihydro-2H-quinolin-1-yl}acetic acid methyl ester (**Example 12**) was hydrolysed according to the method of **Example 7** to afford the title compound. δ_{H}(d₆ DMSO): 8.59 (1H, s), 7.69 (1H, s), 7.33-7.26 (1H, m), 7.18 (1H, s), 6.87-6.79 (2H, m), 4.98-4.76 (2H, m), 4.58 (1H, d), 3.24-3.18 (2H, m); m/z (ES⁺) 417.02 [M+ H]⁺; RT = 3.14min.

### Example 15: 5-Chloro-1H-pyrrolo[2,3-c]pyridine-2-carboxylic acid [7-fluoro-1-(2-hydroxyethyl)-2-oxo-1,2,3,4-tetrahydroquinolin-3-yl}amide

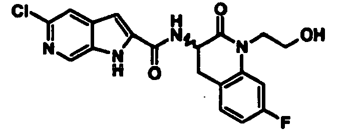

5-Chloro-1H-pyrrolo[2,3-c]pyridine-2-carboxylic acid {1-[2-(*tert-*butyldimethylsilyloxy)ethyl]-7-fluoro-2-oxo-1,2,3,4-tetrahydroquinolin-3-yl}amide (**Preparation 42**) was deprotected according to **Example 9** to afford the tide compound. δ_{H} (CD₃OD): 8.59 (1H, s), 7.68 (1H, s), 7.30-7.23 (1H, m), 7.21-7.13 (2H, m), 6.85-6.77 (1H, m), 4.90-4.80 (1H, m), 4.19-4.00 (2H, m), 3.86-3.72 (2H, m), 3.22-3.11 (2H, m); m/z (ES⁺) = 403.03 [*M*+ H]⁺; RT = 3.21min.

### Example 16: 5-Chloro-1H-pyrrolo[2,3-c]pyridine-2-carboxylic acid [1-(3-hydroxypropyl)-2-oxo-1,2,3,4-tetrahydrquinolin-3-yl]amide

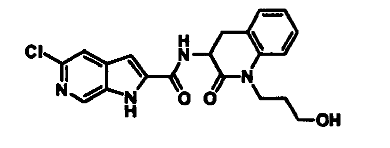

To a stirred solution of 5-chloro-1H-pyrrolo[2,3-c]pyridine-2-carboxylic acid {1-[3-(tert-butyldimethylsilanyloxy)propyl]-2-oxo-1,2,3,4-tetrahydroquinolin-3-yl}amide (**Preparation 43**, 316mg, 0.62mmol) in THF (10mL) was added TBAF (1.0M in THF, 0.92mL, 0.92mmol). The reaction was stirred at rt for 16h. The solvent was removed *in vacuo* and the residue dissolved in methanol (20mL). The precipitate which formed was filtered, washed with methanol, and dried under vacuum affording the title compound as a white crystalline solid. δ_{H} (d₆ DMSO): 1.63-1.80 (2H, m), 3.03-3.19 (2H, m), 3.44-3.50 (2H, m), 3.95-4.00 (2H, m), 4.56 (1H, t), 4.70-4.78 (1H, m), 7.05 (1H, t), 7.22-7.32 (4H, m), 7.78 (1H, s), 8.59 (1H, s), 9.10 (1H, d); m/z (ES⁺) 399 [M+H]⁺; RT = 3.11 min.

### Example 17: 5-Chloro-1H-pyrrolo[2,3-c]pyridine-2-carboxylic acid [1-(N-hydroxycarbamimidoylmethyl)-2-oxo-1,2,3,4-tetrahydroquinolin-3-yl]amide

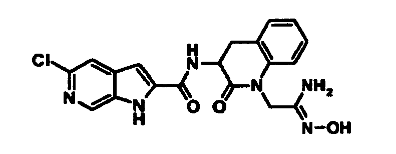

Hydroxylamine hydrochloride (188mg, 2.71mmol) was added to a stirred solution of sodium methoxide (146mg, 2.71mmol) in methanol (15mL) under an argon atmosphere. To this was added 5-chloro-1H-pyrrolo[2,3-c]pyridine-2-carboxylic acid (1-cyanomethyl-2-oxo-1,2,3,4-tetrahydroquinolin-3-yl)amide (**Example 54**, 514mg, 1.35mmol) and THF (10mL) and the reaction mixture stirred at rt for 72h. To this was added a solution of sodium methoxide (73mg,1.35mmol) and hydroxylamine hydrochloride (94mg, 1.35mmol) in methanol (5mL) and the reaction mixture stirred at rt for 24h. The solvent was removed *in vacuo* and the residue triturated with methanol affording the title compound as a white solid. δ_{H} (d₆ DMSO): 3.07 (1H, dd), 3.21 (1H, t), 4.29 (1H, d), 4.76 (1H, d), 4.79-4.86 (1H, m), 5.41 (1H, br s), 7.05 (1H, t), 7.16 (1H, d), 7.25-7.30 (3H, m), 7.79 (1H, s), 8.59 (1H, s), 9.13 (1H, d), 9.18 (1H, s), 12.36 (1H, s); m/z (ES⁺) 413 [M+H]⁺; RT = 2.67min.

### Example 18: 5-Chloro-1H-pyrrolo[2,3-c]pyridine-2-carboxylic acid [1-(2-methanesulfinylethyl)-2-oxo-1,2,3,4-tetrahydroquinolin-3-yl]amide Example 19: 5-Chloro-1H-pyrrolo[2,3-c]pyridine-2-carboxylic acid [1-(2-methanesulfonylethyl)-2-oxo-1,2,3,4-tetrahydroquinolin-3-yl] amide

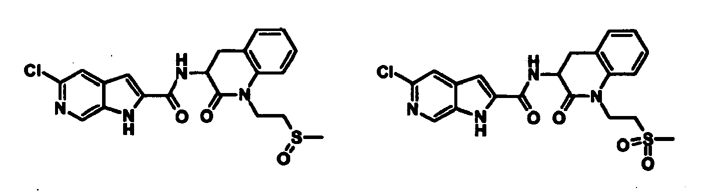

Oxone (370mg, 0.60mmol) in H₂O (10mL) was added to a suspension of 5-chloro-1H-pyrrolo[2,3-c]pyridine-2-carboxylic acid [1-(2-methylsulfanylethyl)-2-oxo-1,2,3,4-tetrahydroquinolin-3-yl]amide (**Example 58**, 250mg, 0.60mmol), in methanol (10mL). The reaction mixture was stirred at rt for 16h. The reaction mixture was diluted with EtOAc (100mL) and washed with saturated NaHCO₃ (100mL). The aqueous phase was extracted into DCM:methanol (9:1, 4 x 150mL) and the combined organics washed with brine (2x50mL), dried (MgSO₄) and concentrated *in vacuo.* The residue was purified by chromatography on silica gel eluting with DCM:methanol (97:3 to 95:5) affording the desired products as white solids.

### 5-Chloro-1H-pyrrolo[2,3-c]pyridine-2-carboxylic acid [1-(2-methanesulfinylethyl-2-oxo-1,2,3,4-tetrahydroquinolin-3-yl]amide:

δ_{H} (d₆ DMSO): 2.60, 2.61 (3H, 2xs), 2.92-3.00 (1H, m), 3.04-3.22 (3H, m), 4.28-4.34 (2H, m), 4.73-4.81 (1H, m), 7.08 (1H, t), 7.27-7.35 (4H, m), 7.79 (1H, s), 8.59 (1H, s), 9.11-9.15 (1H, m), 12.35 (1H, s); m/z (ES⁺) 431 [M+H]⁺; RT = 3.01min.

### 5-Chloro-1H-pyrrolo[2,3-c]pyridine-2-carboxylic acid [1-(2-methanesulfonylethyl)-2-oxo-1,2,3,4-tetrahydroquinolin-3-yl] amide:

δ_{H} (d₆ DMSO): 3.06 (1H, dd), 3.08 (3H, s), 3.20 (1H, app. t), 3.41-3.49 (2H, m), 4.35 (2H, t), 4.74-4.81 (1H, m), 7.09 (1H, t), 7.23 (1H, d), 7.27 (1H, s), 7.31-7.37 (2H, m), 7.79 (1H, s), 8.59 (1H, s), 9.13 (1H, d), 12.35 (1H, s); m/z (ES⁺) 447 [M+H]⁺; RT = 3.14min.

### Example 20: 5-Chloro-1H-pyrrolo[2,3-c]pyridine-2-carboxylic acid [2-oxo-1-(1H-tetrazol-5-ylmethyl)-1,2,3,4-tetrahydroquinolin-3-yl] amide

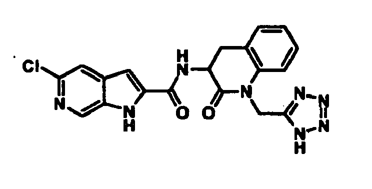

To a solution of 5-chloro-1H-pyrrolo[23-c]pyridine-2-carboxylic acid (1-cyanomethyl-2-oxo-1,2,3,4-tetrahydroquinolin-3-yl)amide (**Example 54**, 200mg, 0.53mmol) in NMP (7mL) was added sodium azide (120mg,1.84mmol) and triethylamine hydrochloride (239mg, 1.74mmol). The reaction mixture was heated to 150°C for 3h, then cooled to rt and partitioned between citric acid solution (2M, 40mL) and EtOAc (3x40mL). The combined organic fractions were washed with brine (2x30mL), dried (MgSO₄) and concentrated *in vacuo.* The residue was diluted with methanol (20mL) affording a brown precipitate which was filtered, washed with methanol, and dried under vacuum affording the title product as a brown solid. δ_{H} (d₆ DMSO): 3.12 (1H, dd), 3.29 (1H, t), 4.91-4.99 (1H, m), 5.31 (1H, d), 5.59 (1H, d), 7.07-7.10 (2H, m), 7.26-7.29 (2H, m), 7.34 (1H, d), 7.79 (1H, s), 8.59 (1H, s), 9.19 (1H, d), 12.35 (1H, s); m/z (ES⁺) 423 [M+H]⁺; RT = 3.11min.

### Example 21: 5-Chloro-1H-pyrrolo[2,3-c]pyrridine-2-carboxylic acid [1-(2-hydroxyethyl)-2-oxo-1,2,3,4-tetrahydroquinolin-3-yl]amide

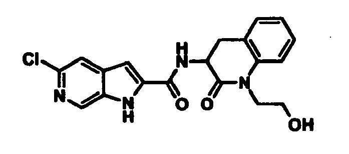

To a stirred solution of 5-chloro-1H-pyrrolo[2,3-c]pyridine-2-carboxylic acid {1-[2-(tert-butyldimethylsilanyloxy)ethyl]-2-oxo-1,2,3,4-tetrahydroquinolin-3-yl}amide (**Preparation 44,** 200mg, 0.40mmol) in THF (5mL) was added TBAF (1.0M in THF, 0.60mL, 0.60mmol). The reaction was stirred at rt for 16h. The solvent was removed *in vacuo* and the residue partitioned between water (30mL) and EtOAc (2x30mL). The combined organic fractions were dried (MgSO₄) concentrated *in vacuo* and purified by chromatography on silica gel eluting with methanol:DCM (3:47) affording the title compound as a white solid. δ_{H} (d₆ DMSO) 3.05 (1H, dd), 3.16 (1H, t), 3.55-3.64 (2H, m), 3.88-3.95 **(1H,** m), 4.01-4.08 (1H, m), 4.73-4.81 (1H, m), 4.86 (1H, t), 7.03-7.08 (1H, m), 7.27-7.31 (4H, m), 7.79 (1H, s), 8.59 (1H, s), 9.11 (1H, d), 12.35 (1H, s); m/z (ES⁺) 385 [M+H]⁺; RT = 3.15min.

### Example 22: {3-[(5-Chloro-1H-pyrrolo[2,3-c]pyridine-2-carbonyl)amino]-2-oxo-3,4-dihydro-2H-quinolin-1-yl}acetic acid methyl ester

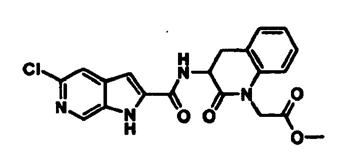

DMTMM (465mg, 1.68mmol) was added to a suspension of 5-chloro-1H-pyrrolo[2,3-c]pyridine-2-carboxylic acid (**Preparation 3,** 300mg, 1.53mmol) and (3-amino-2-oxo-3,4-dihydro-2H-quinolin-1-yl)acetic acid methyl ester (WO03/074532, 393mg, 1.68mmol) in ethanol (15mL), and the reaction mixture was stirred at rt for 16h. The solvent was removed *in vacuo* and the residue partitioned between water (50mL) and EtOAc (4x50mL). The combined organic fractions were concentrated *in vacuo* and the residue triturated with ether affording the title compound as a beige solid m/z (ES⁺) 413 [M+H]⁺; RT = 3.32min.

### Example 23: {3-[(5-Chloro-1H-pyrrolo[2,3-c]pyridine-2-carbonyl)amino]-2-oxo-3,4-dihydro-2H-quinolin-1-yl}acetic acid

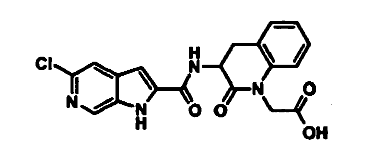

To a stirred suspension of {3-[(5-chloro-1H-pyrrolo[2,3-c]pyridine-2-carbonyl)amino]-2-oxo-3,4-dihydro-2H-quinolin-1-yl}acetic acid methyl ester (**Example 22**, 2g, 4.84mmol) in THF (100mL) was added LiOH solution (2M, 4.8mL, 9.69mmol). The reaction mixture was stirred at rt for 16h. The solvent was removed *in vacuo* and the residue dissolved in water (100mL) and acidified with hydrochloric acid solution (2M) affording a beige precipitate, which was filtered and dried under air affording the title compound as a beige solid. δ_{H} (d₆ DMSO): 3.09 (1H, dd), 3.23 (1H, t), 4.54 (1H, d), 4.74 (1H, d), 4.77-4.84 (1H, m), 7.02 (1H, d), 7.07 (1H, t), 7.27 (1H, s), 7.29-7.32 (2H, m), 7.79 (1H, s), 8.59 (1H, s), 9.19 (1H, d), 12.36 (1H, s); m/z (ES⁺) 399 [M+H]⁺; RT = 3.07min.

### Example 24: (2-{3-[(5-Chloro-1H-pyrrolo[2,3-c]pyridine-2-carbonyl)amino]-2-oxo-3,4-dihydro-2H-quinolin-1-yl}ethyl)carbamic add tert-butyl ester

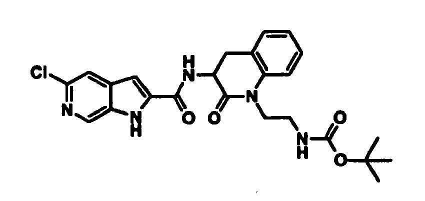

Sodium borohydride (1g, 26.3mmol) was added to a suspension of 5-chloro-1H-pyrrolo[2,3-c]pyridine-2-oxylic acid (1-cyanomethyl-2-oxo-1,2,3,4-tetrahydroquinolin-3-yl)amide (**Example 54**, 1g, 2.63mmol), cobalt chloride hexahydrate (1.25g, 5.27mmol) and tert-butyl dicarbonate (1.15g, 5.27mmol) in THF (10mL) and methanol (15mL) over a period of 15min. The reaction mixture was stirred at rt for 5 days. The reaction mixture was filtered through celite and washed with methanol:H₂O (9:1, 100mL). The filtrate was concentrated *in vacuo* and the residue partitioned between saturated NaHCO₃ solution (100mL) and EtOAc (3x50mL). The combined organic fractions were dried (MgSO₄) concentrated *in vacuo* and purified by chromatography on silica gel eluting with methanol:DCM (1:24) affording the title compound as a beige solid. δ_{H} (d₆ DMSO): 1.33 (9H, s), 3.05 (1H, dd), 3.15-3.20 (3H, m), 3.79-3.86 (1H, m), 3.97-4.04 (1H, m), 4.74-4.81 (1H, m), 7.00-7.07 (2H, m), 7.27-7.32 (4H, m), 7.78 (1H, s), 8.59 (1H, s), 9.08 (1H, d), 12.34 (1H, s); m/z (ES⁺) 484 [M+H]⁺; RT = 3.82min.

### Example 25: 5-Chloro-1H-pyrrolo[2,3-c]pyridine-2-carboxylic acid [1-(2-aminoethyl)-2-oxo-1,2,3,4-tetrahydroquinolin-3-yl]amide trifluoroacetate

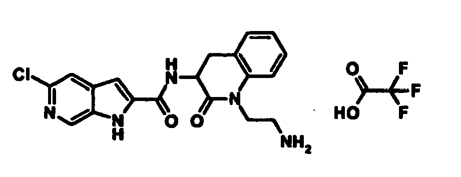

(2-{3-[(5-Chloro-1H-pyrrolo[2,3-c]pyridine-2-carbonyl)amino]-2-oxo-3,4-dihydro-2H-quinolin-1-yl}ethyl)carbamic acid tert-butyl ester (**Example 24**, 620mg, 1.28mmol) was dissolved in TFA:H₂O (9:1, 5mL) and the reaction stirred at rt for 2h. Toluene (20mL) was added to the reaction mixture and the solvent concentrated *in vacuo* affording the title compound as a yellow solid. δ_{H} (d₆ DMSO): 3.05-3.10 (3H, m), 3.22 (1H, t), 3.97-4.04 (1H, m), 4.26-4.33 (1H, m), 4.83-4.90 (1H, m), 7.09 (1H, t), 7.23-7.27 (2H, m), 7.31-7.35 (2H, m), 7.78 (1H, s), 7.94 (3H, br s), 8.60 1H, s), 9.12 (1H, d),12.36 (1H, s); m/z (ES⁺) 384 [M+H]⁺; RT = 2.65min.

### Example 26: 5-Chloro-1H-pyrrolo[2,3-c]pyridine-2-carboxylic acid [1-(2-methanesulfonylaminoethyl)-2-oxo-1,2,3,4-tetrahydroquinolin-3-yl]amide

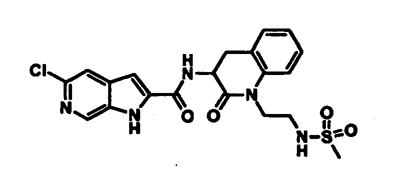

Methane sulfonyl chloride (34µL, 0.44mmol) was added to a solution of 5-chloro-1H-pyrrolo[2,3-c]pyridino-2-carboxylic acid [1-(2-aminoethyl)-2-oxo-1,2,3,4-tetrahydroquinolin-3-yl]amide trifluoroacetate (**Example 25**, 200mg, 0.40mmol) in DCM (10mL) and triethylamine (188µL, 0.84mmol) at 0°C. The reaction mixture was stirred at 0°C for 1h, then rt for 16h. The solvent was removed *in vacuo* and the residue triturated with methanol (20mL) affording the title compound as an off-white solid. m/z (ES⁺) 462 [M+H]⁺; RT = 3.26min.

### Example 27: 5-Chloro-1H-pyrrolo[2,3-c]pyridine-2-carboxylic acid [1-(2-acetylaminoethyl)-2-oxo-1,2,3,4-tetrahydroquinolin 3-yl]amide

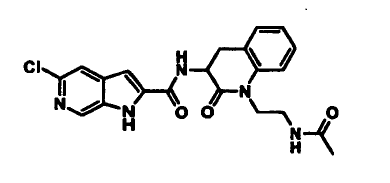

5-Chloro-1H-pyrrolo[2,3-c]pyridine-2-carboxylic acid [1-(2-amino-ethyl)-2-oxo-1,2,3,4-tetrahydroquinolin-3-yl]amide trifluoroacetate (**Example 25**, 200mg, 0.40mmol) was added to a solution of HOBt (54mg, 0.40mmol) in DMF (5mL), acetic acid (23µL, 0.40mmol) and DIPEA (0.22mL, 1.25mmol). After 5min, EDCI (100mg, 0.52mmol) was added and the reaction mixture stirred at rt for 16h. The solvent was removed *in vacuo* and the residue partitioned between water (30mL) and EtOAc (3x30mL). The combined organic fractions were dried (MgSO₄) concentrated *in vacuo* and purified by chromatography on silica gel eluting with methanol:DCM (1:19) affording the title compound as a white solid. δ_{H} (d₆ DMSO): 1.76 (3H, s), 3.05 (1H, dd), 3.14-3.17 (1H, m), 3.24-3.29 (2H, m), 3.81-3.88 (1H, m), 3.97-4.04 (1H, m), 4.74-4.81 (1H, m), 7.03-7.07 (1H, m), 7.27 (1H, s), 7.30-7.35 (3H, m), 7.78 (1H, s), 8.07 (1H, t), 8.59 (1H, s), 9.09 (1H, d), 12.34 (1H, s); m/z (ES) 426 [M+H]⁺; RT = 3.15mm.

### Example 28: 5-Chloro-1H-pyrrolo[2,3-c]pyridine-2-carboxylic acid {1-[2-(4-hydroxypiperidin-1-yl)-2-oxoethyl]-2-oxo-1,2,3,4-tetrahydroquinolin-3-yl}amide

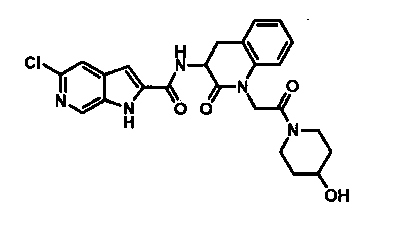

DMTMM (83mg, 0.30mmol) was added to a stirred solution of {3-[(5-chloro-1H-pyrrolo[2,3-c]pyridine-2-carbonyl)amino]-2-oxo-3,4-dihydro-2H-quinolin-1-yl}acetic acid (**Example 23**, 100mg, 0.25mmol) and 4-hydroxypiperidine hydrochloride (38mg, 0.28mmol) in ethanol (10mL) and 4-methylmorpholine (30)µL, 0.28mmol). The reaction was stirred at rt for 16h. The solvent was removed *in vacuo* and the residue triturated with H₂O affording the title compound as a white solid. m/z (ES⁺) 482 [M+H]⁺; RT = 2.97min.

### Examples 29 - 47:

The procedure described in **Example 28** was used to prepare the compounds of **Examples 29-**47 from {3-[(5-chloro-1H-pyrrolo[2,3-c]pyridine-2-carbonyl)amino]-2-oxo-3,4-dihydro-2H-quinolin-1-yl} acetic acid (**Example 23**) and the appropriate amine.

**TABLE**

| **Ex** | **Structure** | **Name** | **RT (min)** | **m/z (ES⁺)** |
|---|---|---|---|---|
| **29** | 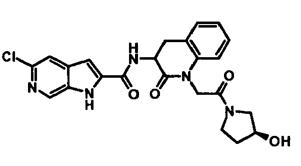 | 5-Chloro-1H-pyrrolo[2,3-c]pyridine-2-carboxylic acid {1-[2-(3-hydroxypyrrolidin-1-yl)-2-oxo-ethyl]-2-oxo-1,2,3,4-tetrahydroquinolin-3-yl } amide | 2.99 | 468 [*M* + H]⁺ |
| **30** | 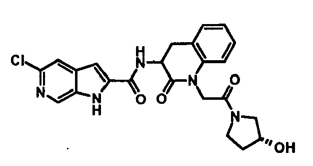 | 5-Chloro-1H-pyrrolo[2,3-c]pyridine-2-carboxylic acid {1-[2-(3-hydroxypyrrolidin-1-yl)-2-oxo-ethyl]-2-oxo-1,2,3,4-tetrahydroquinolin-3-yl}amide | 3.01 | 468 [*M* + H]⁺ |
| **31** | 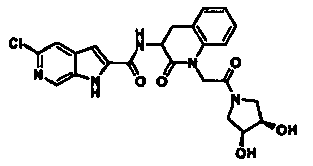 | 5-Chloro-1H-pyrrolo[2,3-c]pyridine-2-carboxylic acid {1-[2-(3,4-dihydroxy pyrrolidin-1-yl)-2-oxo-ethyl]-2 -oxo-1,2,3,4-tetrahydro-quinolin-3-yl}amide | 2.89 | 484 [*M*+H]⁺ |
| **32** | 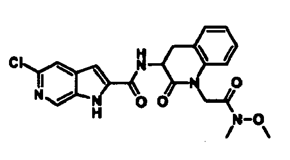 | 5-Choro-1H-pyrrolo[2,3-c]pyridine-2-carboxylic acid {1-[(methoxymethylcarbamoyl)methyl]-2-oxo-1,2,3,4-tetrahydroquinolin-3-yl}amide | 3.44 | 442 [*M* + H]⁺ |
| **33** | 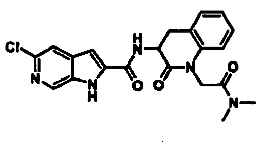 | 5-Chloro-1H-pyrrolo[2,3-c]pyridine-2-carboxylic acid (1-dimethylcarbamoylmethyl-2-oxo-1,2,3,4-tetrahydroquinolin-3-yl)amide | 2.90 | 426 [*M* + H]⁺ |
| **34** | 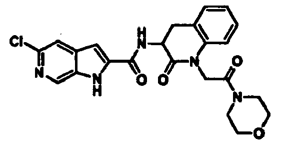 | 5-Chloro-1H-pyrrolo[2,3-c]pyridine-2-carboxylic acid [1-(2-moipholin-4-yl-2-oxo-ethyl)-2-oxo-1,2,3,4-tetrahydroquinolin-3-yl]amide | 3.04 | 468 [*M* + H]⁺ |
| **35** | 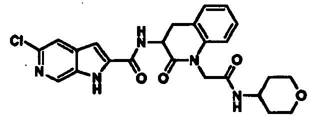 | 5-Chloro-1H-pyrrolo[2,3-c]pyridine-2-carboxylic acid {2-oxo-1-[(tetrahydropyran-4-ylcarbamoyl)methyl]-1,2,3,4-tetrahydroquinolin-3-yl}amide | 2.90 | 482 [*M* + H]⁺ |
| **36** | 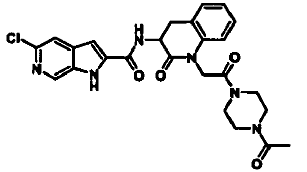 | 5-Chloro-1H-pyrrolo[2,3-c]pyridine-2-carboxylic acid {1-[2-(4-acetylpiperazin-1-yl)-2-oxo-ethyl]-2-oxo-1,2,3,4-tetrahydroquinolin-3-yl}amide | 2.99 | 509 [*M* + H]⁺ |
| **37** | 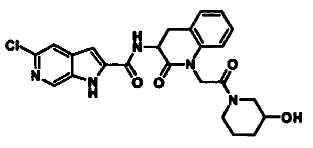 | 5-Chloro-1H-pyrrolo[2,3-c]pyridine-2-carboxylic acid {1-[2-(3-hydroxypiperidin-1-yl)-2-oxo-ethyl]-2-oxo-1,2,3,4-tetrahydroquinolin-3-yl}amide | 3.07 | 482 [*M* + H]⁺ |
| **38** | 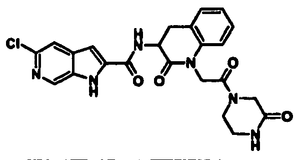 | 5-Chloro-1H-pyrrolo[2,3-c]pyridine-2-carboxylic acid {2-oxo-1-[2-oxo-2-(3-oxo-piperazin-1-yl)ethyl]-1,2,3,4-tetrahydroquinolin-3-yl}amide | 3.06 | 481 [*M* + H]⁺ |
| **39** | 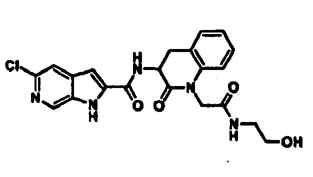 | 5-Chloro-1H-pyrrolo[2,3-c]pyridine-2-carboxylic acid {1-[(2-hydroxyethylcarbamoyl)methyl]-2-oxo-1,2,3,4-tetrahydroquinolin-3-yl}amide | 2.90 | 442 [*M* + H]⁺ |
| **40** | 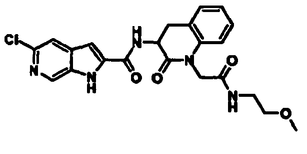 | 5-Chloro-1H-pyrrolo[2,3-c]pyridine-2-carboxylic acid {1-[(2-methoxyethylcarbamoyl)methyl]-2-oxo-1,2,3,4-tetrahydroquinolin-3-yl}amide | 3.26 | 456 [*M* + H]⁺ |
| **41** | 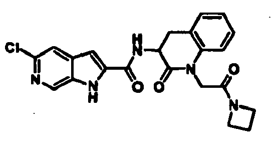 | 5-Chloro-1H-pyrrolo[2,3-c]pyridine-2-carboxylic acid [1-(2-azetidin-1-yl-2-oxoethyl)-2-oxo-1,2,3,4-tetrahydroquinolin-3-yl]amide | 3.09 | 438 [*M* + H]⁺ |
| **42** | 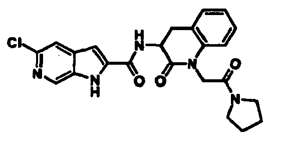 | 5-Chloro-1H-pyrrolo[2,3-c]pyridine-2-carboxylic acid [2-oxo-1-(2-oxo-2-pyrrolidin-1-yl-ethyl)-1,2,3,4-tetrahydroquinolin-3-yl]amide | 3.15 | 452 [*M*+H]⁺ |
| **43** | 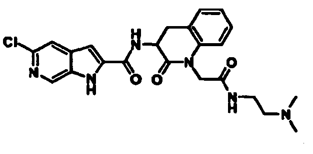 | 5-Chloro-1H-pyrrolo[2,3-c]pyridine-2-carboxylic acid {1-[(2-dimethylaminoethylcarbamoyl)methyl]-2-oxo-1,2,3,4-tetrahydroquinolin-3-yl}amide | 2.77 | 469 [*M* + H]⁺ |
| **44** | 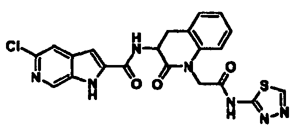 | 5-Chloro-1H-pyrrolo[2,3-c]pyridine-2-carboxylic acid [2-oxo-1-([1,3,4]thiadiazol-2-ylcarbamoylmethyl)-1,2,3,4-tetrahydroquinolin-3-yl]amide | 3.12 [*M* + H]⁺ | 482 [*M* + H]⁺ |
| **45** | 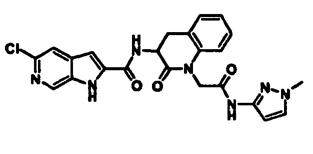 | 5-Chloro-1H-pyrrolo[2,3-c]pyridine-2-carboxylic acid {1-[(1-methyl-1H-pyrazol-3-ylcarbamoyl)methyl]-2-oxo-1,2,3,4-tetrahydroquinolin-3-yl}amide | 3.07 | 478 [*M* + H]⁺ |
| **46** | 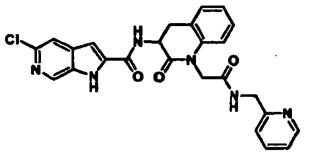 | 5-Chloro-1H-pyrrolo[2,3-c]pyridine-2-carboxylic acid (2-oxo-1-{[(Pyridin-2-ylmethyl)-carbamoyl]methyl}-1,2,3,4-tetrahydroquinolin-3-yl)amide | 2.90 | 489 [*M* + H]⁺ |
| | | | | |
| **47** | 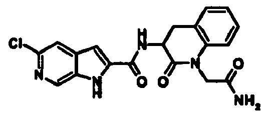 | 5-Chloro-1H-pyrrolo[2,3-c]pyridine-2-carboxylic acid (1-carbamoylmethyl-2-oxo-,2,3,4-tetrahydroquinolin-3-yl)amide | 2.92 | 398 [*M* + H]⁺ |

### Example 48: 5-Chloro-1H-pyrrolo[2,3c]pyridine-2-carboxylic acid (2-oxo-1,2,3,4-tetrahydroquinolin-3-yl)amide

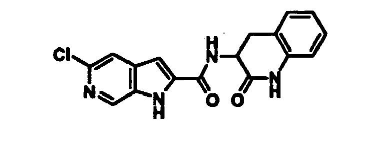

5-Chloro-1H-pyrrolo[2,3-c]pyridine-2-carboxylic acid (**Preparation 3**, 50mg 0.25mmol), 3-amino-3,4-dihydro-1H-quinolin-2-one (**Preparation 15**, 41mg, 0.25mmol) and HOBt (34mg, 0.25mmol) were dissolved in DMF (5mL) and DIPEA (89gL, 0.51mmol). After 5min, EDCI (63mg, 0.33mmol) was added and the reaction mixture stirred at rt for 16h. The solvent was removed in *vacuo* and the residue partitioned between water (30mL) and EtOAc (3x30mL): The combined organic fractions were washed with brine (20mL), dried (MgSO₄) and concentrated in *vacuo.* Purification by chromatography on silica gel eluting with methanol:DCM (1:24) afforded the title compound as a yellow solid. δ_{H} (CD₃OD): 3.26 (2H, d), 4.93 (1H, t), 6.95 (1H, d), 7.06 (1H, t), 7.24 (1H, s), 7.26-7.31 (2H, m), 7.74 (1H, s), 8.65 (1H, s); m/z (ES⁺) 341 [M+H]⁺; RT = 3.04 min.

### Examples 49 - 59:

The procedure described in **Example 48** was used to prepare the compounds of **Examples 49 - 59** from 5-chloro-1H-pyrrolo[2,3-c]pyridine-2-carboxylic acid (**Preparation 3**) and the appropriate amine.

**TABLE**

| **Ex** | **Structure** | **Amine** | **RT (min)** | **m/z (ES⁺)** |
|---|---|---|---|---|
| **49** | 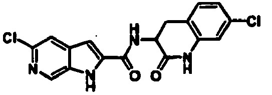 | Preparation 19 | 3.22 | 375 [*M* + H]⁺ |
| **50** | 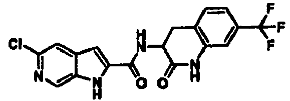 | Preparation 20 | 3.45 | 409 [*M* + H]⁺ |
| **51** | 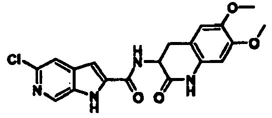 | Preparation 21 | 3.01 | 401 [*M* + H]⁺ |
| **52** | 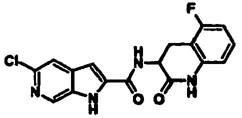 | Preparation 22 | 3.14 | 359 [*M* + H]⁺ |
| **53** | 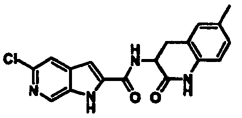 | Preparation 23 | 3.20 | 355 [*M* + H]⁺ |
| **54** | 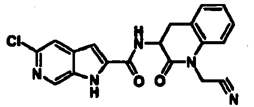 | WO03/074532 | 3.42 | 380 [*M* + H]⁺ |
| **55** | 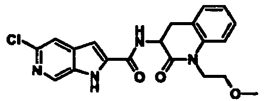 | WO03/074532 WO03/074S32 | 3.42 | 399 [*M* + H]⁺ |
| **56** | 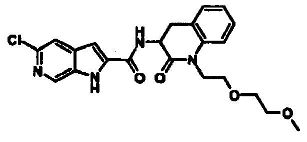 | Preparation 18 | 3.42 | 443 [*M* + H]⁺ |
| **57** | 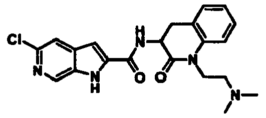 | WO03/074532 | 2.72 | 412 [*M* + H]⁺ |
| **58** | 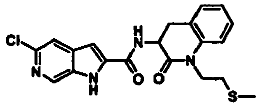 | WO03/074532 | 3.52 | 415 [*M* + H]⁺ |
| **59** | 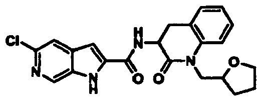 | Preparation 17 | 3.37 | 425 [*M* + H]⁺ |

### Examples 60 - 63:

The procedure described in **Example 48** was used to prepare the compounds of **Examples 60 - 63** from 5-chloro-1H-pyrrolo[2,3-b]pyridine-2-carboxylic acid (**Preparation 5**) and the appropriate amine.

**TABLE**

| **Ex** | **Structure** | **Amine** | **RT (min)** | **m/z (ES)** |
|---|---|---|---|---|
| **60** | 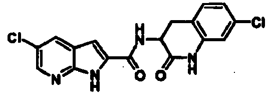 | Preparation 19 | 3.61 | 375 [*M* + H]⁺ |
| **61** | 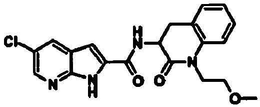 | WO03/074532 | 3.54 | 399 [*M* + H]⁺ |
| **62** | 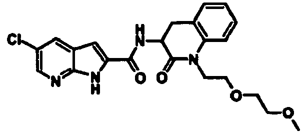 | Preparation 18 | 3.51 | 443 [*M* + H]⁺ |
| **63** | 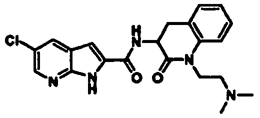 | WO03/074532 | 2.69 | 412 [*M* + H]⁺ |

### Example 64: {7-Chloro-3-[(5-chloro-1H-pyrrolo[2,3-c]pyridine-2-carbonyl)amino]-2-oxo-3,4-dihydro-2H-quinolin-1-yl}acetic acid methyl ester

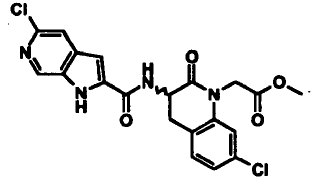

To a solution of 5-chloro-1H-pyrrolo[2,3-c]pyridine-2-carboxylic acid (**Preparation 3**, 240mg, 1.22mmol), EDCI (292mg, 1.5 1mmol) and HOBt monohydrate (190mg, 1.24mmol) in DMF (15mL) was added 3-amino-7-chloro-2-oxo-3,4-dihydro-2H-quinolin-1-yl)acetic acid methyl ester (**Preparation 50**, 330mg, 1.23mmol) and DIPEA (0.47mL, 2.70mmol). The resulting solution was stirred for 12h at rt before the reaction mixture was partitioned between EtOAc (50mL) and water/brine (100mL, 1:1). The layers were separated and the aqueous phase extracted with EtOAc (3x50mL), then the combined organics were washed with dilute HCl solution (1M, 50mL), dilute NaOH solution (1M, 50mL) and brine (50mL). The organic phase was dried (MgSO₄), filtered and concentrated *in vacuo.* Addition of methanol (~2mL) led to the precipitation of the title compound, which was obtained as an off-white solid after filtration. δ_{H} (d₆ DMSO): 3.10-3.24 (2H, m), 3.70 (3H, s), 4.72 (1H, d), 4.78-4.86 (2H, m), 7.15 (1H, dd), 7.24 (1H, d), 7.26 (1H, s), 7.36 (1H, d), 7.80 (1H, s), 8.60 (1H, s), 9.29 (1H, d), 12.36 (1H, br s); m/z (ES⁺) = 446.99 [M+H]⁺; RT = 3.45 min.

### Example 65: {7-Chloro-3-[(5-chloro-1H-pyrrolo[2,3-c]pyridine-2-carbonyl)amino]-2-oxo-3,4-dihydro-2H-quinolin-1-yl} acetic acid

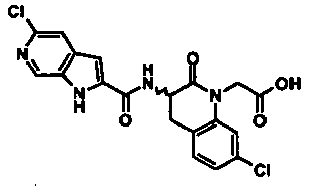

To a solution of {7-chloro-3-[(5-chloro-1H-pyrrolo[2,3-c]pyridine-2-carbonyl)amino]-2-oxo-3,4-dihydro-2H-quinolin-1-yl}acetic acid methyl ester (**Example 64**, 100mg, 0.22mmol) in a mixture of methanol and DMSO (1/1, 10mL) was added NaOH solution (1N, 0.5mL). After stirring for 2h at 80°C the solvents were removed *in vacuo* and the resulting oil taken up in water (100mL). The aqueous layer was washed with EtOAc (30mL) and then made acidic with dilute hydrochloric acid (IN). Extraction with EtOAc (4x50mL), washing of the combined extracts with brine (50mL) and concentration after drying (MgSO₄) gave a crude product. Purification by preparative thin layer chromatography on silica gel (eluent: DCM / methanol / acetic acid / water: 160 / 30 / 5 / 3) gave the title compound as colourless wax. δ_{H} (d₄ MeOH): 3.21-3.24 (2H, m), 4.61, 485 (2H, 2xd), 4.94 (1H, dd), 7.04 (1H, d), 7.10 (1H; dd), 7.19 (1H, s), 7.28 (1H, d), 7.69 (1H, s), 8.59 (1H, s); m/z (ES⁻) = 431.03 [M-H]⁻; RT = 3.26 min.

### Example 66: 5-Chloro-1H-pyrrolo[2,3-c]pyridine-2-carboxylic acid [7-chloro-1-(2-hydroxyethyl)-2-oxo-1,2,3,4-tetrahydroquinolin-3-yl]amide

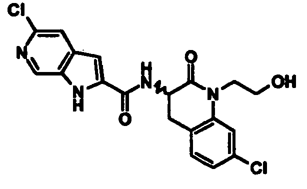

The title compound was obtained as a side product in the preparation of **Preparation 64**. TLC (hexane / ethyl acetate: 1 / 3): R_{f} 0.15; δ_{H} (d₆ DMSO): 3.07-3.18 (2H, m), 3.61 (2H, m), 3.89 (1H, m), 4.04 (1H, m), 4.81 (1H, m), 4.92 (1H, appt), 7.12 (1H, dd), 7.27 (1H, s), 7.31 (1H, d), 7.43 (1H, d), 7.80 (1H, s), 8.60 (1H, s), 9.13 (1H, d), 12.36 (1H, br s); m/z (ES⁺) = 419.11 [M+H]⁺; RT = 3.42 min.

### Example 67: 6-Chloro-1H-pyrrolo[3,2-c]pyridine-2-carboxylic acid (2-oxo-1,2,3,4-tetrahydroquinolin 3-yl)amide

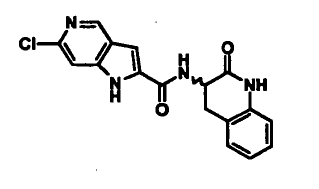

To a solution of 6-chloro-1*H*-pyrrolo[3,2-c]pyridine-2-carboxylic acid (**Preparation 14**, 40mg, 0.20mmol), EDCI (39.5mg, 0.21mmol) and HOBt monohydrate (31mg, 0.20mmol) in DMF (3mL) was added 3-amino-3,4-dihydro-1H-quinolin-2-one hydrochloride (A. L. Davies et al., Arch. Biochem. Biophys., 102, 1963, 48-51*,* 43.8mg, 0.20mmol) and DIPEA (114µL, 0.65mmol). The resulting solution was stirred for 12h at rt before the reaction mixture was partitioned between EtOAc (50mL) and water/brine (100mL, 1:1). The layers were separated and the aqueous phase extracted with EtOAc (3x50mL), then the combined organics were washed with dilute HCl solution (1M, 50mL), dilute NaOH solution (1M, 50mL) and brine (50mL). The organic phase was dried (MgSO₄), filtered and concentrated *in vacuo.* Addition of methanol (~2mL) led to the precipitation of the title compound, which was obtained as an off-white solid after filtration. δ_{H} (d₆ DMSO): 3.08-3.21 (2H, m), 4.77 (1H, m), 6.91 (1H, d), 6.97 (1H, m), 7.20-7.25 (2H, m), 7.40 (2H, apps), 8.81 (1H, s), 9,90 (1H, d), 10.41 (1H, s), 12.22 (1H, br s); m/z (ES⁺) = 340.92 [M+H]⁺; RT= 3.42 min.

### Example 68: 6-Chloro-1H-pyrrolo[3,2-c]pyridine-2-carboxylic add (7-chloro-2-oxo-1,2,3,4-tetrahydroquinolin-3-yl)amide

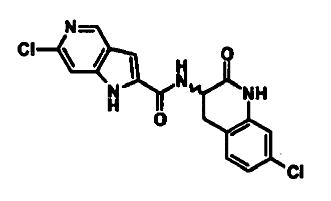

To a solution of 6-chloro-1*H*-pyrrolo[3,2-c]pyridine-2-carboxylic acid (**Preparation 14**, 40mg, 0.20mmol), EDCI (39.5mg, 0.21mmol) and HOBt monohydrate (31mg, 0.20mmol) in DMF (3mL) was added 3-amino-7-chloro-3,4-dihydro-1H-quinolin-2-one (**Preparation 19**, 43.8 mg, 0.20mmol) and DIPEA (78µL, 0.45mmol). The resulting solution was stirred for 12h at rt before the reaction mixture was partitioned between EtOAc (50mL) and water/brine (100mL, 1:1). The layers were separated and the aqueous phase extracted with EtOAc (3x50mL), then the combined organics were washed with dilute HCl solution (1M, 50mL), dilute NaOH solution (1M, 50mL) and brine (50mL). The organic phase was dried (MgSO₄), filtered and concentrated *in vacuo.* Addition of methanol (~2mL) led to the precipitation of the title compound, which was obtained as an off-white solid after filtration. δ_{H} (d₆ DMSO): 3.12-3.18 (2H, m), 4.77 (1H, m), 6.93 (1H, d), 7.02 (1H, dd), 7.27 (1H, d), 7.40 (2H, m), 8.81 (1H, s), 9.01 (1H, d), 10.52 (1H, s), 12.21 (1H, br s); m/z (ES⁺)= 374.85 [M+H]⁺; RT = 3.40 min.

### Example 69: 5-Chloro-1H-pyrrolo[2,3-c]pyridine-2-carboxylic acid (2-oxo-1,2,3,4-tetrahydro-[1,5]naphthyridin-3-yl)amide

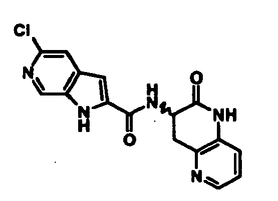

To a solution of 5-chloro-1H-pyrrolo[2,3-c]pyridine-2-carboxylic acid (**Preparation 3**, 50mg, 0.25mmol), EDCI (60mg, 0.31mmol) and HOBt monohydrate (40mg, 0.26mmol) in DMF (5mL) was added of 3-amino-3,4-dihydro-1H-[1,5]naphthyridin-2-one dihydrochloride (WO 03/074532, 60mg, 0.25mmol) and DIPEA (186µL, 1.07mmol) and the resulting solution stirred for 12h at rt. After concentration *in vacuo* the residue was partitioned between THF (50mL) and carbonate buffer (100mL, pH 10.5). The layers were separated and the aqueous phase extracted with THF (3x50mL). The combined THF fractions were washed with brine (50mL), dried (MgSO₄), filtered and concentrated. Addition of methanol (~2mL) led to the precipitation of the title compound, which was obtained as an off-white solid after filtration. δ_{H} (d₆ DMSO): 3.23 (1H, dd), 3.38 (1H, dd), 4.95 (1H, m), 7.25 (3H, m), 7.80 (1H, s), 8.14 (1H, m), 8.61 (1H, s), 9.11 (1H, d), 10.50 (1H, s), 12.37 (1H, br s); m/z (ES⁺) = 342.03 [M-2HCl+H]⁺; RT = 2.40 min.

### Examples 70-73:

The following compounds were prepared according to the method of **Example 69** from chloro-1H-pyrrolo[2,3-c]pyridine-2-carboxylic acid (**Preparation 3**) and the appropriate amine:

| **Ex** | **Structure** | **Name** | **RT (min)** | **M/z(ES⁺)** |
|---|---|---|---|---|
| **70** | 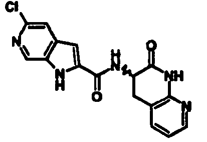 | 5-Chloro-1H-pyrrolo[2,3-c]pyridine-2-carboxylic acid (2-oxo-1,2,3,4-tetrahydro-[1,8]naphthyridin-3-yl)amide | 2.87 | 342.06 [M+H]⁺ |
| **71** | 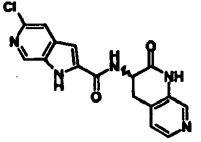 | 5-Chloro-1H-pyrrolo[2,3-c]pyridine-2-carboxylic acid (2-oxo-1,2,3,4-tetrahydro-[1,7]naphthyridin-3-yl)amide | 2.40 | 341.99 [M+H]⁺ |
| **72** | 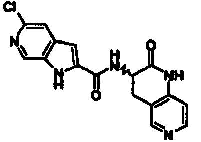 | 5-Chloro-1H-pyrrolo[2,3-c]pyridine-2-carboxylic acid (2-oxo- 1,2,3,4-tetrahydro-[1,6]naphthyridin-3-yl)amide | 2.32 | 342.00 [M+H]⁺ |
| **73** | 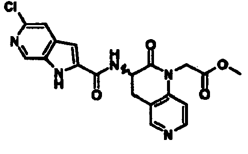 | {3-[(5-Chloro-1H-pyrrolo[2,3-c]pyridine-2-carbonyl)amino]-2-oxo-3,4-dihydro-2H-[1,6]naphthyridin-1-yl}-acetic acid methyl ester | 2.59 | 414.03 [M+H]⁺ |

### Example 74: 5-Chloro-1H-pyrrolo[2,3-c]pyridine-2-carboxylic acid [1-(2-hydroxyethyl)-2-oxo-1,2,3,4-tetrahydro-[1,5]naphthyridin-3-yl]amide

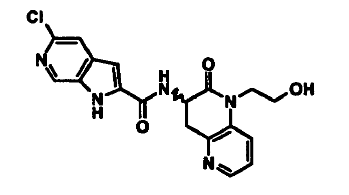

To a solution of 5-chloro-1H-pyrrolo[2,3-c]pyridine-2-carboxylic acid {1-[2-(tert-butyldimethylsilanyloxy)ethyl]-2-oxo-1,2,3,4-tetrahydro-[1,5]naphthyridin-3-yl}amide (**Preparation 63**, 176mg, 0.35mmol) in THF 10mL) was added tetrabutylammonium fluoride (0.6mL, 1N solution in THF). After 2h the solvent was removed *in vacuo.* Purification of the residue by flash chromatography on silica gel (eluent: DCM / methanol: 9/1) gave the title compound as a colourless oil. δ_{H} (d₆ DMSO): 3.18 (1H, dd), 3.40 (1H, dd), 3,61 (2H, m), 3.93, 4.03 (2H, 2xm), 4.89 (1H, appt), 4.96 (1H, m), 7.28 (1H, s), 7.34 (1H, dd), 7.73 (1H, d), 7.80 (1H, s), 8.20 (1H, d), 8.60 (1H, s), 9.16 (1H, d), 12.38 (1H, br s); m/z (ES⁺) = 386.04 [M+H]⁺; RT = 2.67 min.

### Example 75: {3-[(5-Chloro-1H-pyrrolo[2,3-c]pyridine-2-carbonyl)amino]-2-oxo-3,4-dihydro-2H-[1,5]naphthyridin-1-yl)acetic acid methyl ester

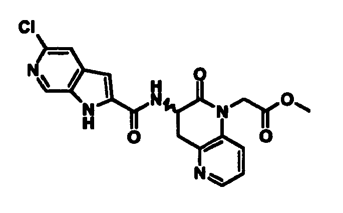

The title compound was prepared according to the method of **Example 69** from chloro-1H-pyrrolo[2,3-c]pyridino-2-carboxylic acid (**Preparation 3**) and 3-amino-2-oxo-3,4-dihydro-2H-[1,5]naphthyridin-1-yl)acetic acid methyl ester (**Preparation 60**). δ_{H} (d₆ DMSO): 3.22 (1H, dd), 3.48 (1H, dd), 3.69 (3H, s), 4.70, 4.84 (2H, 2d), 5.00 (1H, ddd), 7.27 (1H, s), 7.36 (1H, dd), 7.51 (1H, d), 7.80 (1H, s), 8.24 (1H, d), 8.60 (1H, s), 9.23 (1H, d), 12.37 (1H, br s); m/z (ES⁺) = 414.05 [M+H]⁺; RT = 2.90 min.

### In vitro GP activity

### Materials

α-D-Glucose-1-phosphate (disodium salt), Glycogen, D-Glucose, Malachite Green Hydrochloride, Ammonium Molybdate tetrahydrate, BSA, HEPES and rabbit muscle phosphorylase a (P1261) were purchased from Sigma. All other reagents were analytical grade.

### Method

### Glycogen phosphorylase assay in vitro:

An assay for glycogen phosphorylase activity in the reverse direction was developed based on the method described by Engers et al., Can. J Biochem.,1970, 48, 746-754]. Rabbit muscle glycogen phosphorylase a (Sigma) was reconstituted at a stock concentration of 100µg/mL in 25mM Tris/HCl. The pH was measured in a 96-well plate in a final volume of 100µL containing 50mM Hepes pH 7.2, 7.5mM glucose, 0.5mM glucose-1-phosphate and 1mg/mL glycogen. After incubation at 30°C for 30min, the inorganic phosphate released from glucose-1-phosphate was measured by the addition of 150µL of malachite green/molybdate solution prepared as follows: 5mL of 4.2% ammonium molybdate in 4N HCl, 15mL of 0.045% malachite green, 50µL of Tween 20. Following a 30min incubation at rt, the absorbance was measured at 620nm. For IC₅₀ determination, 10µL of a serial dilution of compound (100µM to 0.004µM) in DMSO was added to each reaction in duplicate with the equivalent concentration of DMSO added to the control uninhibited reaction. Dose response curves were then obtained by plotting % inhibition versus log₁₀ compound concentration. IC₅₀ is defined as the concentration of compound achieving 50% inhibition under the assay conditions described.

The **Examples** have an IC₅₀ of < 1mM. It is advantageous that the measured IC₅₀ be lower than 100µM. It is still more advantageous for the IC₅₀ to be lower than 50µM. It is even more advantageous for the IC₅₀ to be lower than 5µM. It is yet more advantageous for the IC₅₀ to be lower than 0.5µM.

## Claims

1. A compound of Formula (I): 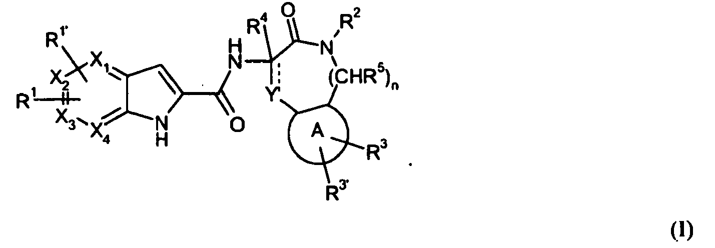 or a stereoisomer, or a pharmaceutically acceptable salt thereof, wherein:
one of X₁, X₂, X₃ and X₄ is N and the others are C;
---- is a single or double bond;
when ---- is a single bond Y is CHR⁶, NH, O, S, SO₂, CHR⁶O, CHR⁶S, CHR⁶SO₂, CHR⁶CO or CH₂CHR⁶; and when ---- is a double bond Y is GR⁶ or N;
A is aryl or heteroaryl;
R¹ and R¹ are independently selected from hydrogen, halogen, hydroxy, cyano, C₁₋₆alkyl, C₁₋₆alkoxy, fluoromethyl, difluoromethyl, trifluoromethyl, C₂₋₆alkenyl, C₂₋₆alkynyl, aryl, -C₁₋₆alkylaryl, - C₁₋₆alkylheteroaryl and aryloxy;
R² is hydrogen, C₁₋₆alkyl optionally substituted by cyano, O-C₁₋₄alkyl-OR⁷, OR⁷, COOR⁷, CONR⁸R⁹, CONR⁸OR⁹, C(NH₂)=NOH, NR¹⁶R¹⁷, NHC(O)OR¹⁶, NHS(O)₂R¹⁸, NHC(O)R¹⁸, SR¹⁶, S(O)R¹⁸ or S(O)₂R¹⁸; or R² is C₁₋₄alkyl-CONR¹⁰R¹¹, C₂₋₆alkenyl, aryl, -C₁₋₆alkylaryl, -C₁₋₆alkylheterocyclyl or -C₁₋₆alkylheteroaryl;
R³and R³ are independently selected from hydrogen, halogen, hydroxy, cyano, C₁₋₄alkyl, C₁₋₄alkoxy, fluoromethyl, difluoromethyl, trifluoromethyl, ethenyl and ethynyl;
when ---- is a single bond R⁴ is hydrogen, C₁₋₆alkyl, aryl, or C₂₋₆alkenyl or when ---- is a double bond R⁴ is absent;
R⁵ and R⁶ are independently selected from hydrogen, C₁₋₆alkyl, aryl, C₂₋₆alkenyl, cyano, tetrazole, COOR¹² CONR¹²R¹³ and CONR¹²OR¹³.
R⁷, R⁸ and R⁹ are independently selected from hydrogen and C₁₋₄alkyl;
R¹⁰ and R¹¹ are independently selected from hydrogen, C₁₋₄alkyl optionally substituted by OR⁷, COOR⁷ or NR¹⁴R¹⁵, aryl, heteroaryl, C₃₋₇cycloalkyl, heterocyclyl, -C₁₋₄alkylaryl, -C₁₋₄alkylheteroaryl, -C₁₋₄alkylC₃₋₇cycloalkyl or -C₁₋₄alkylheterocyclyl wherein any of the rings is optionally substituted by 1 or 2 substituents independently selected from halogen, hydroxy, cyano, C₁₋₄alkyl, C₁₋₄alkoxy, fluoromethyl, difluoromethyl and trifluoromethyl;
or R¹⁰ and R¹¹ together with the nitrogen to which they are attached form a 4- to 7-membered heterocycle optionally containing a further heteroatom selected from N and O, which heterocycle is optionally substituted by C₁₋₄alkyl, halo, OR⁷ or COR⁷, or two bonds on a ring carbon of the heterocycle optionally can form an oxo (=O) substituent;
R¹²and R¹³ are independently selected from hydrogen, C₁₋₄alkyl, aryl, -C₁₋₄alkylaryl and -C₁₋₄alkylheteroaryl;
or R¹² and R¹³ may be cyclised to form an optionally substituted 4- to 7-membered heterocycle;
R¹⁴ and R¹⁵ are independently selected from hydrogen and C₁₄alkyl;
or R¹⁴ and R¹⁵ together with the nitrogen to which they are attached form a 4- to 7-membered heterocycle optionally containing a further heteroatom selected from N and O, which heterocycle is optionally substituted by C₁₋₄alkyl, halo, OR⁷ or COR⁷, or two bonds on a ring carbon of the heterocycle optionally can form an oxo ( =O ) substituent;
R¹⁶ and R¹⁷ are independently selected from hydrogen and C₁₋₄alkyl;
R¹⁸ is C₁₋₄alkyl; and
n is 0 or 1.

2. A compound according to claim 1 of Formula (1a): 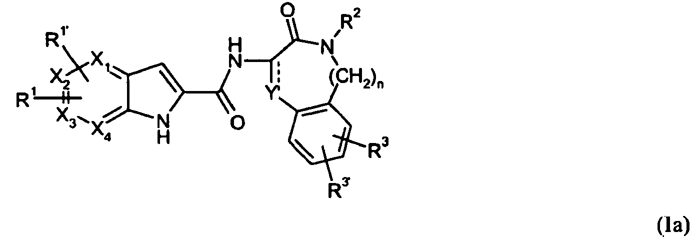 or a stereoisomer, or a pharmaceutically acceptable salt thereof, wherein:
one of X₁, X₂, X₃ and X₄ is N and the others are C;
---- is a single or double bond;
when ---- is a single bond Y is CH₂, NH or O; and when ---- is a double bond Y is CH or N;
R¹ and R^{1'} are independently selected from hydrogen, halogen, hydroxy, cyano, C₁₋₄alkyl, C₁₋₄alkoxy, fluoromethyl, difluoromethyl, trifluoromethyl, ethenyl and ethynyl;
R² is hydrogen, C₁₋₄atkyl optionally substituted by cyano, O-C₁₋₄alkyl-OR⁴, OR⁴, COOR⁴, CONR⁵R⁶, CONR⁵OR⁶, C(NH₂)=N(OH), NR¹⁶R¹⁷, NHC(O)OR¹⁶, NHS(O)₂R¹⁸, NHC(O)R¹⁸, SR¹⁶, S(O)R¹⁸ or S(O)₂R¹⁸; or R² is C₁₋₄alkyl-CONR⁷R⁸ or -C₁₋₄alkylheterocyclyl;
R³ and R^{3'} are independently selected from hydrogen, halogen, hydroxy, cyano, C₁₋₄alkyl, C₁₋₄alkoxy, fluoromethyl, difluoromethyl, trifluoromethyl, ethenyl and ethynyl;
R⁴, R⁵ and R⁶ are independently selected from hydrogen and C₁₋₄alkyl;
R⁷ and R⁸ are independently selected from hydrogen, C₁₋₄alkyl optionally substituted by OR⁴, COOR⁴ or NR⁹R¹⁰, aryl, heteroaryl, C₃₋₇cycloalkyl, heterocyclyl, -C₁₋₄alkylaryl, -C₁₋₄alkylheteroaryl, - C₁₋₄alkylC₃₋₇cycloalkyl or -C₁₋₄alkylheterocyclyl wherein any of the rings is optionally substituted by or 2 substituents independently selected from halogen, hydroxy, cyano, C₁₋₄alkyl, C₁₋₄alkoxy, fluoromethyl, difluoromethyl and trifluoromethyl;
or R⁷ and R⁸ together with the nitrogen to which they are attached form a 4- to 7-membered heterocycle optionally containing a further heteroatom selected from N and O, which heterocycle is optionally substituted by C₁₋₄alkyl, halo, OR⁴ or COR⁴, or two bonds on a ring carbon of the heterocycle optionally can form an oxo ( =O ) substituent;
R⁹ and R¹⁰ are independently selected from hydrogen and C₁₋₄alkyl;
or R⁹ and R¹⁰ together with the nitrogen to which they are attached form a 4- to 7-membered heterocycle optionally containing a further heteroatom selected from N and O, which heterocycle is optionally substituted by C₁₋₄alkyl, halo, OR⁴ or COR⁴, or two bonds on a ring carbon of the heterocycle optionally can form an oxo ( =O ) substituent;
R¹⁶ and R¹⁷ are independently selected from hydrogen and C₁₋₄alkyl;
R¹⁸ is C₁₋₄alkyl; and
n is 0 or 1.

3. A compound according to claim 1 or 2, or a stereoisomer, or a pharmaceutically acceptable salt thereof, wherein X₃ is N.

4. A compound according to any one of the preceding claims, or a stereoisomer, or a pharmaceutically acceptable salt thereof, wherein R¹ and R^{1'} are each independently, halogen, cyano or hydrogen.

5. A compound according to claim 4, or a stereoisomer, or a pharmaceutically acceptable salt thereof, wherein one of R¹ and R^{1'} is hydrogen and the other is a 5-halo or 5-cyano group.

6. A compound according to any one of the preceding claims, or a stereoisomer, or a pharmaceutically acceptable salt thereof, wherein when R² is C₁₋₄alkyl-CO-NR⁵R⁶ or R² is C₁₋₆alkyl-CO-NR⁸R⁹ as the case may be it is CH₂-CO-NR⁵R⁶ or CH₂-CO-NR⁸R⁹ as the case may be.

7. A compound according to any one of the preceding claims, or a stereoisomer, or a pharmaceutically acceptable salt thereof, wherein R³ and R^{3'} are independently selected from hydrogen, halogen, C₁₋₄alkyl, C₁₋₄alkoxy, and trifluoromethyl.

8. A compound according to claim 7, or a stereoisomer, or a pharmaceutically acceptable salt thereof, wherein at least one of R³ and R^{3'} is hydrogen.

9. A compound according to any one of the preceding claims, or a stereoisomer, or a pharmaceutically acceptable salt thereof, wherein n is 0.

10. A compound selected from any one of Examples 1 to 75, as the free base or a pharmaceutically acceptable salt thereof.

11. A composition comprising a compound according to any one of claims 1 to 10, or a stereoisomer, or a pharmaceutically acceptable salt thereof, and a pharmaceutically acceptable carrier.

12. A compound according to any one of claims 1 to 10, or a stereoisomer, or a pharmaceutically acceptable salt thereof, for use in the prophylactic or therapeutic treatment of hyperglycemia or diabetes.

13. A compound according to any one of claims 1 to 10, or a stereoisomer, or a pharmaceutically acceptable salt thereof, for use in the prevention of diabetes in a human demonstrating pre-diabetic hyperglycemia or impaired glucose tolerance.

14. A compound according to any one of claims 1 to 10, or a stereoisomer, or a pharmaceutically acceptable salt thereof, for use in the prophylactic or therapeutic treatment of hypercholesterolemia, hyperinsulinemia, hyperlipidemia, atherosclerosis or myocardial ischemia, or as a cardioprotectant.

15. The use of a compound according to any one of claims 1 to 10, or a stereoisomer or a pharmaceutically acceptable salt thereof, in the manufacture of a medicament for the prophylactic or therapeutic treatment of a disease or disorder as defined in any one of claims 12 to 14.

16. A process for the production of a compound of Formula (1) according to claim 1, comprising coupling a carboxylic acid of Formula (II), or a protected or activated derivative thereof, with an amine of Formula (III):

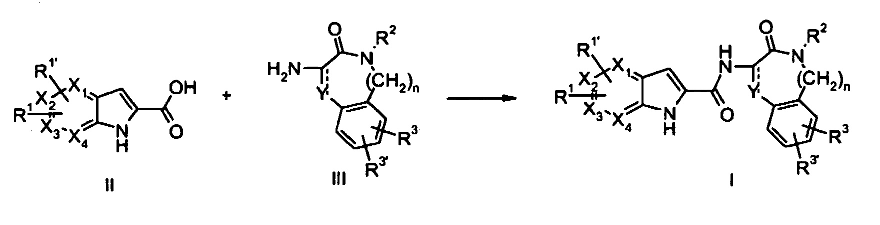

## Patentansprüche

1. Verbindungen der Formel (I): 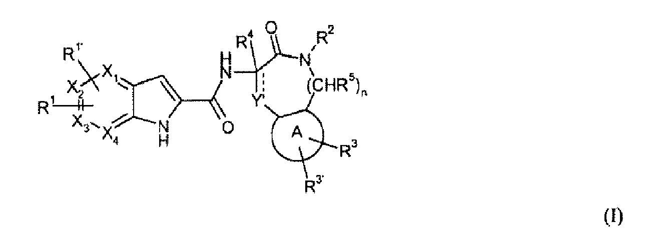 und deren stereoisomere und pharmazeutisch unbedenkliche Salze, wobei:
einer der Reste X₁, X₂, X₃ und X₄ für N steht und die anderen für C stehen;
---- für eine Einfachbindung oder eine Doppelbindung steht;
wenn ---- für eine Einfachbindung steht, Y für CHR⁶, NH, O, S, SO₂, CHR⁶O, CHR⁶S, CHR⁶SO₂, CHR⁶CO oder CH₂CHR⁶; und wenn ---- für eine Doppelbindung steht, Y für CR⁶ oder N steht;
A für Aryl oder Heteroaryl steht;
R¹ und R^{1'} unabhängig voneinander ausgewählt sind aus Wasserstoff, Halogen, Hydroxy, Cyano, C₁₋₆-Alkyl, C₁₋₆-Alkoxy, Fluormethyl, Difluormethyl, Trifluormethyl, C₂₋₆-Alkenyl, C₂₋₆-Alkinyl, Aryl, -C₁₋₆-Alkylaryl, -C₁₋₆-Alkylheteroaryl und Aryloxy;
R² für Wasserstoff, C₁₋₆-Alkyl, gegebenenfalls substituiert durch Cyano, O-C₁₋₄-Alkyl-OR⁷, OR⁷, COOR⁷, CONR⁸R⁹, CONR⁸OR⁹, C (NH₂) =NOH, NR¹⁶R¹⁷, NHC(O)OR¹⁶, NHS (O)₂R¹⁸, NHC(O)R¹⁸, SR¹⁶, S (O) R¹⁸ oder S (O) ₂R¹⁸ steht; oder R² für C₁₋₄-Alkyl-CONR¹⁰R¹¹, C₂₋₆-Alkenyl, Aryl, -C₁₋₆-Alkylaryl, -C₁₋₆-Alkylheterocyclyl oder -C₁₋₆-Alkylheteroaryl steht; R³ und R^{3'} unabhängig voneinander ausgewählt sind aus Wasserstoff, Halogen, Hydroxy, Cyano, C₁₋₄-Alkyl, C₁₋₄-Alkoxy, Fluormethyl, Difluormethyl, Trifluormethyl, Ethenyl und Ethinyl;
wenn ---- für eine Einfachbindung steht, R⁴ für Wasserstoff, C₁₋₆-Alkyl, Aryl oder C₂₋₆-Alkenyl steht, oder, wenn ---- für eine Doppelbindung steht, R⁴ fehlt;
R⁵ und R⁶ unabhängig voneinander ausgewählt sind aus Wasserstoff, C₁₋₆-Alkyl, Aryl, C₂₋₆-Alkenyl, Cyano, Tetrazol, COOR¹², CONR¹²R¹³ und CONR¹²OR¹³;
R⁷, R⁸ und R⁹ unabhängig voneinander ausgewählt sind aus Wasserstoff und C₁₋₄-Alkyl;
R¹⁰ und R¹¹ unabhängig voneinander ausgewählt sind aus Wasserstoff, C₁₋₄-Alkyl, gegebenenfalls substituiert durch OR⁷, COOR⁷ oder NR¹⁴R¹⁵, Aryl, Heteroaryl, C₃₋₇-Cycloalkyl, Heterocyclyl, -C₁₋₄-Alkylaryl, -C₁₋₄-Alkylheteroaryl, -C₁₋₄-Alkyl-C₃₋₇-cycloalkyl oder -C₁₋₄-Alkylheterocyclyl, wobei die Ringe jeweils gegebenenfalls durch einen oder zwei Substituenten unabhängig voneinander ausgewählt aus Halogen, Hydroxy, Cyano, C₁₋₄-Alkyl, C₁₋₄-Alkoxy, Fluormethyl, Difluormethyl und Trifluormethyl substituiert sind;
oder R¹⁰ und R¹¹ zusammen mit dem Stickstoff, an den sie gebunden sind, einen 4- bis 7gliedrigen Heterocyclus bilden, der gegebenenfalls ein weiteres aus N und O ausgewähltes Heteroatom enthält, wobei der Heterocyclus gegebenenfalls substituiert ist durch C₁₋₄-Alkyl, Halogen, OR⁷ oder COR⁷, oder zwei Bindungen an einem Ringkohlenstoffatom des Heterocyclus gegebenenfalls einen Oxosubstituenten (=O) bilden können;
R¹² und R¹³ unabhängig voneinander ausgewählt sind aus Wasserstoff, C₁₋₄-Alkyl, Aryl, -C₁₋₄-Alkylaryl und -C₁₋₄-Alkylheteroaryl;
oder R¹² und R¹³ unter Bildung eines gegebenenfalls substituierten 4- bis 7gliedrigen Heterocyclus cyclisiert sein können;
R¹⁴ und R¹⁵ unabhängig voneinander ausgewählt sind aus Wasserstoff und C₁₋₄-Alkyl;
oder R¹⁴ und R¹⁵ zusammen mit dem Stickstoff, an den sie gebunden sind, einen 4- bis 7gliedrigen Heterocyclus bilden, der gegebenenfalls ein weiteres aus N und O ausgewähltes Heteroatom enthält, wobei der Heterocyclus gegebenenfalls substituiert ist durch C₁₋₄-Alkyl, Halogen, OR⁷ oder COR⁷, oder zwei Bindungen an einem Ringkohlenstoffatom des Heterocyclus gegebenenfalls einen Oxosubstituenten (=O) bilden können;
R¹⁶ und R¹⁷ unabhängig voneinander ausgewählt sind aus Wasserstoff und C₁₋₄-Alkyl;
R¹⁸ für C₁₋₄-Alkyl steht; und
n für 0 oder 1 steht.

2. Verbindungen nach Anspruch 1 der Formel (1a): 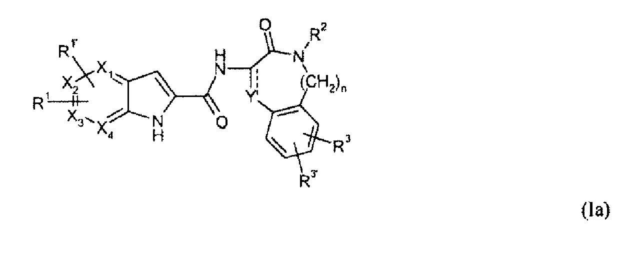 und deren Stereoisomere und pharmazeutisch unbedenkliche Salze, wobei:
einer der Reste X₁, X₂, X₃ und X₄ für N steht und die anderen für C stehen;
---- für eine Einfachbindung oder Doppelbindung steht;
wenn ---- für eine Einfachbindung steht, Y für CH₂, NH oder O steht; und wenn ---- für eine Doppelbindung steht, Y für CH oder N steht;
R¹ und R^{1'} unabhängig voneinander ausgewählt sind aus Wasserstoff, Halogen, Hydroxy, Cyano, C₁₋₄-Alkyl, C₁₋₄-Alkoxy, Fluormethyl, Difluormethyl, Trifluormethyl, Ethenyl und Ethinyl;
R² für Wasserstoff, C₁₋₄-Alkyl, gegebenenfalls substituiert durch Cyano, O-C₁₋₄-Alkyl-OR⁴, OR⁴, COOR⁴, CONR⁵R⁶ CONR⁵OR⁶, C (NH₂) =N(OH), NR¹⁶R¹⁷, NHC(O)OR¹⁶, NHS(O)₂R¹⁸, NHC(O)R¹⁸, SR¹⁶, S (O) R¹⁸ oder S (O) ₂R¹⁸ steht; oder R² für C₁₋₄-Alkyl-CONR⁷R⁸ oder -C₁₋₄-Alkylheterocyclyl steht;
R³ und R^{3'} unabhängig voneinander ausgewählt sind aus Wasserstoff, Halogen, Hydroxy, Cyano, C₁₋₄-Alkyl, C₁₋₄-Alkoxy, Fluormethyl, Difluormethyl, Trifluormethyl, Ethenyl und Ethinyl;
R⁴, R⁵ und R⁶ unabhängig voneinander ausgewählt sind aus Wasserstoff und C₁₋₄-Alkyl;
R⁷ und R⁸ unabhängig voneinander ausgewählt sind aus Wasserstoff, C₁₋₄-Alkyl, gegebenenfalls substituiert durch OR⁴, COOR⁴ oder NR⁹R¹⁰, Aryl, Heteroaryl, C₃₋₇-Cycloalkyl, Heterocyclyl, -C₁₋₄-Alkylaryl, -C₁₋₄-Alkylheteroaryl, -C₁₋₄-Alkyl-C₃₋₇-cycloalkyl oder -C₁₋₄-Alkylheterocyclyl, wobei die Ringe jeweils gegebenenfalls durch einen oder zwei Substituenten unabhängig voneinander ausgewählt aus Halogen, Hydroxy, Cyano, C₁₋₄-Alkyl, C₁₋₄-Alkoxy, Fluormethyl, Difluormethyl und Trifluormethyl substituiert sind;
oder R⁷ und R⁸ zusammen mit dem Stickstoff, an den sie gebunden sind, einen 4- bis 7gliedrigen Heterocyclus bilden, der gegebenenfalls ein weiteres aus N und O ausgewähltes Heteroatom enthält, wobei der Heterocyclus gegebenenfalls substituiert ist durch C₁₋₄-Alkyl, Halogen, OR⁴ oder COR⁴, oder zwei Bindungen an einem Ringkohlenstoffatom des Heterocyclus gegebenenfalls einen Oxosubstituenten (=O) bilden können;
R⁹ und R¹⁰ unabhängig voneinander ausgewählt sind aus Wasserstoff und C₁₋₄-Alkyl;
oder R⁹ und R¹⁰ zusammen mit dem Stickstoff, an den sie gebunden sind, einen 4- bis 7gliedrigen Heterocyclus bilden, der gegebenenfalls ein weiteres aus N und O ausgewähltes Heteroatom enthält, wobei der Heterocyclus gegebenenfalls substituiert ist durch C₁₋₄-Alkyl, Halogen, OR⁴ oder COR⁴, oder zwei Bindungen an einem Ringkohlenstoffatom des Heterocyclus gegebenenfalls einen Oxosubstituenten (=O) bilden können;
R¹⁶ und R¹⁷ unabhängig voneinander ausgewählt sind aus Wasserstoff und C₁₋₄-Alkyl;
R¹⁸ für C₁₋₄-Alkyl steht; und
n für 0 oder 1 steht.

3. Verbindungen nach Anspruch 1 oder 2 und deren Stereoisomere und pharmazeutisch unbedenkliche Salze, wobei X₃ für N steht.

4. Verbindungen nach einem der vorhergehenden Ansprüche und deren Stereoisomere und pharmazeutisch unbedenkliche Salze, wobei R¹ und R^{1'} jeweils unabhängig voneinander für Halogen, Cyano oder Wasserstoff stehen.

5. Verbindungen nach Anspruch 4 und deren Stereoisomere und pharmazeutisch unbedenkliche Salze, wobei einer der Reste R¹ und R^{1'} für Wasserstoff und der andere für eine 5-Halogen- oder 5-Cyanogruppe steht.

6. Verbindungen nach einem der vorhergehenden Ansprüche und deren Stereoisomere und pharmazeutisch unbedenkliche Salze, wobei, wenn R² für C₁₋₄-Alkyl-CO-NR⁵R⁶ steht oder R² für C₁₋₆-Alkyl-CO-NR⁸R⁹ steht, dieses für CH₂-CO-NR⁵R⁶ bzw. CH₂-CO-NR⁸R⁹ steht.

7. Verbindungen nach einem der vorhergehenden Ansprüche und deren Stereoisomere und pharmazeutisch unbedenkliche Salze, wobei R³ und R^{3'} unabhängig voneinander ausgewählt sind aus Wasserstoff, Halogen, C₁₋₄-Alkyl, C₁₋₄-Alkoxy und Trifluormethyl.

8. Verbindungen nach Anspruch 7 und deren Stereoisomere und pharmazeutisch unbedenkliche Salze, wobei wenigstens einer der Reste R³ und R^{3'} für Wasserstoff steht.

9. Verbindungen nach einem der vorhergehenden Ansprüche und deren Stereoisomere und pharmazeutisch unbedenkliche Salze, wobei n für 0 steht.

10. Verbindungen ausgewählt aus einem der Beispiele 1 bis 75 als freie Base oder pharmazeutisch unbedenkliches Salz davon.

11. Zusammensetzung, enthaltend eine Verbindung nach einem der Ansprüche 1 bis 10 oder ein Stereoisomer oder ein pharmazeutisch unbedenkliches Salz davon und einen pharmazeutisch unbedenklichen Träger.

12. Verbindungen nach einem der Ansprüche 1 bis 10 und deren Stereoisomere und pharmazeutisch unbedenkliche Salze zur Verwendung bei der prophylaktischen oder therapeutischen Behandlung von Hyperglykämie oder Diabetes.

13. Verbindungen nach einem der Ansprüche 1 bis 10 und deren Stereoisomere und pharmazeutisch unbedenkliche Salze zur Verwendung bei der Prävention von Diabetes in einem Menschen, der prädiabetische Hyperglykämie oder eine gestörte Glukosetoleranz zeigt.

14. Verbindungen nach einem der Ansprüche 1 bis 10 und deren Stereoisomere und pharmazeutisch unbedenkliche Salze zur Verwendung bei der prophylaktischen oder therapeutischen Behandlung von Hypercholesterinämie, Hyperinsulinämie, Hyperlipidämie, Atherosklerose oder myokardialer Ischämie, oder als herzschützendes Mittel.

15. Verwendung einer Verbindung nach einem der Ansprüche 1 bis 10 oder eines Stereoisomers oder eines pharmazeutisch unbedenklichen Salzes davon bei der Herstellung eines Medikaments zur prophylaktischen oder therapeutischen Behandlung einer wie in einem der Ansprüche 12 bis 14 definierten Krankheit bzw. Erkrankung.

16. Verfahren zur Herstellung einer Verbindung der Formel (I) nach Anspruch 1, bei dem man eine Carbonsäure der Formel (II) oder ein geschütztes oder aktiviertes Derivat davon mit einem Amin der Formel (III) koppelt:

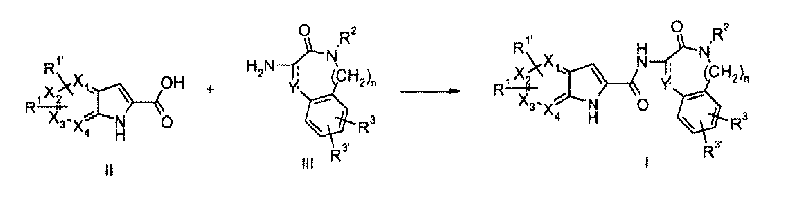

## Revendications

1. Composé de formule (I) : 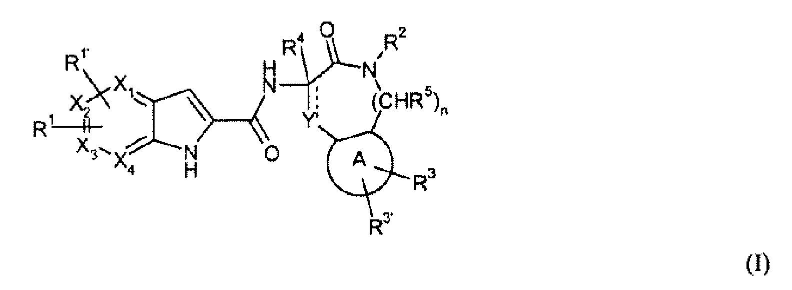 ou un stéréoisomère, ou un sel pharmaceutiquement acceptable de celui-ci, dans laquelle :
l'un parmi X₁, X₂, X₃ et X₄ est un atome d'azote et les autres sont un atome de carbone ;
---- est une liaison simple ou double ;
lorsque ---- est une liaison simple, Y est un groupe CHR⁶, un groupe NH, un atome d'oxygène, un atome de soufre, un groupe SO₂, un groupe CHR⁶O, un groupe CHR⁶S, un groupe CHR⁶SO₂, un groupe CHR⁶CO ou un groupe CH₂CHR⁶ ; et lorsque ---- est une double liaison, Y est un groupe CR⁶ ou un atome d'azote ;
A est un groupe aryle ou un groupe hétéroaryle ;
R¹ et R^{1'} sont choisis indépendamment parmi un atome d'hydrogène, un atome d'halogène, un groupe hydroxy, un groupe cyano, un groupe alkyle en C₁₋₆, un groupe alcoxy en C₁₋₆, un groupe fluorométhyle, un groupe difluorométhyle, un groupe trifluorométhyle, un groupe alcényle en Z₂₋₆, un groupe alcynyle en C₂₋₆, un groupe aryle, un groupe -C₁₋₆-alkylaryle, un groupe -C₁₋₆-alkylhétéroaryle et un groupe aryloxy ;
R² est un atome d'hydrogène, un groupe alkyle en C₁₋₆ éventuellement substitué par un groupe cyano, un groupe O-C₁₋₄-alkyl-OR⁷, un groupe OR⁷, un groupe COOR⁷, un groupe CONR⁸R⁹, un groupe CONR⁸OR⁹, un groupe C (NH₂) =NOH, un groupe NR¹⁶R¹⁷, un groupe NHC(O)OR¹⁶, un groupe NHS (O)₂R¹⁸, un groupe NHC (O) R¹⁸, un groupe SR¹⁶, un groupe S (O) R¹⁸ ou un groupe S (O) ₂R¹⁸ ; ou R² est un groupe C₁₋₄-alkyl-CONR¹⁰R¹¹, un groupe alcényle en Z₂₋₆, un groupe aryle, un groupe -C₁₋₆-alkylaryle, un groupe -C₁₋₆-alkylhétérocyclyle ou un groupe -C₁₋₆-alkylhétéroaryle ;
R³ et R^{3'} sont choisis indépendamment parmi un atome d'hydrogène, un atome d'halogène, un groupe hydroxy, un groupe cyano, un groupe alkyle en C₁₋₄, un groupe alcoxy en C₁₋₄, un groupe fluorométhyle, un groupe difluorométhyle, un groupe trifluorométhyle, un groupe éthényle et un groupe éthynyle ;
lorsque ---- est une liaison simple, R⁴ est un atome d'hydrogène, un groupe alkyle en C₁₋₆, un groupe aryle ou un groupe alcényle en C₂₋₆, ou, lorsque ---- est une double liaison, R⁴ est absent ;
R⁵ et R⁶ sont choisis indépendamment parmi un atome d'hydrogène, un groupe alkyle en C₁₋₆, un groupe aryle, un groupe alcényle en C₂₋₆, un groupe cyano, un groupe tétrazole, un groupe COOR¹², un groupe CONR¹²R¹³ et un groupe CONR¹²OR¹³ ;
R⁷, R⁸ et R⁹ sont choisis indépendamment parmi un atome d'hydrogène et un groupe alkyle en C₁₋₄ ;
R¹⁰ et R¹¹ sont choisis indépendamment parmi un atome d'hydrogène, un groupe alkyle en C₁₋₄ éventuellement substitué par un groupe OR⁷, un groupe COOR⁷ ou un groupe NR¹⁴R¹⁵, un groupe aryle, un groupe hétéroaryle, un groupe cycloalkyle en C₃₋₇, un groupe hétérocyclyle, un groupe -C₁₋₄-alkylaryle, un groupe -C₁₋₄-alkylhétéroaryle, un groupe -C₁₋₄-alkyl-C₃₋₇-cycloalkyle ou un groupe -C₁₋₄-alkylhétérocyclyle, où l'un quelconque des noyaux est éventuellement substitué par 1 ou 2 substituants choisis indépendamment parmi un atome d'halogène, un groupe hydroxy, un groupe cyano, un groupe alkyle en C₁₋₄, un groupe alcoxy en C₁₋₄, un groupe fluorométhyle, un groupe difluorométhyle et un groupe trifluorométhyle ;
ou R¹⁰ et R¹¹, conjointement avec l'atome d'azote auquel ils sont fixés, forment un hétérocycle à 4 à 7 chaînons renfermant éventuellement un hétéroatome supplémentaire choisi parmi un atome d'azote et un atome d'oxygène, lequel hétérocycle est éventuellement substitué par un groupe alkyle en C₁₋₄, un groupe halogéno, un groupe OR⁷ ou un groupe COR⁷, ou deux liaisons sur un atome de carbone cyclique de l'hétérocycle peuvent former éventuellement un substituant oxo (= O) ; ;
R¹² et R¹³ sont choisis indépendamment parmi un atome d'hydrogène, un groupe alkyle en C₁₋₄, un groupe aryle, un groupe -C₁₋₄-alkylaryle et un groupe -C₁₋₄-alkylhétéroaryle ;
ou R¹² et R¹³ peuvent être cyclisés pour former un hétérocycle à 4-7 chaînons, éventuellement substitué ;
R¹⁴ et R¹⁵ sont choisis indépendamment parmi un atome d'hydrogène et un groupe alkyle en C₁₋₄ ;
ou R¹⁴ et R¹⁵, conjointement avec l'atome d'azote auquel ils sont fixés, forment un hétérocycle à 4 à 7 chaînons renfermant éventuellement un hétéroatome supplémentaire choisi parmi un atome d'azote et un atome d'oxygène, lequel hétérocycle est éventuellement substitué par un groupe alkyle en C₁₋₄, un groupe halogéno, un groupe OR⁷ ou un groupe COR⁷, ou deux liaisons sur un atome de carbone cyclique de l'hétérocycle peuvent former éventuellement un substituant oxo (= O) ;
R¹⁶ et R¹⁷ sont choisis indépendamment parmi un atome d'hydrogène et un groupe alkyle en C₁₋₄ ;
R¹⁸ est un groupe alkyle en C₁₋₄ ; et
n vaut 0 ou 1.

2. Composé selon la revendication 1 de formule (Ia) : 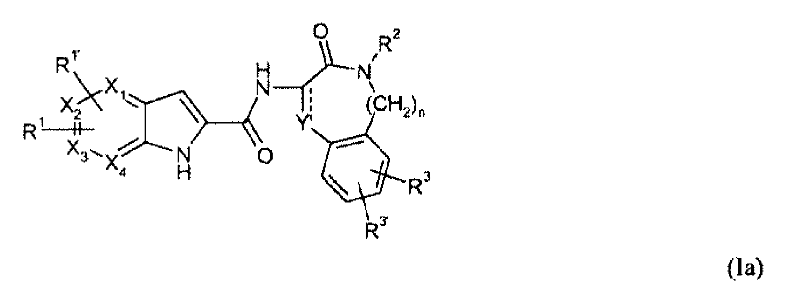 ou un stéréoisomère ou un sel pharmaceutiquement acceptable de celui-ci, dans laquelle :
l'un parmi X₁, X₂, X₃ et X₄ est un atome d'azote et les autres sont un atome de carbone ;
---- est une liaison simple ou double ;
lorsque ---- est une liaison simple, Y est un groupe CH₂, un groupe NH ou un atome d'oxygène ; et lorsque ---- est une double liaison, Y est un groupe CH ou un atome d'azote ;
R¹ et R^{1'} sont choisis indépendamment parmi un atome d'hydrogène, un atome d'halogène, un groupe hydroxy, un groupe cyano, un groupe alkyle en C₁₋₄, un groupe alcoxy en C₁₋₄, un groupe fluorométhyle, un groupe difluorométhyle, un groupe trifluorométhyle, un groupe éthényle et un groupe éthynyle ;
R² est un atome d'hydrogène, un groupe alkyle en C₁₋₄ éventuellement substitué par un groupe cyano, un groupe O-C₁₋₄-alkyl-OR⁴, un groupe OR⁴, un groupe COOR⁴, un groupe CONR⁵R⁶, un groupe CONR⁵OR⁶, un groupe C(NH₂)=N(OH), un groupe NR¹⁶R¹⁷, un groupe NHC(O)OR¹⁶, un groupe NHS (O)₂R¹⁸, un groupe NHC(O)R¹⁸, un groupe SR¹⁶, un groupe S (O) R¹⁸ ou un groupe S (O) ₂R¹⁸ ; ou R² est un groupe C₁₋₄-alkyl-CONR⁷R⁸ ou un groupe -C₁₋₄-alkylhétérocyclyle ;
R³ et R^{3'} sont choisis indépendamment parmi un atome d'hydrogène, un atome d'halogène, un groupe hydroxy, un groupe cyano, un groupe alkyle en C₁₋₄, un groupe alcoxy en C₁₋₄, un groupe fluorométhyle, un groupe difluorométhyle, un groupe trifluorométhyle, un groupe éthényle et un groupe éthynyle ;
R⁴, R⁵ et R⁶ sont choisis indépendamment parmi un atome d'hydrogène et un groupe alkyle en C₁₋₄ ;
R⁷ et R⁸ sont choisis indépendamment parmi un atome d'hydrogène, un groupe alkyle en C₁₋₄ éventuellement substitué par un groupe OR⁴, un groupe COOR⁴ ou un groupe NR⁹R¹⁰, un groupe aryle, un groupe hétéroaryle, un groupe cycloalkyle en C₃₋₇, un groupe hétérocyclyle, un groupe -C₁₋₄-alkylaryle, un groupe -C₁₋₄-alkylhétéroaryle, un groupe -C₁₋₄-alkyl-C₃₋₇-cycloalkyle ou un groupe -C₁₋₄-alkylhétérocyclyle, où l'un quelconque des noyaux est éventuellement substitué par 1 ou 2 substituants choisis indépendamment parmi un atome d'halogène, un groupe hydroxy, un groupe cyano, un groupe alkyle en C₁₋₄, un groupe alcoxy en C₁₋₄, un groupe fluorométhyle, un groupe difluorométhyle et un groupe trifluorométhyle ;
ou R⁷ et R⁸, conjointement avec l'atome d'azote auquel ils sont fixés, forment un hétérocycle à 4 à 7 chaînons renfermant éventuellement un hétéroatome supplémentaire choisi parmi un atome d'azote et un atome d'oxygène, lequel hétérocycle est éventuellement substitué par un groupe alkyle en C₁₋₄, un groupe halogéno, un groupe OR⁴ ou un groupe COR⁴, ou deux liaisons sur un atome de carbone cyclique de l'hétérocycle peuvent former éventuellement un substituant oxo (= O) ; ;
R⁹ et R¹⁰ sont choisis indépendamment parmi un atome d'hydrogène et un groupe alkyle en C₁₋₄ ;
ou R⁹ et R¹⁰, conjointement avec l'atome d'azote auquel ils sont fixés, forment un hétérocycle à 4 à 7 chaînons renfermant éventuellement un hétéroatome supplémentaire choisi parmi un atome d'azote et un atome d'oxygène, lequel hétérocycle est éventuellement substitué par un groupe alkyle en C₁₋₄, un groupe halogéno, un groupe OR⁴ ou un groupe COR⁴, ou deux liaisons sur un atome de carbone cyclique de l'hétérocycle peuvent former éventuellement un substituant oxo (= O) ; ;
R¹⁶ et R¹⁷ sont choisis indépendamment parmi un atome d'hydrogène et un groupe alkyle en C₁₋₄ ;
R¹⁸ est un groupe alkyle en C₁₋₄ ; et
n vaut 0 ou 1.

3. Composé selon la revendication 1 ou 2, ou un stéréoisomère, ou un sel pharmaceutiquement acceptable de celui-ci, dans lequel X₃ est un atome d'azote.

4. Composé selon l'une quelconque des revendications précédentes, ou un stéréoisomère, ou un sel pharmaceutiquement acceptable de celui-ci, dans lequel R¹ et R^{1'} sont chacun indépendamment un atome d'halogène, un groupe cyano ou un atome d'hydrogène.

5. Composé selon la revendication 4, ou un stéréoisomère, ou un sel pharmaceutiquement acceptable de celui-ci, dans lequel l'un parmi R¹ et R^{1'} est un atome d'hydrogène et l'autre est un groupe 5-halogéno ou un groupe 5-cyano.

6. Composé selon l'une quelconque des revendications précédentes, ou un stéréoisomère, ou un sel pharmaceutiquement acceptable de celui-ci, dans lequel, lorsque R² est un groupe C₁₋₄-alkyl-CO-NR⁵R⁶ ou R² est un groupe C₁₋₆-alkyl-CO-NR⁸R⁹, selon le cas, il peut s'agir d'un groupe CH₂-CO-NR⁵R⁶ ou d'un groupe CH₂-CO-NR⁸R⁹, selon le cas.

7. Composé selon l'une quelconque des revendications précédentes, ou un stéréoisomère, ou un sel pharmaceutiquement acceptable de celui-ci, dans lequel R³ et R^{3'} sont choisis indépendamment parmi un atome d'hydrogène, un atome d'halogène, un groupe alkyle en C₁₋₄, un groupe alcoxy en C₁₋₄ et un groupe trifluorométhyle.

8. Composé selon la revendication 7, ou un stéréoisomère, ou un sel pharmaceutiquement acceptable de celui-ci, dans lequel au moins l'un parmi R³ et R^{3'} est un atome d'hydrogène.

9. Composé selon l'une quelconque des revendications précédentes, ou un stéréoisomère, ou un sel pharmaceutiquement acceptable de celui-ci, dans lequel n vaut 0**.**

10. Composé choisi parmi l'un quelconque des Exemples 1 à 75, sous la forme de la base libre ou d'un sel pharmaceutiquement acceptable de celui-ci.

11. Composition comprenant un composé selon l'une quelconque des revendications 1 à 10, ou un stéréoisomère, ou un sel pharmaceutiquement acceptable de celui-ci, et un support pharmaceutiquement acceptable.

12. Composé selon l'une quelconque des revendications 1 à 10, ou un stéréoisomère, ou un sel pharmaceutiquement acceptable de celui-ci, destiné à être utilisé pour le traitement prophylactique ou thérapeutique de l'hyperglycémie ou du diabète.

13. Composé selon l'une quelconque des revendications 1 à 10, ou un stéréoisomère, ou un sel pharmaceutiquement acceptable de celui-ci, destiné à être utilisé pour la prévention du diabète chez un être humain présentant une hyperglycémie pré-diabétique ou une tolérance du glucose altérée.

14. Composé selon l'une quelconque des revendications 1 à 10, ou un stéréoisomère, ou un sel pharmaceutiquement acceptable de celui-ci, destiné à être utilisé pour le traitement prophylactique ou thérapeutique de l'hypercholestérolémie, de l'hyperinsulinémie, de l'hyperlipidémie, de l'athérosclérose ou de l'ischémie du myocarde, ou en tant qu'agent cardioprotecteur.

15. Utilisation d'un composé selon l'une quelconque des revendications 1 à 10, ou d'un stéréoisomère, ou d'un sel pharmaceutiquement acceptable de celui-ci, pour la fabrication d'un médicament destiné au traitement prophylactique ou thérapeutique d'une maladie ou d'un trouble tel que défini dans l'une des revendications 12 à 14.

16. Procédé de production d'un composé de formule (I) selon la revendication 1, comprenant le couplage d'un acide carboxylique de formule (II), ou d'un dérivé protégé ou activé de celui-ci, avec une amine de formule (III) :
